(19) Europäisches Patentamt
European Patent Office
Office européen des brevets

(11) EP 3 889 150 A1

(12) # EUROPEAN PATENT APPLICATION
published in accordance with Art. 153(4) EPC

(43) Date of publication:
**06.10.2021 Bulletin 2021/40**

(21) Application number: **19904699.6**

(22) Date of filing: **25.12.2019**

(51) Int Cl.:
*C07D 405/14* (2006.01) *C07D 401/14* (2006.01)
*C07D 401/12* (2006.01) *C07D 487/04* (2006.01)
*C07D 403/12* (2006.01) *C07D 471/04* (2006.01)
*C07D 231/56* (2006.01) *A61K 31/4439* (2006.01)
*A61K 31/4545* (2006.01) *A61K 31/519* (2006.01)
*A61K 31/416* (2006.01) *A61K 31/498* (2006.01)
*A61K 31/497* (2006.01) *A61P 37/00* (2006.01)
*A61P 19/02* (2006.01) *A61P 35/00* (2006.01)
*A61P 19/06* (2006.01) *A61P 17/06* (2006.01)
*A61P 37/02* (2006.01) *A61P 9/10* (2006.01)
*A61P 3/00* (2006.01)

(86) International application number:
**PCT/CN2019/128347**

(87) International publication number:
**WO 2020/135513 (02.07.2020 Gazette 2020/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.12.2018 CN 201811593893**

(71) Applicant: **Shanghai Meiyue Biotech Development Co., Ltd.**
**Shanghai 200120 (CN)**

(72) Inventors:
- **FENG, Yan**
  **Shanghai 201206 (CN)**
- **YE, Guozhong**
  **Shanghai 201206 (CN)**
- **LI, Shiqiang**
  **Shanghai 201206 (CN)**
- **HU, Zhilong**
  **Shanghai 201206 (CN)**
- **WANG, Chaodong**
  **Shanghai 201206 (CN)**

(74) Representative: **Gille Hrabal Partnerschaftsgesellschaft mbB**
**Patentanwälte**
**Brucknerstraße 20**
**40593 Düsseldorf (DE)**

(54) **COMPOUND SERVING AS IRAK INHIBITOR**

(57) The present disclosure relates a compound as an IRAK inhibitor. Specifically, the present disclosure provides a compound of formula I, or a cis-trans isomer, an optical isomer, a racemate, a pharmaceutically acceptable salt, a prodrug, a deuterated derivative thereof, a hydrate or a solvate thereof. The compounds disclosed herein have potent inhibitory effects on IRAK and thus have therapeutic effect on IRAK-related diseases.

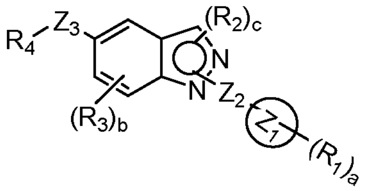

I



Printed by Jouve, 75001 PARIS (FR)

## Description

The present application claims the priority right of prior Chinese Patent Application No. 201811593893.X filed on Dec. 25, 2018 before China National Intellectual Property Administration, titled "COMPOUND AS IRAK INHIBITOR", which is incorporated herein by reference in its entirety.

## TECHNICAL FIELD

The present disclosure relates to the field of pharmaceutical chemistry, and in particular to a compound as an IRAK inhibitor, a method for preparing the same and use of the same in preparing a medicament.

## BACKGROUND

Interleukin-1 receptor-associated kinase (IRAK) family are intracellular serine-threonine kinases, including: IRAK1 (interleukin-1 receptor-associated kinase-1), IRAK2 (interleukin-1 receptor-associated kinase-2), IRAK-M (interleukin-1 receptor-associated kinase-M) and IRAK4 (interleukin-1 receptor-associated kinase-4). A common feature of these four members is the typical N-terminal death domain that mediates the interaction between the MyD88 family adapter and the central kinase domain. In the interleukin-1 receptor-associated kinase (IRAK) family, IRAK4 is a key factor downstream of the Toll-like receptor (TLR)/interleukin-1 receptor (IL-1R)-mediated inflammatory signaling pathway. When the binding of ligand to a pathogen-specific molecule (e.g., lipopolysaccharide, polypeptide and viral DNA, etc.) recognized by the extracellular portion of TLR, the intracellular portion recruits MyD88 and other factors to form complexes and initiate IRAK1 autophosphorylation, thereby activating downstream serine-threonine kinase TAK1, promoting NF-κB and MAPK signaling pathways, producing proinflammatory cytokines, chemokines and destructive enzymes, and ultimately leading to inflammatory responses that mediate innate immunity. IL-1R is involved in host defense and hematopoiesis and serves as a bridge connecting the innate immunity and acquired immunity. (Flannery, et. al, Biochem. Pharmacol., 2010, 80 (12):1981-1991).

Rheumatoid arthritis (RA) is a chronic, inflammatory, systemic autoimmune disease prominently characterized by non-suppurative inflammation in joints and joint tissues. RA is mainly manifested by synovitis of joints, which eventually causes damage to various tissues (such as cartilages of joints, ligaments and tendons) and multiple organs. Studies have shown that a variety of immune cells participate and mediate the autoimmune inflammation in RA patients, including T/B lymphocytes, macrophages, neutrophils, and others. Meanwhile, a large number of researches have demonstrated the direct association between cytokines and RA, such as interleukins (IL-1/IL-6 and the like) and TNF-α.

Studies have shown that IRAK4 inhibitors can effectively block the production of the proinflammatory cytokine tumor necrosis factor (TNF) in LPS or CpG-induced human leukocytes; in a mouse model of collagen-induced arthritis, IRAK4 inhibitors can significantly inhibit the release of TNF, thereby controlling disease progression; in a mouse model of MyD88-dependent inflammatory gout, IRAK4 inhibitors are able to dose-dependently block leukocyte infiltration (Priscilla N, et. al., J. Exp. Med., 2015, 13 (212):2189-2201).

Therefore, it is believed that the excessive activation of IRAK4-dependent TLR/IL-1R signaling pathway is closely related to the development and progression of rheumatoid arthritis. It has been confirmed in various studies that IRAK4 activation is closely related to the onset and progression of diseases such as tumors, gout, systemic lupus erythematosus, multiple sclerosis, metabolic syndrome, atherosclerosis, myocardial infarction, sepsis, inflammatory bowel disease, asthma, and allergy (Chaudhary D, et. al., J. Med. Chem. 2015, 58 (1):96-110).

Therefore, there is a need in the art to develop a novel kinase cytokine inhibitor to improve the efficacy of treating the related diseases.

## SUMMARY

To improve the above technical problems, the present disclosure provides a compound having a novel structure and targeting IRAK (especially IRAK4) and use thereof.

In a first aspect of the present disclosure, provided is a compound of formula I, or a cis-trans isomer, an optical isomer, a racemate, a pharmaceutically acceptable salt, a prodrug, a deuterated derivative, a hydrate or a solvate thereof:

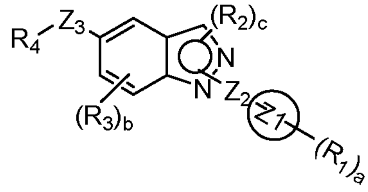

I

wherein in the formula I:

$Z_1$ is selected from substituted or unsubstituted $C_3$-$C_{12}$ cycloalkylenyl, and substituted or unsubstituted 3-12 membered heterocycloalkylenyl;

$Z_2$ is selected from a chemical bond (absent), substituted or unsubstituted $C_1$-$C_6$ alkylenyl, substituted or unsubstituted $C_2$-$C_6$ alkenylenyl, and substituted or unsubstituted $C_2$-$C_6$ alkynylenyl;

each $R_1$ is independently selected from hydroxyl, thiol, carboxyl, substituted or unsubstituted $C_1$-$C_{12}$ alkyl, substituted or unsubstituted $C_3$-$C_{12}$ cycloalkyl, substituted or unsubstituted $C_1$-$C_{12}$ hydroxyalkyl, substituted or unsubstituted $C_1$-$C_{12}$ thiolalkyl, substituted or unsubstituted $C_1$-$C_{12}$ aminoalkyl, substituted or unsubstituted $C_3$-$C_{12}$ hydroxycycloalkyl, substituted or unsubstituted $C_3$-$C_{12}$ thiolcycloalkyl, substituted or unsubstituted $C_3$-$C_{12}$ aminocycloalkyl, substituted or unsubstituted $C_1$-$C_{12}$ alkoxy, substituted or unsubstituted $C_1$-$C_{12}$ alkylthio, -N($R_5$)$R_6$, substituted or unsubstituted $C_1$-$C_8$ carboxyl, substituted or unsubstituted $C_2$-$C_8$ acyl, substituted or unsubstituted $C_2$-$C_8$ ester group, and halogen;

$R_2$ and $R_3$ are each independently selected from a chemical bond, substituted or unsubstituted $C_1$-$C_{10}$ alkyl, substituted $C_3$-$C_{10}$ cycloalkyl, halogen, $C_1$-$C_{10}$ haloalkyl, $C_1$-$C_{12}$ hydroxyalkyl, cyano, nitro, -A-$R_7$, -N($R_5$)$R_6$, substituted or unsubstituted $C_6$-$C_{12}$ aryl, and substituted or unsubstituted 5-12 membered heteroaryl;

$R_5$ and $R_6$ are each independently selected from hydrogen, substituted or unsubstituted $C_1$-$C_{10}$ alkyl, and substituted or unsubstituted $C_3$-$C_8$ cycloalkyl;

A is selected from a chemical bond, S and O;

$R_7$ is selected from hydrogen, substituted or unsubstituted $C_1$-$C_{10}$ alkyl, substituted or unsubstituted $C_2$-$C_{10}$ alkenylenyl, substituted or unsubstituted $C_3$-$C_8$ cycloalkyl, substituted or unsubstituted 3-12 membered heterocycloalkyl, substituted or unsubstituted 5-20 membered heteroaryl, and -$R_8$-$R_9$;

$R_8$ is selected from a chemical bond, substituted or unsubstituted $C_1$-$C_6$ alkylenyl, and substituted or unsubstituted $C_2$-$C_6$ alkenylenyl;

$R_9$ is selected from substituted or unsubstituted $C_3$-$C_{12}$ cycloalkyl, and substituted or unsubstituted $C_3$-$C_{12}$ heterocycloalkyl;

$Z_3$ is selected from carbonyl,

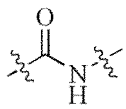

and

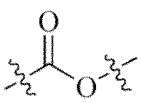
;

$R_4$ is selected from substituted or unsubstituted $C_6$-$C_{20}$ aryl, and substituted or unsubstituted 5-20 membered heteroaryl;

wherein each of the "substituted" means that one, two or more (preferably 1, 2, 3 or 4) hydrogen atoms on the group are substituted by a substituent selected from: $C_1$-$C_{12}$ hydroxyalkyl, $C_2$-$C_8$ acyl, $C_3$-$C_8$ cycloalkyl, $C_1$-$C_6$ alkyl, $C_1$-$C_6$ alkoxy, $C_1$-$C_6$ alkylthio, hydroxyl, thiol, amino, nitro, halogen, 3-12 membered heterocycloalkyl, cyano, $C_1$-$C_{10}$ haloalkyl, $C_3$-$C_8$ halocycloalkyl, $C_2$-$C_4$ ester group, $C_2$-$C_4$ acylamino, $C_1$-$C_4$ carboxyl, $C_2$-$C_6$ alkenyl, $C_2$-$C_6$ alkynyl, $C_6$-$C_{12}$ aryl, and 5-12 membered heteroaryl; and

the heterocycloalkylenyl, heterocycloalkyl and heteroaryl each independently comprises 1-3 (preferably 1, 2 or 3) heteroatoms selected from N, O and S;

a is selected from 1, 2, 3 and 4;
b is selected from 0, 1, 2 and 3; and
c is selected from 0 and 1.

In another preferred embodiment, the heterocycloalkylenyl, heterocycloalkyl and heteroaryl each independently comprises one N atom;

In another preferred embodiment, $Z_2$ is connected with the N atom on the pyrazole ring.

In another preferred embodiment, $Z_3$ represents

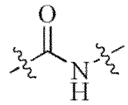

or

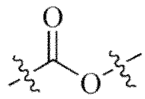

,

wherein N or O is connected with the phenyl ring.

In another preferred embodiment, the heterocycloalkylenyl comprises 1-2 hetero atoms selected from N.

In another preferred embodiment, a is 1.

In another preferred embodiment, b is 1.

In another preferred embodiment, c is 1.

In another preferred embodiment, c is 0.

In another preferred embodiment, a is 1, b is 1, and/or c is 0.

In another preferred embodiment, the isomer is a cis-trans isomer.

In another preferred embodiment, the compound has a structure of formula Ia:

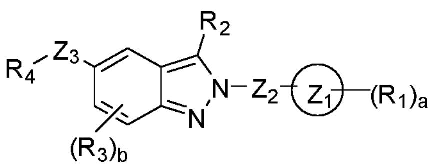

Ia

wherein in the formula Ia, $R_1$, $R_2$, $R_3$, $R_4$, $Z_1$, $Z_2$, $Z_3$, a and b are defined as above.

In another preferred embodiment, b is 1.

In another preferred embodiment, the compound has a structure of formula Ib:

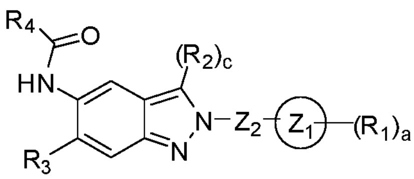

Ib

wherein in the formula Ib, $R_1$, $R_2$, $R_3$, $R_4$, $Z_1$, $Z_2$, a and c are defined as above.

In another preferred embodiment, the compound has a structure of formula Ic:

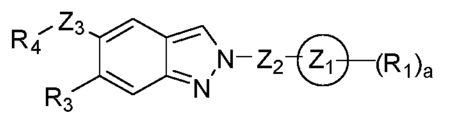

Ic

wherein in the formula Ic, $R_1$, $R_2$, $R_3$, $R_4$, $Z_1$, $Z_2$ and a are defined as above.

In another preferred embodiment, $Z_3$ is -CO-NH-.

In another preferred embodiment, the compound has a structure of formula Id:

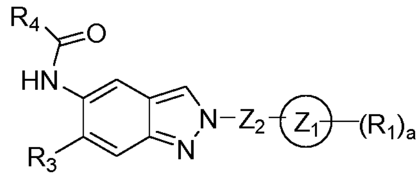

Id

wherein in the formula Id, $R_1$, $R_3$, $R_4$, $Z_1$, $Z_2$ and a are defined as above.

In another preferred embodiment, the compound has a structure of formula Ie:

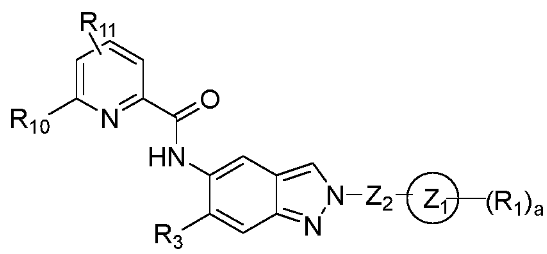

Ie

wherein in the formula Ie, $R_1$, $R_3$, $Z_1$ and $Z_2$ are defined as above; and $R_{10}$ and $R_{11}$ are each independently selected from a chemical bond, substituted or unsubstituted $C_1$-$C_8$ alkyl, and substituted or unsubstituted $C_3$-$C_6$ cycloalkyl.

In another preferred embodiment, $R_{10}$ is substituted or unsubstituted $C_1$-$C_3$ alkyl, and $R_{11}$ is a chemical bond, wherein the "substituted" means that one, two or more hydrogen atoms (preferably all hydrogen atoms) in the $C_1$-$C_3$ alkyl are substituted by halogen (preferably F).

In another preferred embodiment, the compound comprises one or more features selected from the group consisting of:

(i) $Z_1$ is selected from substituted or unsubstituted $C_3$-$C_8$ cycloalkylenyl, and substituted or unsubstituted 3-8 membered heterocycloalkylenyl;

(ii) $Z_2$ is selected from a chemical bond, and substituted or unsubstituted $C_1$-$C_6$ alkylenyl;

(iii) $R_1$ is selected from hydroxyl, thiol, carboxyl, substituted or unsubstituted $C_1$-$C_6$ alkyl, substituted or unsubstituted $C_3$-$C_8$ cycloalkyl, substituted or unsubstituted $C_1$-$C_6$ hydroxyalkyl, substituted or unsubstituted $C_1$-$C_6$ thiolalkyl, substituted or unsubstituted $C_1$-$C_6$ aminoalkyl, substituted or unsubstituted $C_3$-$C_6$ hydroxycycloalkyl, substituted or unsubstituted $C_3$-$C_6$ thiolcycloalkyl, substituted or unsubstituted $C_3$-$C_6$ aminocycloalkyl, substituted or unsubstituted $C_1$-$C_6$ alkoxy, substituted or unsubstituted $C_1$-$C_6$ alkylthio, -N($R_5$)$R_6$, substituted or unsubstituted $C_1$-$C_4$ carboxyl, substituted or unsubstituted $C_2$-$C_6$ acyl, substituted or unsubstituted $C_2$-$C_6$ ester group, and halogen;

(iv) $R_2$ and $R_3$ are each independently selected from a chemical bond, substituted or unsubstituted $C_1$-$C_6$ alkyl, substituted $C_3$-$C_8$ cycloalkyl, halogen, $C_1$-$C_6$ haloalkyl, $C_1$-$C_{12}$ hydroxyalkyl, cyano, nitro, -A-$R_7$, -N($R_5$)$R_6$, and 5-12 membered heteroaryl; and

(v) $R_4$ is selected from substituted or unsubstituted $C_6$-$C_{12}$ aryl, and substituted or unsubstituted 5-14 membered heteroaryl.

In another preferred embodiment, A is O.

In another preferred embodiment, $R_7$ is selected from hydrogen, substituted or unsubstituted $C_1$-$C_6$ alkyl, substituted or unsubstituted $C_3$-$C_8$ cycloalkyl, substituted or unsubstituted $C_3$-$C_8$ heterocycloalkyl, -$C_1$-$C_6$ alkylenyl-substituted or unsubstituted 3-8 membered heterocycloalkyl, and -$C_1$-$C_6$ alkylenyl-substituted or unsubstituted $C_3$-$C_8$ cycloalkyl.

In another preferred embodiment, A is a chemical bond.

In another preferred embodiment, $R_7$ is selected from substituted or unsubstituted pyrazolyl, and substituted or unsubstituted pyridinyl.

In another preferred embodiment, $R_5$ and $R_6$ are each independently selected from hydrogen, substituted or unsubstituted $C_1$-$C_6$ alkyl, and substituted or unsubstituted $C_3$-$C_8$ cycloalkyl.

In another preferred embodiment, $Z_2$ is selected from a chemical bond and $C_1$-$C_4$ alkylenyl; where $Z_2$ is $C_1$-$C_4$ alkylenyl, it is directly connected to N on the indazole ring. In another preferred embodiment, $Z_1$ is selected from substituted or unsubstituted $C_3$-$C_6$ cycloalkylenyl, and substituted or unsubstituted 3-5 membered heterocycloalkylenyl.

In another preferred embodiment, a is selected from 1 and 2.

In another preferred embodiment, $R_1$ is selected from $C_1$-$C_4$ hydroxyalkyl, halogen, -CO-$C_1$-$C_4$ hydroxyalkyl, carboxyl, -A-$R_7$, -COO-$C_1$-$C_4$ alkyl, wherein A is O, $R_7$ is selected from H and substituted or unsubstituted $C_1$-$C_4$ alkyl;

In another preferred embodiment, $R_2$ is a chemical bond.

In another preferred embodiment, $R_3$ is selected from hydroxy-$C_1$-$C_4$ alkyl, -A-$R_7$, and substituted or unsubstituted 5-6 membered heteroaryl, wherein A is O, $R_7$ is selected from substituted or unsubstituted 3-6 membered heterocycloalkyl, -$C_1$-$C_4$ alkylenyl-substituted or unsubstituted $C_3$-$C_6$ cycloalkyl, -$C_1$-$C_4$ alkylenyl-substituted or unsubstituted 3-6 membered heterocycloalkyl, $C_1$-$C_4$ alkyl, absent, -N($C_1$-$C_4$ alkyl)$_2$, and halogen; wherein the "substituted" refers to a substitution by the following groups: $C_1$-$C_4$ alkyl, -CO-$C_1$-$C_4$ alkyl, and cyano.

In another preferred embodiment, b is 1.

In another preferred embodiment, $Z_3$ is

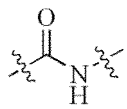

,

and the N is connected with the phenyl ring in the indazole ring.

In another preferred embodiment, $R_4$ is selected from substituted or unsubstituted 5-10 membered heteroaryl, and substituted or unsubstituted $C_6$-$C_{12}$ aryl, wherein the "substituted" means that one or more (preferably 1 or 2) hydrogen atoms of the 5-10 membered heteroaryl or $C_6$-$C_{12}$ aryl are substituted by halogen, cyano, $C_1$-$C_4$ alkyl, or $C_1$-$C_3$ haloalkyl.

In another preferred embodiment, $R_4$ is substituted or unsubstituted 5-6 membered heteroaryl having 1 N atom, wherein the "substituted" means that one or more (preferably 1 or 2) hydrogen atoms of the 5-6 membered heteroaryl are substituted by $C_1$-$C_3$ haloalkyl.

In another preferred embodiment, the $C_1$-$C_3$ haloalkyl is trihalo-substituted or perhalo-substituted.

In another preferred embodiment, the compound comprises one or more features selected from the group consisting of:

$Z_1$ is selected from cyclobutylidenyl, azetidinylidenyl and cyclohexylidenyl;
$Z_2$ is selected from a chemical bond, methylenyl and ethylidenyl;
$R_1$ is selected from 2-hydroxyisopropyl, F, 2-hydroxy-2-methylpropyl,

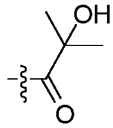

,

hydroxypropyl, hydroxymethyl, hydroxybutyl, hydroxybutyryl, -COOH, hydroxyl, methoxy, -$COOCH_3$, and methyl;
$R_2$ is absent;
$R_3$ is selected from a chemical bond, chloro, fluoro, -O-$R_7$, dimethylamino, methylamino, 2-hydroxyisopropyl and cyano, wherein $R_7$ is selected from tetrahydropyranyl, tetrahydropyridinyl, *N*-methyltetrahydropyridinyl, *N*-acetyltetrahydropyridinyl, tetrahydrofuranyl, cyclopropylmethyl-, *N*-methyltetrahydropyridylmethyl-, methyl, pyrazolyl and 2-pyridinyl;
$Z_3$ is

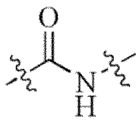
;

and the N is connected with the phenyl ring in the indazole ring;
and $R_4$ is selected from trifluoromethylpyridyl, difluoromethylpyridyl, difluoromethylpyrazinyl, fluoropyridyl,

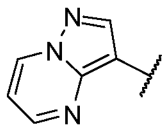
,

trifluoromethylphenyl, methylpyridyl,

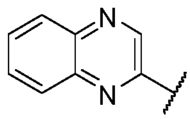
,

cyanopyridyl, cyclopropylpyridyl, and trifluoromethylpyrimidinyl.

In another preferred embodiment, $Z_1$ is selected from cyclobutylidenyl (

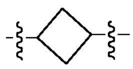

), and heterocyclobutylidenyl containing 1-2 N atoms (preferably,

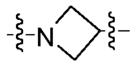

).

In another preferred embodiment, $Z_2$ is selected from a chemical bond and

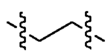
.

In another preferred embodiment, $R_1$ is selected from

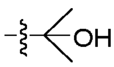
,
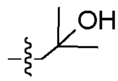
,
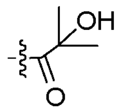
,
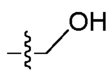
,

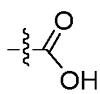
,
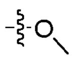
,
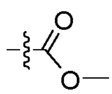
,

-F, -OH and methyl.

In another preferred embodiment, $R_2$ and $R_3$ are each independently selected from hydrogen, chloro, fluoro,

cyano,

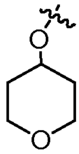, 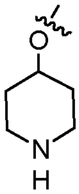, 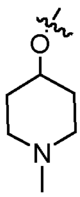, 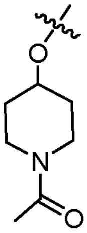, 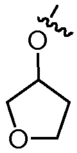, 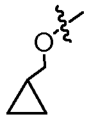, 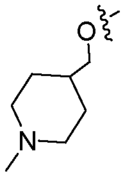, 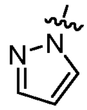, 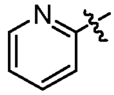,

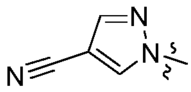, 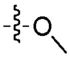, 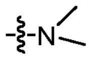, 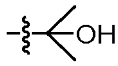

and

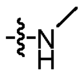.

In another preferred embodiment, $Z_3$ is

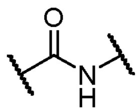.

In another preferred embodiment, $R_4$ is selected from ,

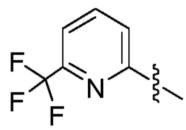, 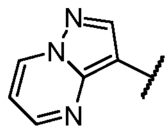, 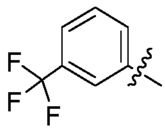,

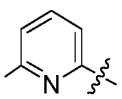, 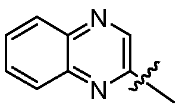, 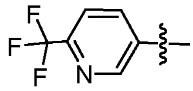, 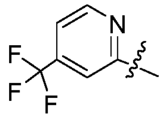, 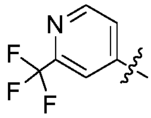, 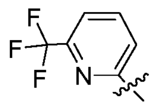,

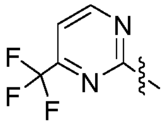, 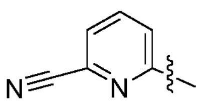, 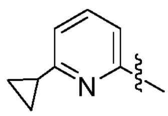, 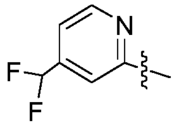, 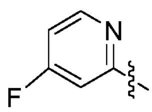, 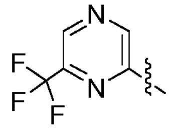

and

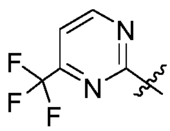.

In another preferred embodiment, the compound is selected from the group consisting of:

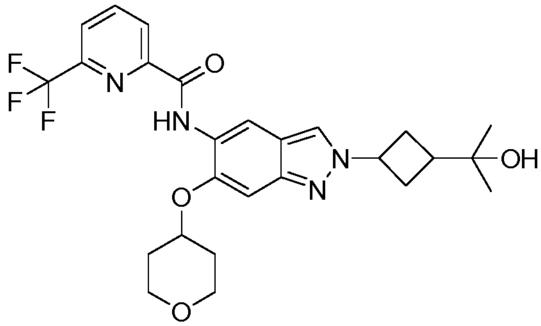

I1

,

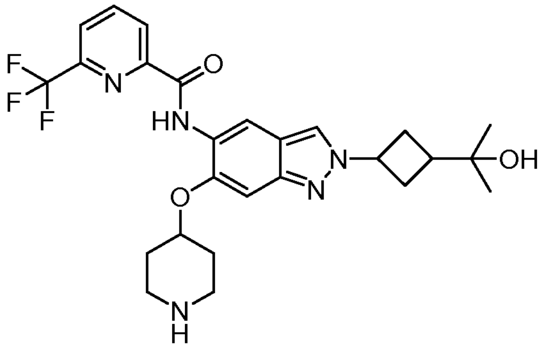

I2

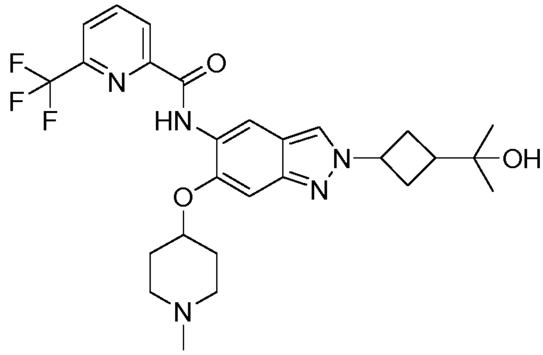

I3

,

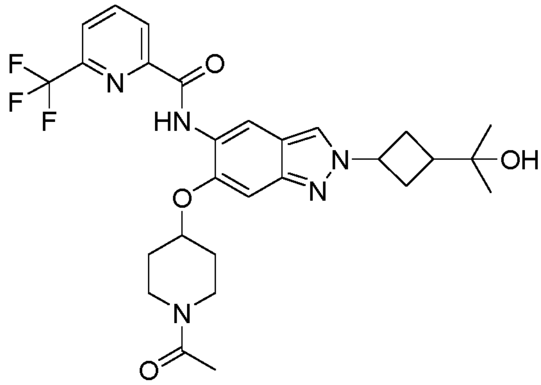

I4

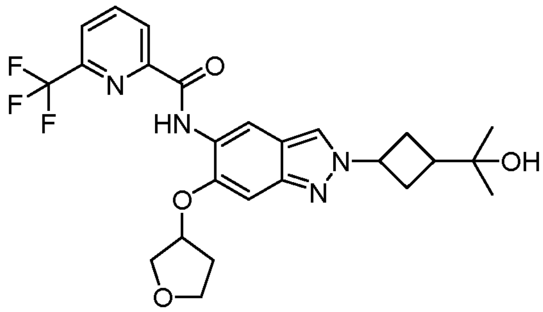

I5

,

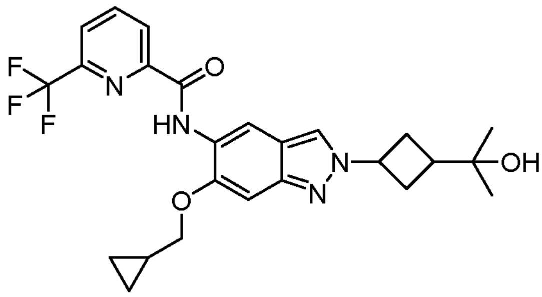

I6

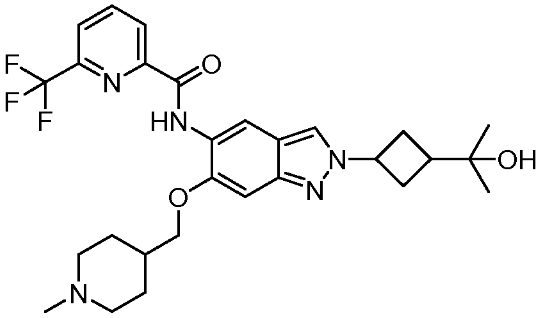

,

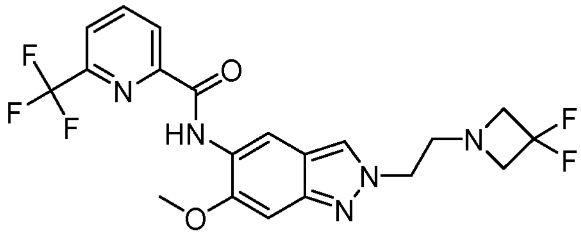

I7 I8

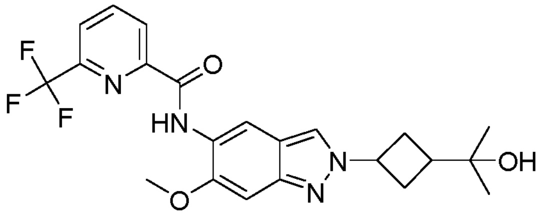

,

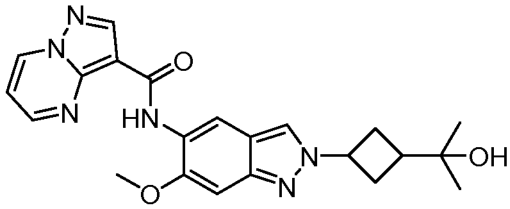

I9 I10

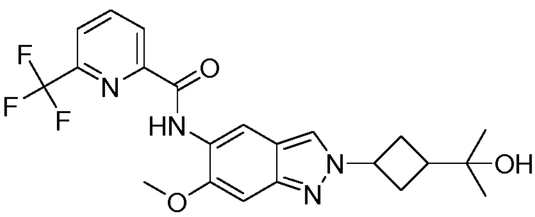

,

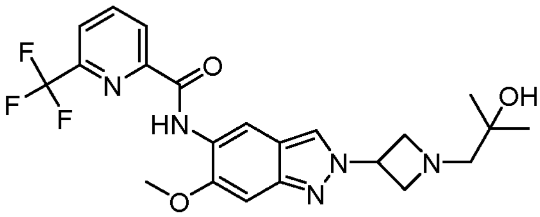

I11 I12

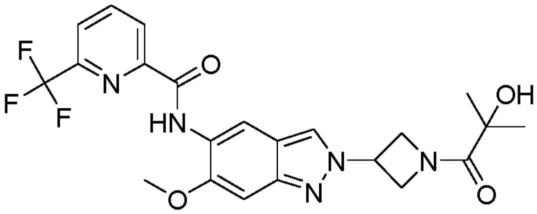

,

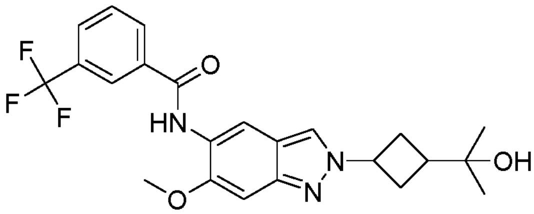

I13 I14

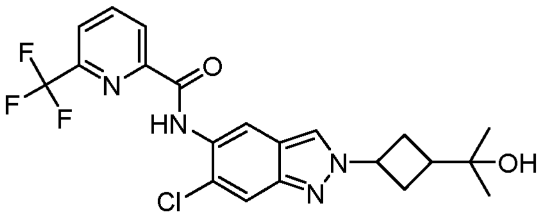

,

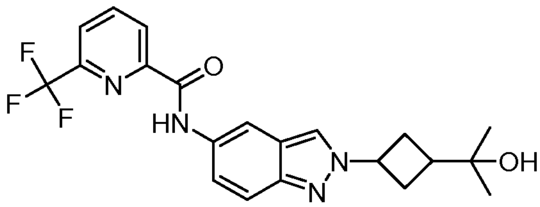

I15 I16

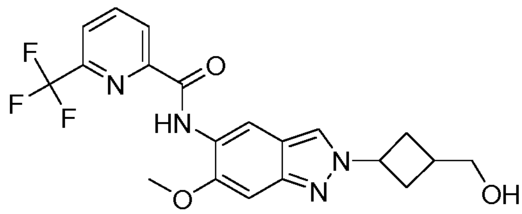,

I17

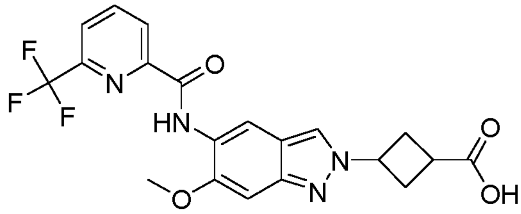

I18

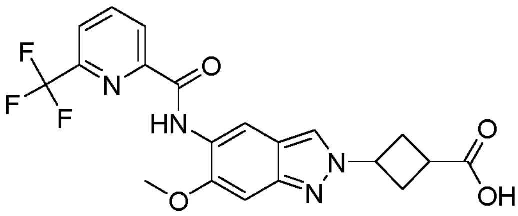,

I19

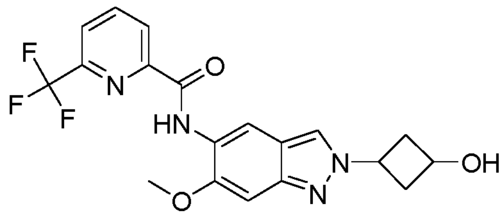

I20

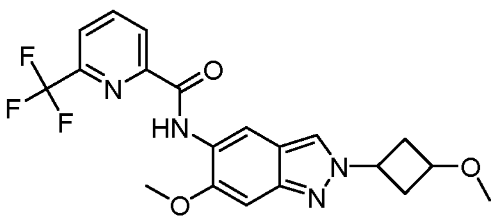,

I21

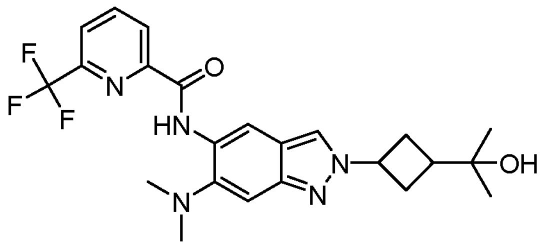

I22

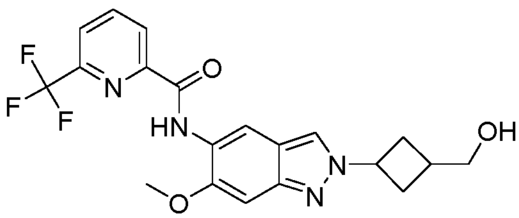,

I23

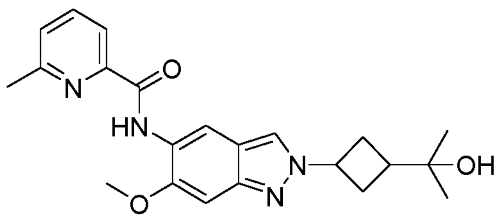

I24

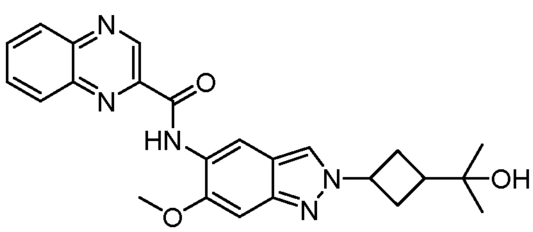,

I25

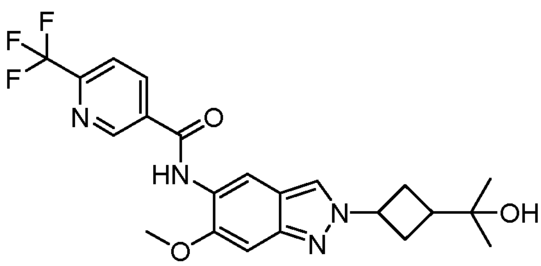

I26

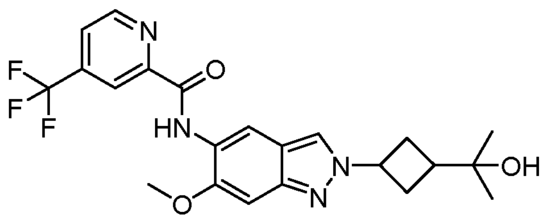

,

I27

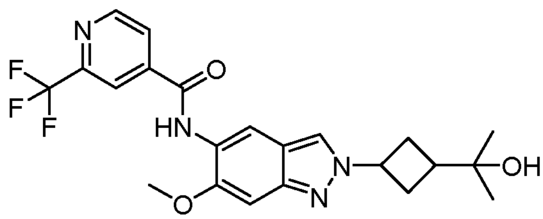

I28

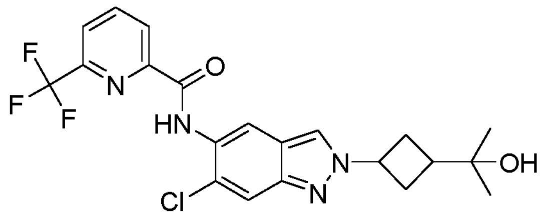

,

I29

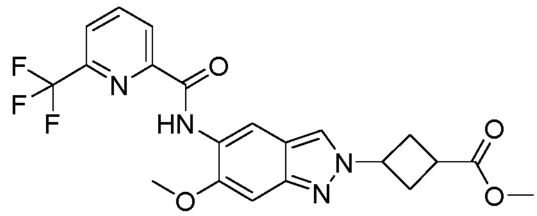

I30

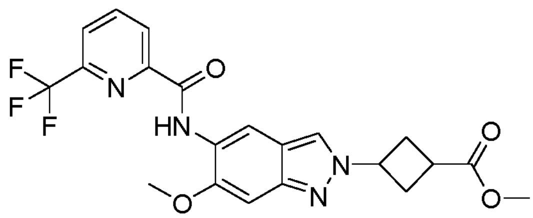

,

I31

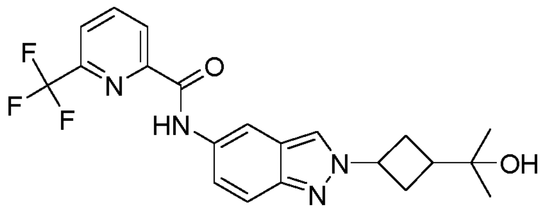

I32

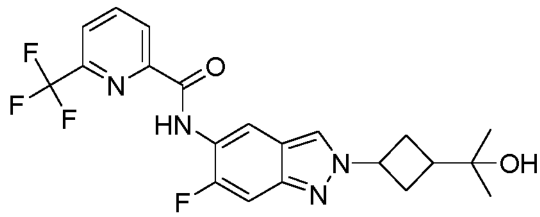

,

I33

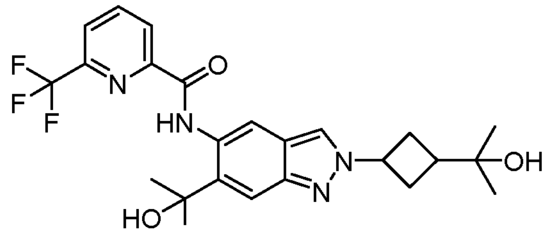

I34

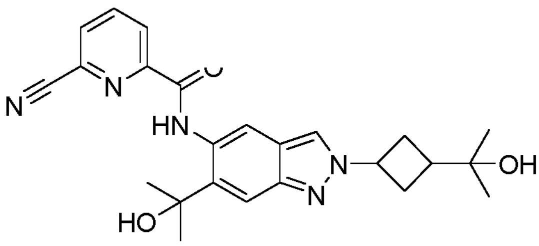

,

I35

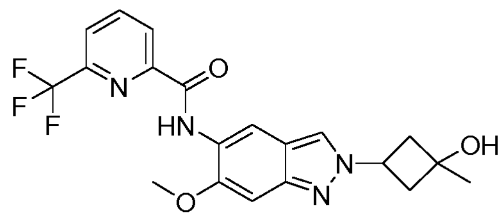

I36

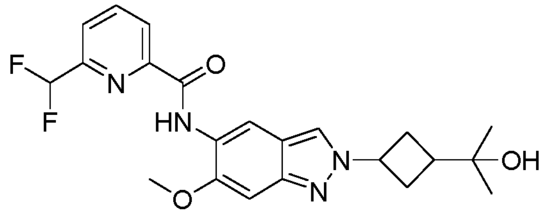

,

I37

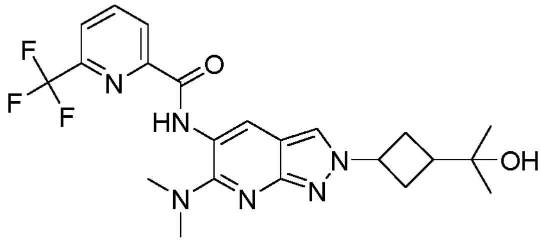

I38

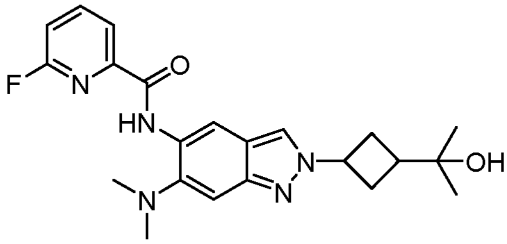

,

I39

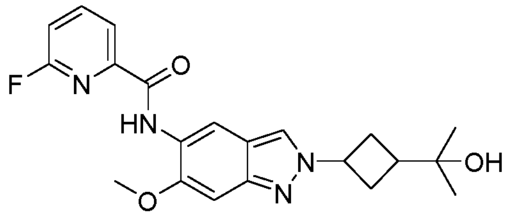

I40

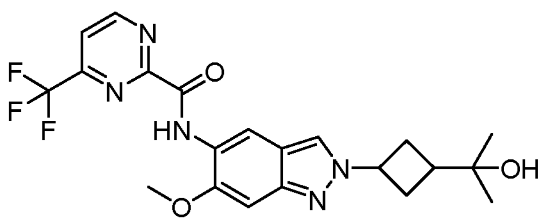

,

I41

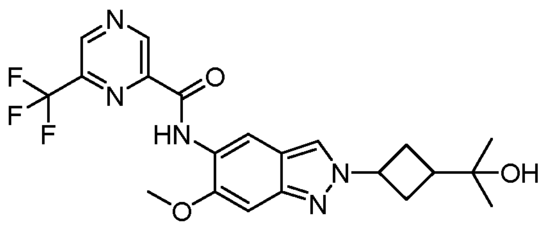

I42

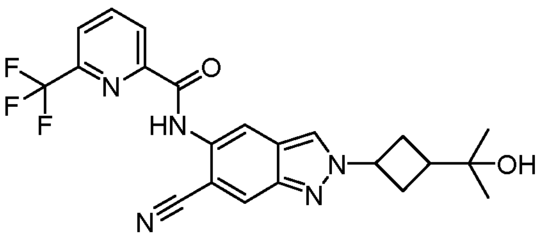

,

I43

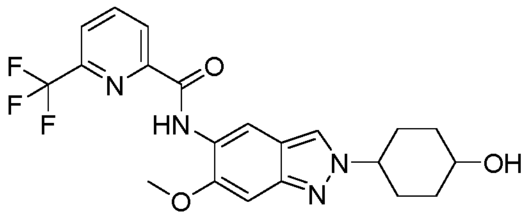

I44

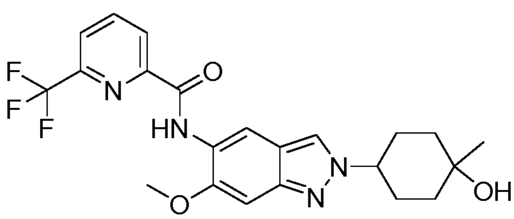

,

I45

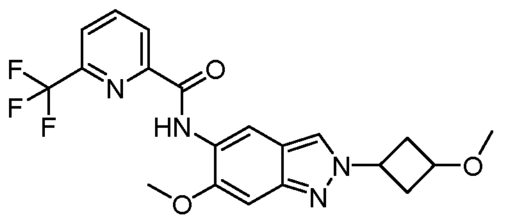

I46

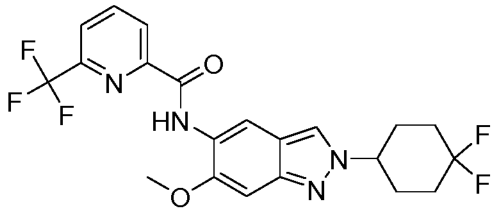

,

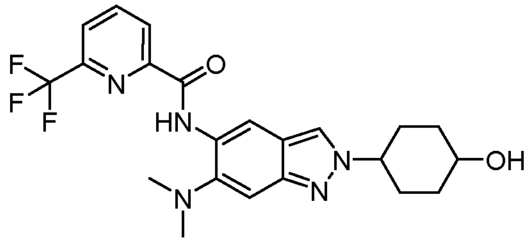

I47 I48

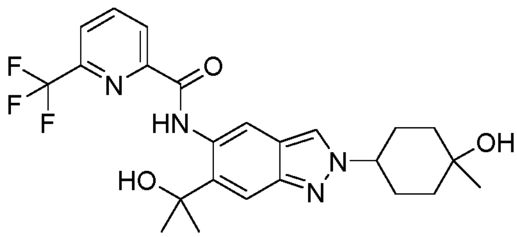

,

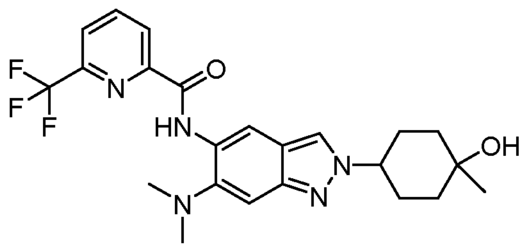

.

I49 I50

In another preferred embodiment, the compound is selected from the group consisting of:

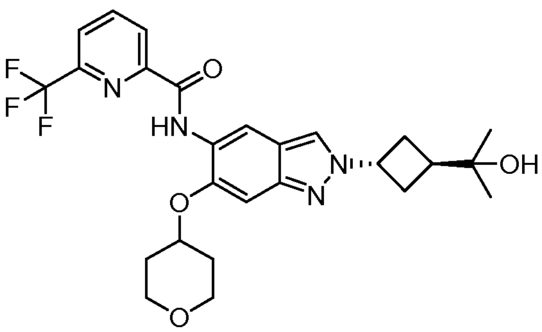

,

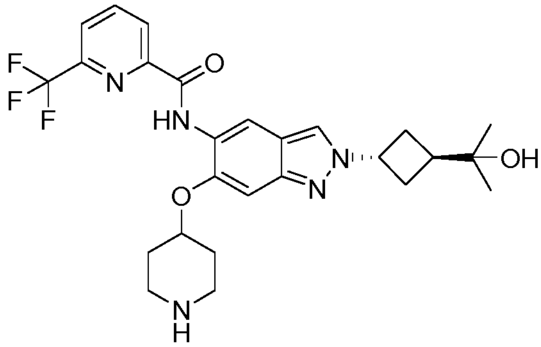

I51 I52

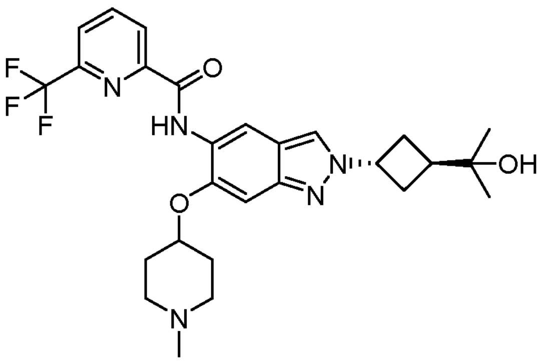

,

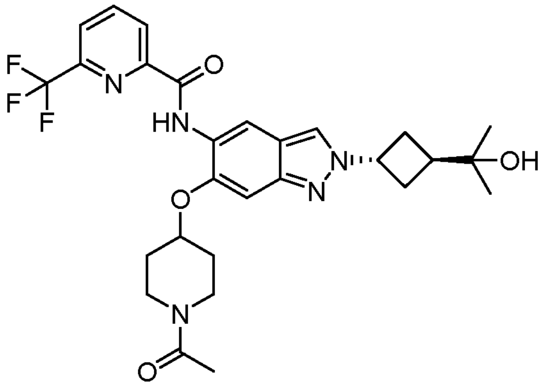

I53 I54

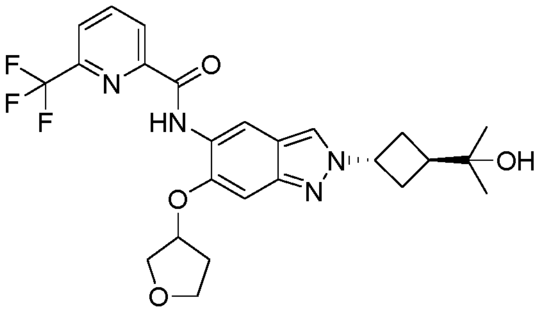

,

I55

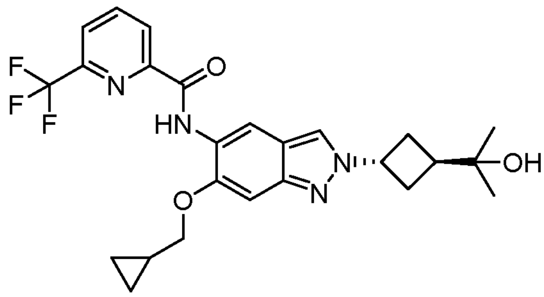

I56

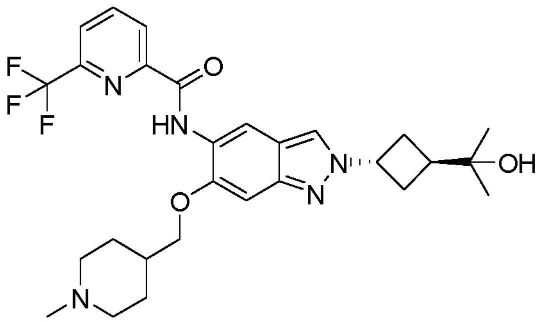

,

I57

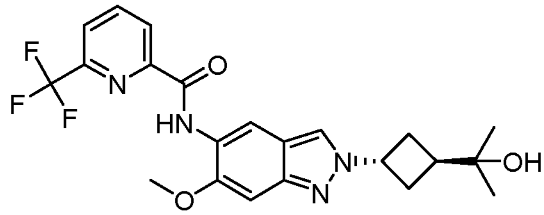

I58

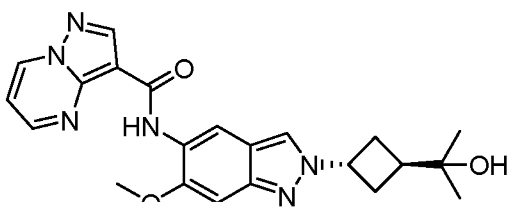

,

I59

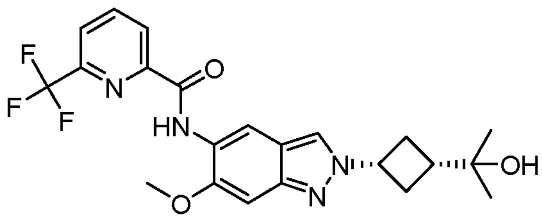

I60

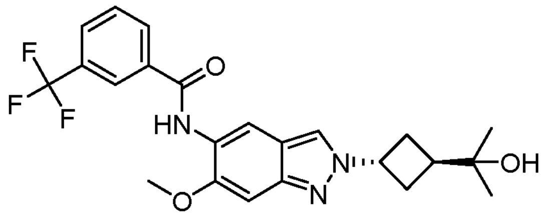

,

I61

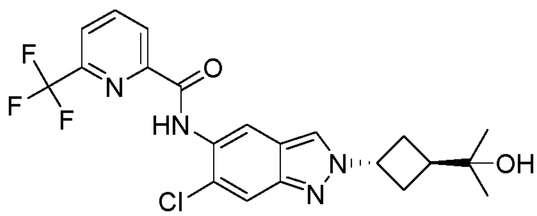

I62

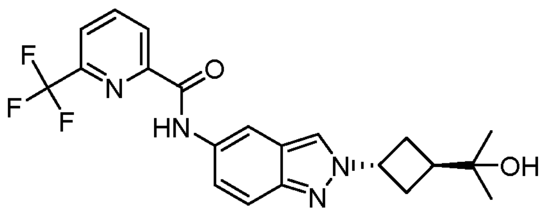

,

I63

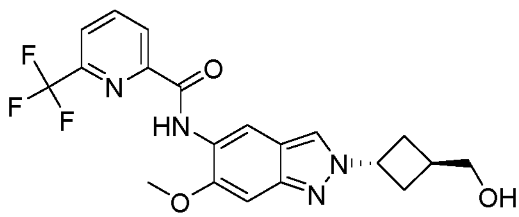

I64

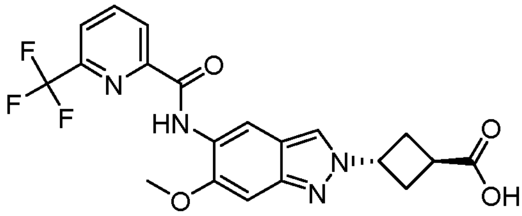,

I65

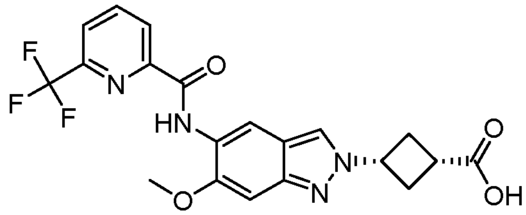

I66

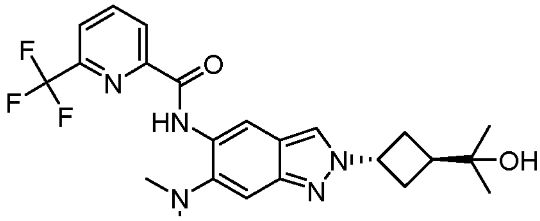,

I67

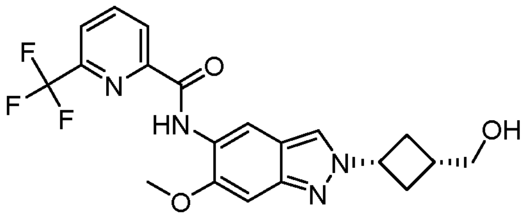

I68

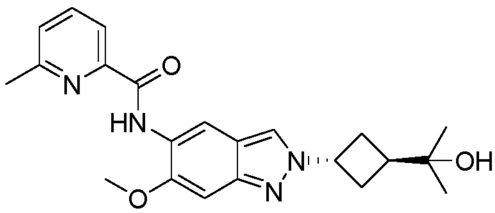,

I69

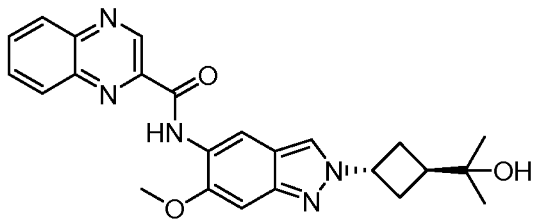

I70

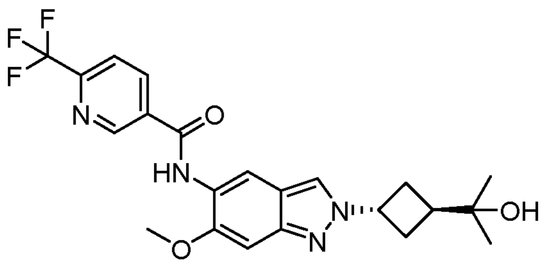,

I71

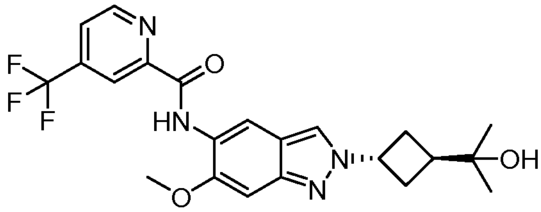

I72

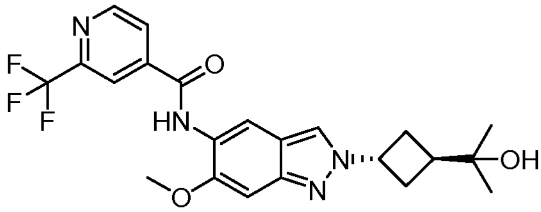,

I73

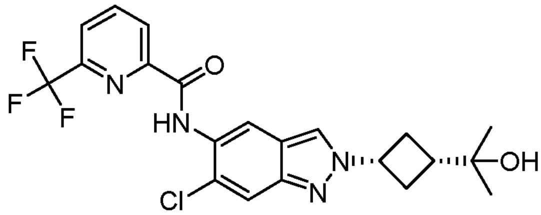

I74

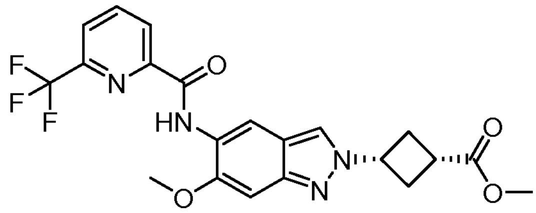,

I75

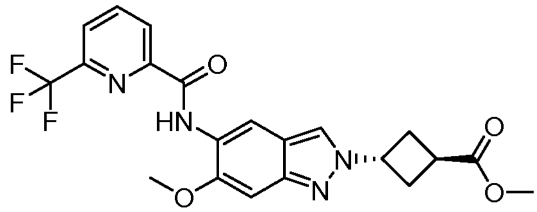

I76

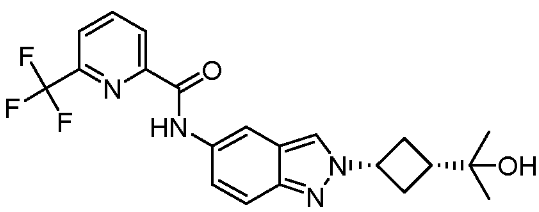,

I77

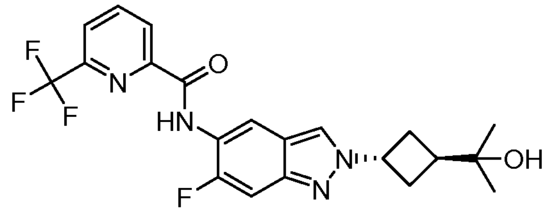

I78

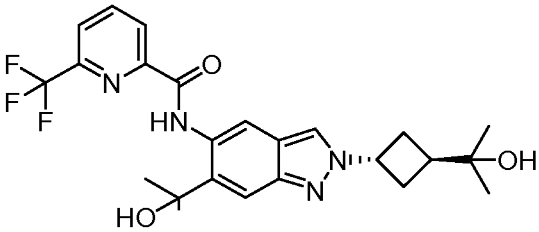,

I79

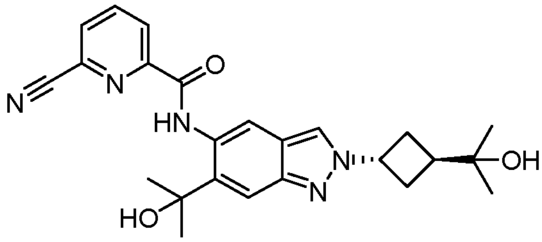

I80

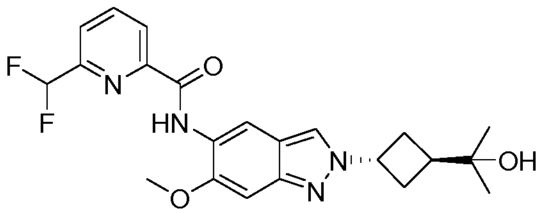,

I81

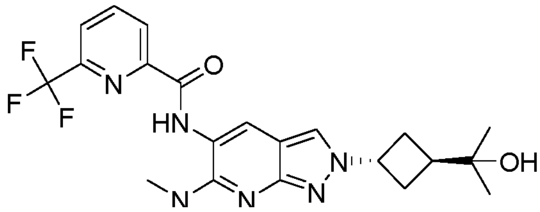

I82

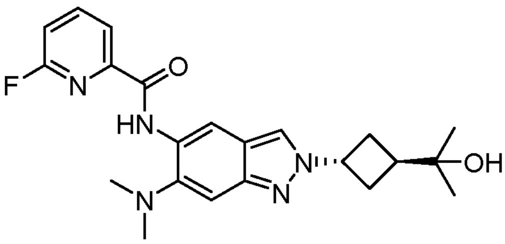,

I83

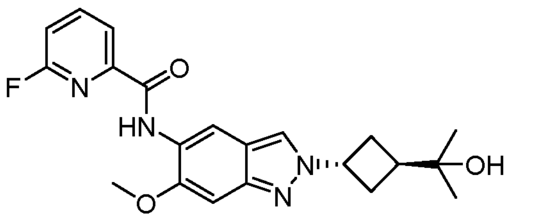

I84

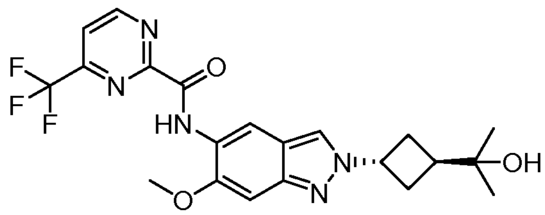

,

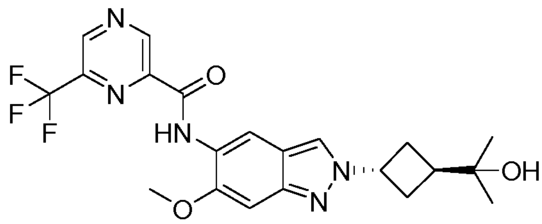

I85

I86

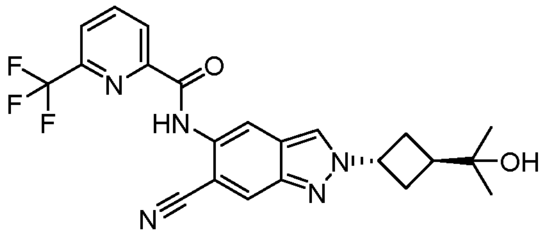

,

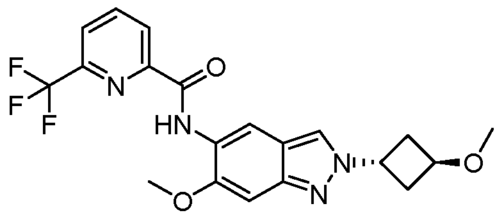

I87

I88

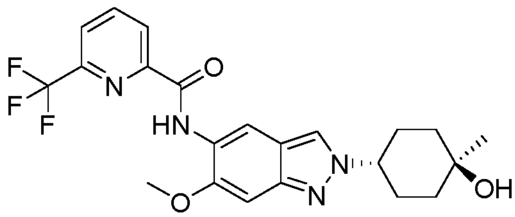

,

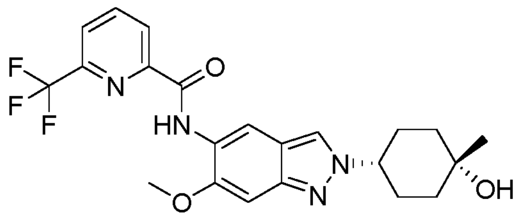

I89

I90

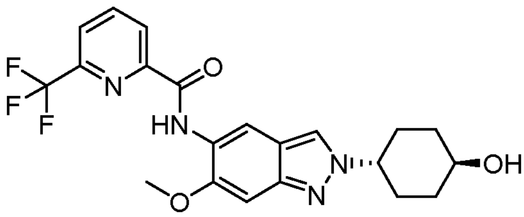

,

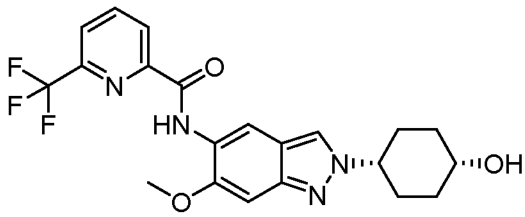

I91

I92

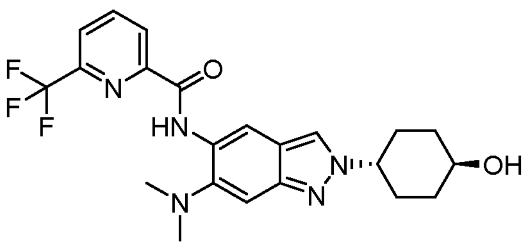

,

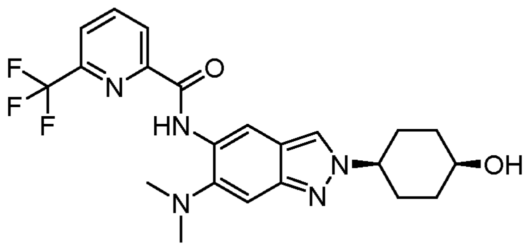

I93

I94

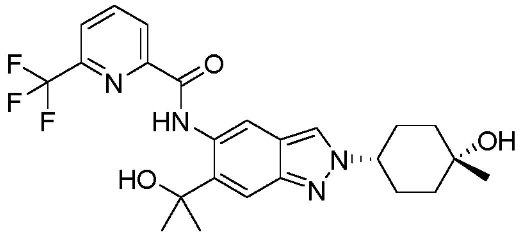,

I95

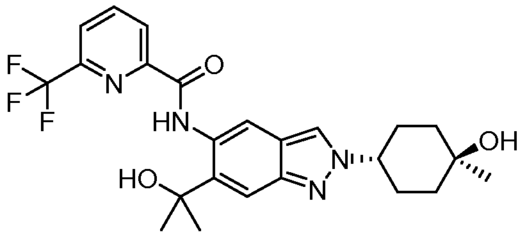

I96

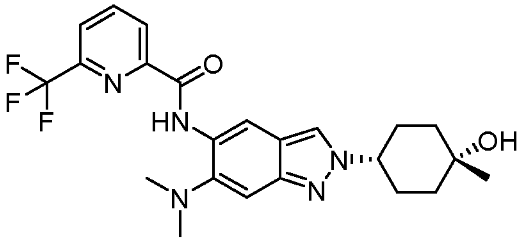,

I97

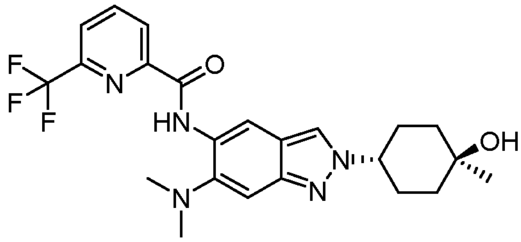

I98

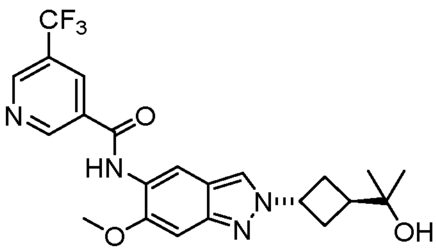,

I99

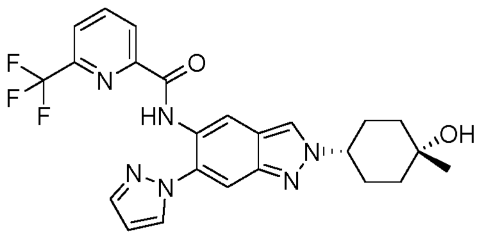

I100

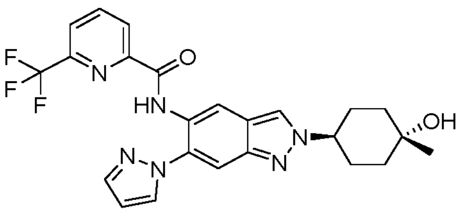,

I101

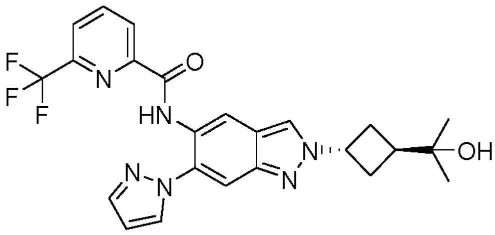

I102

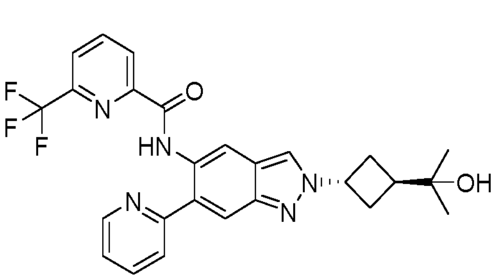,

I103

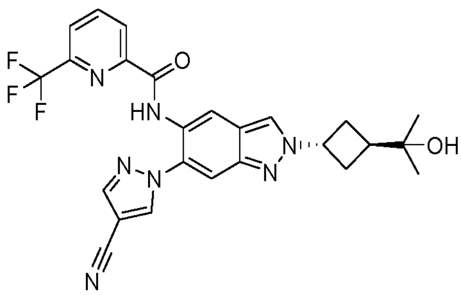

I104

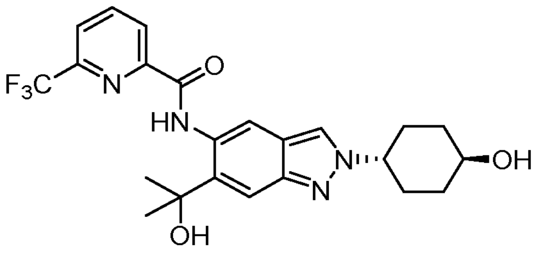

I105

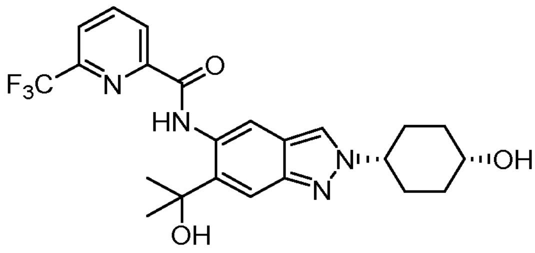

I106

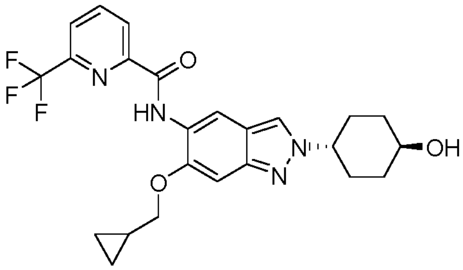

I107

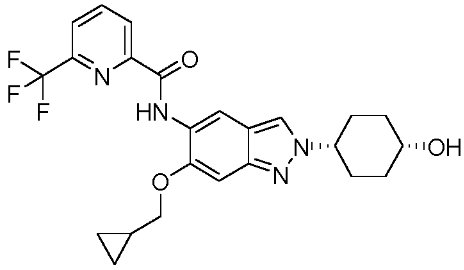

I108

In a second aspect of the present disclosure, provided is a pharmaceutical composition comprising: (i) at least one of the compound, or the cis-trans isomer, optical isome, racemate, pharmaceutically acceptable salt, prodrug, deuterated derivative, hydrate or solvate thereof according to the first aspect of the present disclosure; and optionally (ii) a pharmaceutically acceptable carrier.

In a third aspect of the present disclosure, provided is use of at least one of the compound, or the cis-trans isomer, optical isome, racemate, pharmaceutically acceptable salt, prodrug, deuterated derivative, hydrate or solvate thereof according to the first aspect of the present disclosure for preparing a pharmaceutical composition or formulation, wherein the pharmaceutical composition or formulation is used for:

(a) inhibiting interleukin-1 receptor-associated kinase; and/or
(b) preventing and/or treating a disease associated with interleukin-1 receptor-associated kinase.

In another preferred embodiment, the pharmaceutical composition or formulation is further used for: (c) inhibiting cytokine TNF-$\alpha$; and/or (d) preventing and/or treating a disease associated with cytokine TNF

In another preferred embodiment, the cytokine TNF includes cytokine TNF-$\alpha$.

In another preferred embodiment, the cytokine TNF includes cytokine TNF in a monocyte.

In another preferred embodiment, the monocyte is a peripheral blood monocyte.

In another preferred embodiment, the cytokine TNF includes cytokine TNF-$\alpha$ in a lipopolysaccharide-induced monocyte.

In another preferred embodiment, the disease associated with cytokine TNF is at least one selected from the group consisting of: inflammation, tumor (e.g., cancer), autoimmune disease, metabolic disorder, genetic disorder, immunodeficiency, condition associated with cell death, destructive bone disorder, thrombin-induced platelet aggregation, and liver disease.

In another preferred embodiment, the interleukin-1 receptor-associated kinase (IRAK) is selected from the group consisting of: IRAK1, IRAK2, IRAK-M and IRAK4, or a combination thereof.

In another preferred embodiment, the disease associated with interleukin-1 receptor-associated kinase is at least one selected from the group consisting of: inflammation, cancer, sepsis, autoimmune disease, sepsis, metabolic disorder, genetic disorder, immunodeficiency, condition associated with cell death, destructive bone disorder, thrombin-induced platelet aggregation, and liver disease.

In another preferred embodiment, the inflammation is at least one selected from the group consisting of: rheumatic arthritis, rheumatoid arthritis, systemic lupus erythematosus, conjunctivitis, osteoarthritis, chronic gouty arthritis, and synovitis.

In another preferred embodiment, the tumor is tumor formed in an organ selected from: liver, brain, kidney, vagina, ovary, breast, bladder and colon. In another preferred embodiment, the cancer is at least one selected from the group consisting of: liver cancer, brain cancer, kidney cancer, stomach cancer, vaginal cancer, ovarian cancer, breast

cancer, bladder cancer and colon cancer.

In a fourth aspect of the present disclosure, provided is an *in vitro* non-therapeutic and non-diagnostic method for inhibiting interleukin-1 receptor-associated kinase (IRAK), comprising: contacting interleukin-1 receptor-associated kinase (IRAK) or a cell expressing interleukin-1 receptor-associated kinase (IRAK) with at least one of the compound, or the cis-trans isomer, optical isome, racemate, pharmaceutically acceptable salt, prodrug, deuterated derivative, hydrate or solvate thereof according to the first aspect of the present disclosure, thereby inhibiting interleukin-1 receptor-associated kinase (IRAK).

In a fifth aspect of the present disclosure, provided is an *in vitro* non-therapeutic and non-diagnostic method for inhibiting cytokine TNF, comprising: contacting cytokine TNF or a cell expressing cytokine TNF with the compound, or the cis-trans isomer, optical isome, racemate, pharmaceutically acceptable salt, prodrug, deuterated derivative, hydrate or solvate thereof according to the first aspect of the present disclosure, thereby inhibiting cytokine TNF

In a sixth aspect of the present disclosure, provided is a method for inhibiting interleukin-1 receptor-associated kinase (IRAK) or preventing and/or treating a disease associated with interleukin-1 receptor-associated kinase (IRAK), comprising: administering at least one of the compound, or the cis-trans isomer, optical isome, racemate, pharmaceutically acceptable salt, prodrug, deuterated derivative, hydrate or solvate thereof according to the first aspect of the present disclosure to a subject in need.

In a seventh aspect of the present disclosure, provided is a method for inhibiting cytokine TNF-$\alpha$ or preventing and/or treating a disease associated with cytokine TNF, comprising: administering at least one of the compound, or the cis-trans isomer, optical isome, racemate, pharmaceutically acceptable salt, prodrug, deuterated derivative, hydrate or solvate thereof according to the first aspect of the present disclosure to a subject in need.

It should be understood that within the scope of the present disclosure, the above various technical features of the present disclosure and the technical features specifically described hereinafter (e.g., as in the examples) may be combined with each other to constitute a new or preferred technical solution. Due to limited space, such solutions are not described herein in detail.

## DETAILED DESCRIPTION

By extensive and intensive studies, the inventor developed a compound as an inhibitor of interleukin-1 receptor-associated kinase (particularly, IRAK4) and cytokine TNF-$\alpha$, or the cis-trans isomer, optical isome, racemate, pharmaceutically acceptable salt, prodrug, deuterated derivative, hydrate or solvate thereof, and uses of the same. It is found that the compound disclosed herein can effectively inhibit interleukin-1 receptor-associated kinase (particularly, IRAK4) and cytokine TNF-$\alpha$, and can be used in treatment of various diseases associated with interleukin-1 receptor-associated kinase (particularly, IRAK4) and cytokine TNF-$\alpha$. Based on the above findings, the present disclosure was completed.

Studies also suggest that the compounds disclosed herein have good inhibitory effects on IRAK4, and have excellent selectivity against other kinases. In animal studies, the compounds disclosed herein demonstrated excellent exposure and retention time in animals, excellent inhibitory effects on cytokine TNF-$\alpha$ in LPS-induced human PBMC, and superior efficacy in an *in vivo* model of Balb/c female mice with LPS-induced TNF-$\alpha$ release.

### Term Definition and Description

Unless defined otherwise, all technical and scientific terms used herein have the same meaning as commonly understood by those skilled in the art to which the subject matter belongs.

It is to be understood that substituents and substituted forms of the compounds disclosed herein can be selected by one of ordinary skill in the art to provide chemically stable compounds that can be synthesized by techniques known in the art as well as the methods set forth hereinafter. If substituted with more than one substituent, it is to be understood that the multiple substituents may be on the same carbon atom or on different carbon atoms, so long as a stable structure can be obtained.

As used herein, the term "substituted" or "substitution" refers to a replacement of a hydrogen atom in a group with a non-hydrogen group that satisfies the valence requirement and produces a chemically stable compound, i.e., a compound which does not spontaneously undergo a transformation such as cyclization and elimination.

Unless otherwise specified, each chiral carbon atom (chiral center) in all compounds disclosed herein may optionally be in the R configuration or the S configuration, or a mixture of the the R configuration and the S configuration.

As used herein, the terms "comprise", "contain" and "include" are used interchangeably and encompass technical solutions defined in the closed-ended language and those defined in the semi-closed-ended or open-ended language. In other words, the term encompasses the technical solutions with "consist of" and "consist essentially of".

As used herein, "R1", "$R_1$" and "$R^1$" carry the same meaning and can be used interchangeably. For other symbols such as R2, similar definitions are intended.

As used herein, "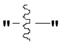" denotes the connection site of a group.

As used herein, the term "alkyl", alone or as part of another substituent, refers to a linear (i.e., unbranched) or branched saturated hydrocarbon group containing only carbon and hydrogen atoms, or a combination of linear and branched groups. Where an alkyl is preceded by a carbon atom number limitation (e.g., $C_1$-$C_{12}$ alkyl), it means that the alkyl contains 1-12 (e.g., 1, 2, 3, 4, 5, 6, 7, 8, 9, 10, 11 or 12) carbon atoms. For example, $C_1$-$C_6$ alkyl refers to alkyl groups containing 1-6 carbon atoms, with representative examples including but not limited to methyl, ethyl, propyl, isopropyl, butyl, isobutyl, *sec*-butyl, *tert*-butyl, or the like.

As used herein, the term "alkenyl", alone or as part of another substituent, refers to a linear or branched carbon chain group having at least one carbon-carbon double bond. Where an alkenyl is preceded by a carbon atom number limitation (e.g., $C_2$-$C_{10}$), it means that the alkenyl contains 2-10 carbon atoms. For example, $C_2$-$C_6$ alkenyl refers to alkenyl groups containing 2-6 carbon atoms, including ethenyl, propenyl, 1,2-butenyl, 2,3-butenyl, butadienyl, or the like.

As used herein, the term "alkynyl", alone or as part of another substituent, refers to an aliphatic hydrocarbon group having at least one carbon-carbon triple bond. The alkynyl can be linear or branched, or a combination thereof. Where an alkynyl is preceded by a carbon atom number limitation (e.g., $C_2$-$C_8$ alkynyl), it means that the alkynyl contains 2-8 carbon atoms. For example, the term "$C_2$-$C_6$ alkynyl" refers to a linear or branched alkynyl group having 2-6 carbon atoms, including ethynyl, propynyl, isopropynyl, butynyl, isobutynyl, *sec*-butynyl, *tert*-butynyl, or the like.

As used herein, the term "cycloalkyl", alone or as part of another substituent, refers to a group having a saturated or partially saturated monocyclic, bicyclic or polycyclic (fused, bridged or spiro) ring system. Where a cycloalkyl is preceded by a carbon atom number limitation (e.g., $C_3$-$C_{12}$), it means that the cycloalkyl contains 3-12 carbon atoms. In some preferred embodiments, the term "$C_3$-$C_8$ cycloalkyl" refers to a saturated or partially saturated monocyclic or bicyclic alkyl group having 3-8 carbon atoms, including cyclopropyl, cyclobutyl, cyclopentyl, cycloheptyl, or the like. "Spirocycloalkyl" refers to a bicyclic or polycyclic group in which a carbon atom (called spiro-atom) is shared among monocyclic rings, wherein those rings may contain one or more double bonds, but none of them has a fully conjugated π-electron system. "Fused cycloalkyl" refers to an all-carbon bicyclic or polycyclic group in which each ring shares a pair of adjacent carbon atoms with the other rings in the system, wherein one or more of the rings may contain one or more double bonds, but none of them has a fully conjugated π-electron system. "Bridged cycloalkyl" refers to an all-carbon polycyclic group in which any two rings share two carbon atoms that are not directly connected to each other, wherein these rings may contain one or more double bonds, but none of them has a fully conjugated π-electron system. Representative examples of cycloalkyl groups include, but are not limited to:

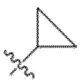
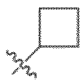
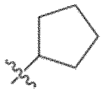
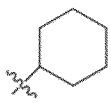
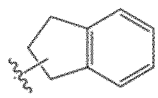
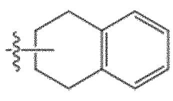
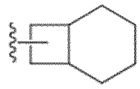

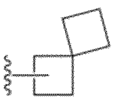
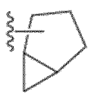
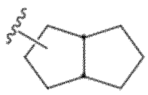
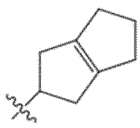
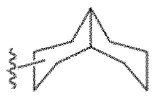
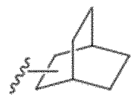

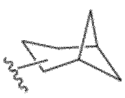
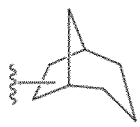

As used herein, the term "heterocycloalkyl", or heterocyclyl, alone or as part of another substituent, refers to a saturated or partially unsaturated monocyclic or polycyclic hydrocarbon substituent in which one or more ring atoms are selected from nitrogen, oxygen and sulfur. Non-limiting examples of monocyclic heterocyclyl include pyrrolidinyl, piperidinyl, piperazinyl, morpholinyl, thiomorpholinyl, homopiperazinyl, etc. Polycyclic heterocyclyl refers to spiro, fused, and bridged heterocyclyl. "Spiroheterocyclyl" refers to a polycyclic heterocyclyl group in which each ring shares an atom (called spiro-atom) with the other rings in the system, wherein one or more ring atoms are heteroatoms selected from nitrogen, oxygen and sulfur. "Fused heterocyclyl" refers to a polycyclic heterocyclyl group in which each ring shares a pair of adjacent carbon atoms with the other rings in the system, wherein one or more of the rings may contain one or more double bonds, but none of them has a fully conjugated π-electron system, and one or more ring atoms are heteroatoms selected from nitrogen, oxygen and sulfur. "Bridged heterocyclyl" refers to a polycyclic heterocyclyl group in which any two rings share two carbon atoms that are not directly connected to each other, wherein these rings may

contain one or more double bonds, but none of them has a fully conjugated π-electron system, and one or more ring atoms are heteroatoms selected from nitrogen, oxygen and sulfur, and the remaining ring atoms are carbon atoms. If both saturated and aromatic rings are present in the heterocyclyl (e.g., the saturated and aromatic rings are fused together), the site of connection to the parent moiety must be at the saturated ring. Note: where the site of connection to the parent moiety is at the aromatic ring, the group is referred to as heteroaryl, rather than heterocyclyl. It is to be understood herein that 3-12 membered heterocycloalkyl refers to a heterocycloalkyl group having 3-12 (e.g., 3, 4, 5, 6, 7, 8, 9, 10, 11 or 12) atoms, and similarly, 3-8 membered heterocycloalkyl refers to a heterocycloalkyl group having 3-8 atoms. Representative examples of heterocycloalkyl groups include, but are not limited to:

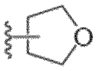 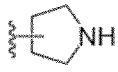 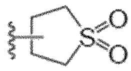 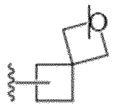 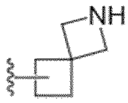 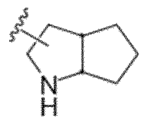 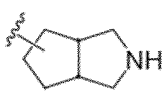

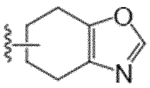 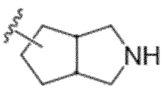 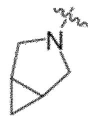 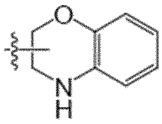 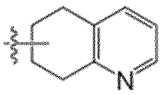 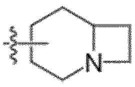

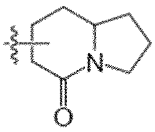 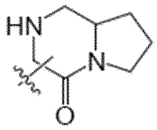 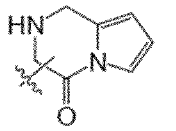 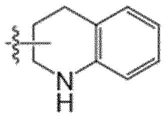

As used herein, the term "alkylenyl" is a divalent alkyl group (i.e., a divalent alkyl group formed by removing one hydrogen from an alkyl group). For example, $C_1$-$C_6$ alkylenyl refers to a divalent $C_1$-$C_6$ alkyl group as defined above. Preferably, the divalent radicals of the alkylenyl groups other than methylenyl are not located on the same carbon atom. Representative examples of alkylenyl groups include, but are not limited to:

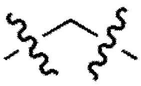, 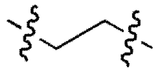, 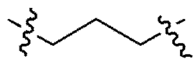,

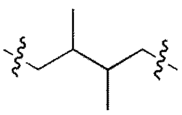,

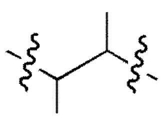, 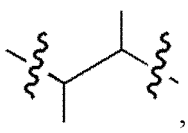, 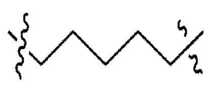

As used herein, the term "cycloalkylenyl" refers to a divalent cycloalkyl group, i.e., a divalent cycloalkyl group formed by removing one hydrogen from a cycloalkyl group, in which at least one of the divalent radicals is not located on an aromatic ring (e.g., an aromatic or heteroaromatic ring). For example, $C_3$-$C_{12}$ cycloalkylenyl refers to a divalent $C_3$-$C_{12}$ cycloalkyl group as defined above. Preferably, the divalent radicals of the cycloalkylenyl are not located on the same carbon atom. Representative examples of cycloalkylenyl groups include, but are not limited to:

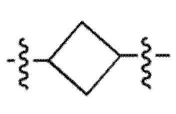, 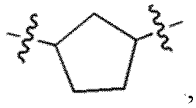, 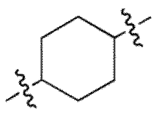, 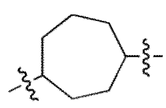.

As used herein, the term "heterocycloalkylenyl" refers to a divalent heterocycloalkyl group, i.e., a divalent cycloalkyl group formed by removing a hydrogen from a cycloalkyl group, in which at least one of the divalent radicals is not located on an aromatic ring (e.g., an aromatic or heteroaromatic ring). For example, 3-12 membered heterocycloalkylenyl refers to a divalent 3-12 membered heterocycloalkyl group as defined above. Preferably, the divalent radicals of the heterocycloalkylenyl are not located on the same carbon atom. Representative examples of heterocycloalkylenyl groups include, but are not limited to:

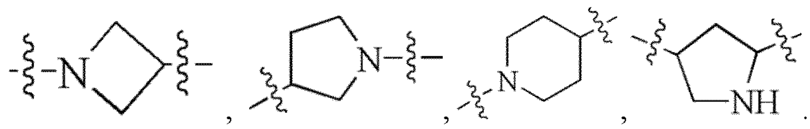, , , .

As used herein, the term "alkenylenyl" refers to a divalent alkenyl group, i.e., a divalent alkenyl group formed by removing one hydrogen from an alkenyl group. Preferably, the divalent radicals of the alkenylenyl are not located on the same carbon atom.

As used herein, the term "alkynylenyl" refers to a divalent alkynyl group, i.e., a divalent alkynyl group formed by removing one hydrogen from an alkynyl group. Preferably, the divalent radicals of the alkynylenyl are not located on the same carbon atom.

As used herein, the term "aryl", alone or as part of another substituent, refers to an all-carbon monocyclic or fused polycyclic group (i.e., rings that share a pair of adjacent carbon atoms) having a conjugated $\pi$-electron system. Where an aryl group is preceded by a carbon atom number limitation, such as $C_6$-$C_{20}$, it means that the aryl contains 6-20 carbon atoms, such as phenyl and naphthyl. The aryl ring may be fused to other cyclic groups (including saturated or unsaturated rings) without heteroatoms such as nitrogen, oxygen or sulfur, and the site of connection to the parent moiety must be at a carbon atom on the ring having the conjugated $\pi$-electron system. Representative examples of aryl groups include, but are not limited to:

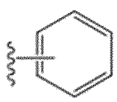 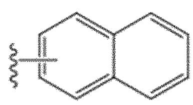 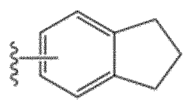 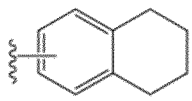 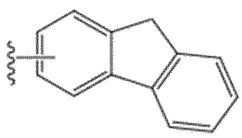.

As used herein, the term "heteroaryl", alone or as part of another substituent, refers to an aromatic heterocyclic system having one, two or more (preferably 1, 2, 3 or 4) heteroatoms that may be a monocyclic ring or polycyclic (bicyclic, tricyclic or polycyclic) ring that are fused together or connected covalently, wherein the heteroatoms herein include oxygen, sulfur and nitrogen. Where a heteroaryl group is preceded by a ring atom number limitation, it means the number of ring atoms including carbon atoms and heteroatoms in the heteroaryl group, for example, a 5-20 membered heteroaryl group means 5 to 20 ring atoms. For example, examples of 5-membered heteroaryl groups include, but are not limited to: pyrrole, furan, thiophene, imidazole, oxazole and thiazole; examples of 6-membered heteroaryl groups include, but are not limited to: pyridine, pyrazine, pyridazine and pyrimidine. The heteroaryl ring can be fused to an aryl, heterocycloalkyl or cycloalkyl ring, wherein the ring connected to the parent moiety is the heteroaryl ring. Representative examples of heteroaryl groups include, but are not limited to:

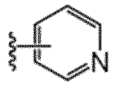 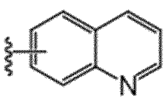 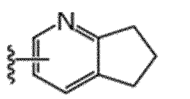 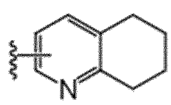 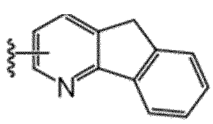 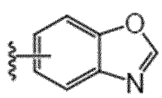

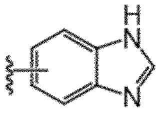 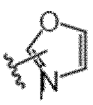 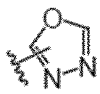 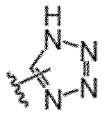 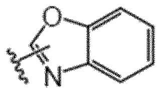 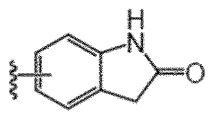 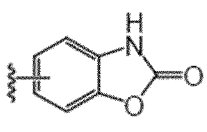

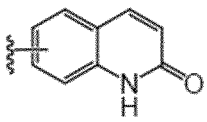 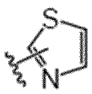 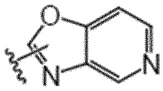 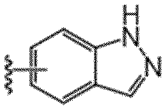 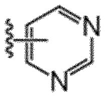 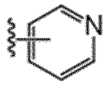 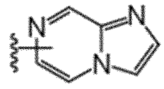

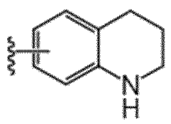 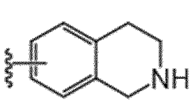 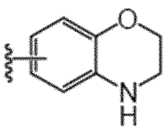 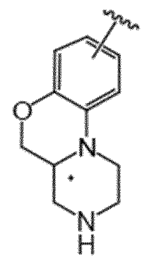 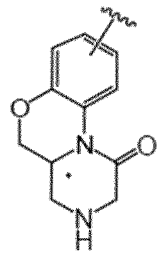 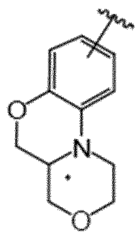

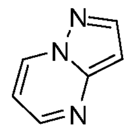

group,

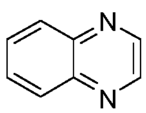

group,

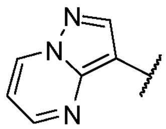 , 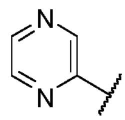 , 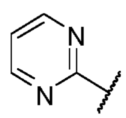 , 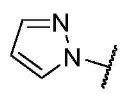 , 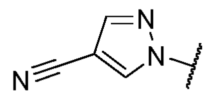 .

As used herein, the term "halogen", alone or as part of another substituent, refers to F, Cl, Br and I.

As used herein, the term "halo-", alone or as part of another substituent, means that one or more (preferably 1, 2, 3 or 4) hydrogen atoms in a group are substituted with halogen.

As used herein, the term "carboxyl", alone or as part of another substituent, refers to a structure of -COOH or R-COOH, wherein R is an alkylenyl group as defined herein. For example, the term "$C_1$-$C_8$ carboxyl" refers to a group having a structure of -($C_0$-$C_7$ alkylenyl)-COOH. Representative examples of carboxyl groups include, but are not limited to: -COOH, $-CH_2$-COOH, $-(CH_2)_2COOH$, $-(CH)CH_3COOH$, or the like.

The term "acyl" refers to an R-CO- group, wherein R is alkyl as defined herein above. For example, "$C_2$-$C_8$ acyl" refers to a group having a $C_1$-$C_7$ alkyl-CO- structure. Representative examples of acyl groups include, but are not limited to: $CH_3$-CO-, $C_2H_5$-CO-, $C_3H_8$-CO-, or the like.

As used herein, the term "ester group", alone or as part of another substituent, refers to a group having a structure of R-CO-O- or -CO-O-R, wherein R is an alkyl group as defined herein. For example, "$C_2$-$C_8$ ester group" refers to a group having a structure of $C_1$-$C_7$ alkyl-CO-O- or -CO-O-$C_1$-$C_7$ alkyl. Representative examples of ester groups include, but are not limited to: $CH_3COO$-, $C_2H_5COO$-, $C_3H_8COO$-, $(CH_3)_2CHCOO$-, $-COOCH_3$, $-COOC_2H_5$, $-COOC_3H_8$, or the like.

As used herein, the term "acylamino", alone or as part of another substituent, refers to a group having a structure of R-CO-NH- or -CO-NH-R, wherein R is an alkyl group as defined herein. For example, "$C_2$-$C_4$ acylamino" refers to a group having a structure of $C_1$-$C_3$ alkyl-CO-NH- or -CO-NH-$C_1$-$C_3$ alkyl. Representative examples of acylamino groups include, but are not limited to: $CH_3$CO-NH-, $C_2H_5$-CO-NH-, $C_3H_8$-CO-NH-, $(CH_3)_2$-CO-NH-, -CO-NH-$CH_3$, -CO-NH-$C_2H_5$, -CO-NH-$C_3H_8$, or the like.

As used herein, the terms "hydroxyalkyl" and "hydroxylalkyl", alone or as part of another substituent, can be used interchangeably and refers to an alkyl group substituted with one or more (preferably 1, 2 or 3) hydroxyl groups, the alkyl being defined herein above, provided that one carbon atom carries no more than one hydroxyl group. Where a hydroxyalkyl group is preceded by a carbon atom number limitation, e.g., $C_1$-$C_{12}$ hydroxyalkyl, it means that the alkyl group in the hydroxyalkyl group has 1-12 carbon atoms. Representative examples of hydroxyalkyl groups include, but are not limited to: hydroxymethyl, 2-hydroxyethyl, 2-hydroxypropyl, (2-hydroxyisopropyl), 2-hydroxy-2-methylpropyl, 3-hydroxypropyl, 1-(hydroxymethyl)-2-methylpropyl, 2-hydroxybutyl, 3-hydroxybutyl, 4-hydroxybutyl, 2,3-dihydroxy-propyl, 2-hydroxy-1-hydroxymethylethyl, 2,3-dihydroxybutyl, 3,4-dihydroxybutyl, 2-(hydroxymethyl)-3-hydroxypropyl, or the like.

As used herein, the terms "thiolalkyl", alone or as part of another substituent, refers to an alkyl group substituted with one or more (preferably 1, 2 or 3) thio groups, the alkyl being defined herein above, provided that one carbon atom carries no more than one thio group. Where a thiolalkyl group is preceded by a carbon atom number limitation, e.g., $C_1$-$C_{12}$ thiolalkyl, it means that the alkyl group in the thiolalkyl group has 1-12 carbon atoms. Representative examples of thiolalkyl groups include, but are not limited to: thiomethyl, 2-thioethyl, 2-thiopropyl, 3-thiopropyl, 1-(thiomethyl)-2-methylpropyl, 2-thiobutyl, 3-thiobutyl, 4-thiobutyl, 2,3-dithiopropyl, 2-thio-1-thiomethylethyl, 2,3-dithiobutyl, 3,4-dithiobutyl, 2-(thiomethyl)-3-thiopropyl, or the like.

As used herein, the terms "aminoalkyl", alone or as part of another substituent, refers to an alkyl group substituted with one or more (preferably 1, 2 or 3) amino groups, the alkyl being defined herein above, provided that one carbon atom carries no more than one amino group. Where an aminoalkyl group is preceded by a carbon atom number limitation, e.g., $C_1$-$C_{12}$ aminoalkyl, it means that the alkyl group in the aminoalkyl group has 1-12 carbon atoms. Representative examples of aminoalkyl groups include, but are not limited to: aminomethyl, 2-aminoethyl, 2-aminopropyl, 3-aminopropyl, 1-(aminomethyl)-2-methylpropyl, 2-aminobutyl, 3-aminobutyl, 4-aminobutyl, 2,3-diaminopropyl, 2-amino-1-aminomethylethyl, 2,3-diaminobutyl, 3,4-diaminobutyl, 2-(aminomethyl)-3-aminopropyl, or the like.

As used herein, the terms "hydroxycycloalkyl", alone or as part of another substituent, refers to a cycloalkyl group substituted with one or more (preferably 1, 2 or 3) hydroxyl groups, the cycloalkyl being defined herein above, provided that one carbon atom carries no more than one hydroxyl group. Where a hydroxycycloalkyl group is preceded by a carbon atom number limitation, e.g., $C_3$-$C_8$ hydroxycycloalkyl, it means that the cycloalkyl group in the hydroxycycloalkyl group has 3-8 carbon atoms. Representative examples of hydroxycycloalkyl groups include, but are not limited to: monohydroxycyclopropyl, dihydroxycyclopropyl, monohydroxycyclopentyl, dihydroxycyclopentyl, monohydroxycyclohexyl, trihydroxycyclohexyl, or the like.

As used herein, the terms "thiolcycloalkyl", alone or as part of another substituent, refers to a cycloalkyl group substituted with one or more (preferably 1, 2 or 3) thio groups, the cycloalkyl being defined herein above, provided that one carbon atom carries no more than one thio group. Where a thiolcycloalkyl group is preceded by a carbon atom number limitation, e.g., $C_3$-$C_8$ thiolcycloalkyl, it means that the cycloalkyl group in the thiolcycloalkyl group has 3-8 carbon atoms. Representative examples of thiolcycloalkyl groups include, but are not limited to: monothiocyclopropyl, dithiocyclopropyl, monothiocyclopentyl, dithiocyclopentyl, monothiocyclohexyl, trithiocyclohexyl, or the like.

As used herein, the terms "aminocycloalkyl", alone or as part of another substituent, refers to a cycloalkyl group substituted with one or more (preferably 1, 2 or 3) amino groups, the cycloalkyl being defined herein above, provided that one carbon atom carries no more than one amino group. Where an aminocycloalkyl group is preceded by a carbon atom number limitation, e.g., $C_3$-$C_8$ aminocycloalkyl, it means that the cycloalkyl group in the aminocycloalkyl group has 3-8 carbon atoms. Representative examples of thiolcycloalkyl groups include, but are not limited to: monoaminocyclopropyl, diaminocyclopropyl, monoaminocyclopentyl, diaminocyclopentyl, monoaminocyclohexyl, triaminocyclohexyl, or the like.

The term "haloalkyl" refers to an alkyl group substituted with one or more (preferably 1, 2, 3, or 4) halogens, the alkyl group being defined herein above. Where a haloalkyl group is preceded by a carbon atom number limitation, e.g., $C_1$-$C_{10}$ haloalkyl, it means that the alkyl group in the haloalkyl group has 1-10 carbon atoms. Representative examples of haloalkyl groups include, but are not limited to: chloromethyl, dichloromethyl, trichloromethyl, dichloroethyl, dichloropropyl, fluoromethyl, difluoromethyl, trifluoromethyl, pentafluoroethyl, heptafluoropropyl, difluorochloromethyl, dichlorofluoromethyl, difluoroethyl, difluoropropyl, or the like.

As used herein, the terms "halocycloalkyl", alone or as part of another substituent, refers to a cycloalkyl group substituted with one or more (preferably 1, 2 or 3) halogen atoms, the cycloalkyl being defined herein above. Where a halocycloalkyl group is preceded by a carbon atom number limitation, e.g., $C_3$-$C_8$ halocycloalkyl, it means that the cycloalkyl group in the halocycloalkyl group has 3-8 carbon atoms. Representative examples of halocycloalkyl groups

include, but are not limited to: difluorocyclopropyl, trifluorocyclopentyl, monofluorocyclohexyl, or the like.

As used herein, the term "alkoxy" or "alkyloxy", alone or as part of another substituent, refers to an R-O- group, wherein R is an alkyl as defined herein. Where an alkoxy group is preceded by a carbon number limitation, e.g., $C_1$-$C_6$ alkoxy or $C_1$-$C_6$ alkyloxy, it means that the alkyl group in the alkoxy or alkyloxy group has 1-6 carbon atoms. Representative examples of alkoxy or alkyloxy groups include, but are not limited to: methoxy, ethoxy, *n*-propoxy, *i*-propoxy, *t*-butoxy, or the like.

As used herein, the term "alkylthio", alone or as part of another substituent, refers to an R-S-group, wherein R is an alkyl as defined herein. Where an alkylthio group is preceded by a carbon number limitation, e.g., $C_1$-$C_6$ alkylthio, it means that the alkyl group in the alkylthio group has 1-6 carbon atoms. Representative examples of alkylthio groups include, but are not limited to: methylthiol, ethylthiol, *n*-propylthiol, isopropylthiol, *t*-butylthiol, or the like.

As used herein, the term "amino", alone or as part of another substituent, denotes $-NH_2$.

As used herein, the term "nitro", alone or as part of another substituent, denotes $-NO_2$.

As used herein, the term "cyano", alone or as part of another substituent, denotes -CN.

As used herein, the terms "methyltetrahydropyridylmethyl" and "(methyltetrahydropyridinyl)methyl" are used interchangeably.

According to the present specification, it is to be construed that all substituents are unsubstituted, unless explicitly described as "substituted" herein. The term "substituted" means that one or more hydrogen atoms on a specific group are substituted with a specific substituent. Specific substituents are those described correspondingly in the preceding text or as present in the examples. Unless otherwise specified, an optionally substituted group may have substituents selected from a specific group at any substitutable positions of the group. The substituents may be the same or different at the positions. A cyclic substituent, such as a heterocyclic group, may be connected to another ring, such as a cycloalkyl group, to form a bicyclic spiro ring system, i.e., the two rings share one carbon atom. It will be understood by those skilled in the art that combinations of substituents contemplated by the present disclosure are those stable or chemically available.

## Active Ingredient

As used herein, the terms "compound of the present disclosure", "compound of formula I of the present disclosure", and the like, are used interchangeably to refer to a compound of formula I, an isomer (including cis-trans isomers, optical isomers) thereof, a racemate thereof, a pharmaceutically acceptable salt thereof, a prodrug thereof, a deuterated derivative thereof, a hydrate thereof, or a solvate thereof. It is to be understood that the term also encompasses mixtures of the above components, and in the compound of formula I, if a chiral carbon atom is present, the chiral carbon atom may be in the R configuration or the S configuration, or a mixture of them.

The term "pharmaceutically acceptable salt" refers to salts formed by the compound disclosed herein with acids or bases that are suitable for use as medicaments. Pharmaceutically acceptable salts include inorganic and organic salts. One sort of preferred salts are those formed by the compound of the present disclosure and acids. Suitable acids for forming salts include, but are not limited to: inorganic acids such as hydrochloric acid, hydrobromic acid, hydrofluoric acid, sulfuric acid, nitric acid, and phosphoric acid; organic acids such as formic acid, acetic acid, propionic acid, oxalic acid, malonic acid, succinic acid, fumaric acid, maleic acid, lactic acid, malic acid, tartaric acid, citric acid, picric acid, methanesulfonic acid, toluenesulfonic acid, and benzenesulfonic acid; and acidic amino acids such as aspartic acid and glutamic acid. Another sort of preferred salts are metal salts of the compound of the present disclosure and bases. Suitable bases for forming salts include, but are not limited to: inorganic bases such as sodium hydroxide, potassium hydroxide, sodium carbonate, sodium bicarbonate and sodium phosphate; and organic bases such as ammonia, triethylamine and diethylamine.

The compound of formula I of the present disclosure can be converted into a pharmaceutically acceptable salt thereof by a conventional method. For example, a solution of an acid can be added to a solution of the compound, and after complete salt formation, the solvent is removed to give a salt of the compound of the present disclosure.

## General Synthetic Methods of the Compound

The present disclosure further provides a method for preparing the compound of the present disclosure.

A representative method for preparing the compound of formula I of the present disclosure comprises:

Process I:

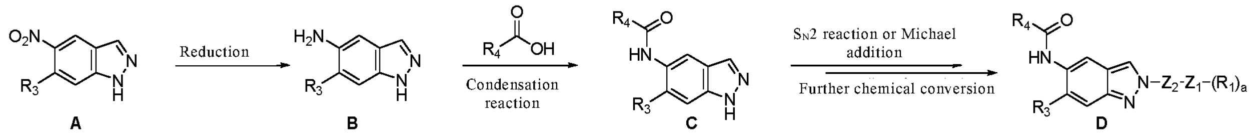


A B C D

The nitro group of compound A is reduced into an amino group to give compound B. The amino group of compound B is condensed with a carboxylic acid $R_4COOH$ to give compound C. Then compound C and an electrophile are subjected to an $S_N2$ reaction or Michael addition to give a structure D substituted with a nitrogen atom at position 2 of the indazole ring. It will be appreciated by those skilled in the art that further chemical conversions of the structure D substituted with a nitrogen atom at position 2 of the indazole ring can be made to yield a desired structure D.

In the above formulae, the groups are defined as above. Reagents and conditions for each step may be selected from those conventional in the art for implementing the preparation methods. Such selection may be made by those skilled in the art based on the acknowledge in the art after the structure of the compound of the present disclosure is disclosed. More specifically, the compounds of the present disclosure can also be conveniently prepared by optionally combining various synthetic methods described herein or known in the art, and such combinations can be easily determined by those skilled in the art to which the present disclosure pertains.

Interleukin-1 Receptor-Associated Kinase (IRAK)

Interleukin-1 receptor-associated kinases (IRAKs) include, but are not limited to: IRAK1 (interleukin-1 receptor-associated kinase-1), IRAK2 (interleukin-1 receptor-associated kinase-2), IRAK-M (interleukin-1 receptor-associated kinase-M) and IRAK4 (Interleukin-1 receptor-associated kinase-4), or a combination thereof.

IRAK (especially IRAK4) channel proteins have been found to be associated with diseases such as inflammation, tumor (e.g., cancer), sepsis, autoimmune disease, metabolic disorder, genetic disorder, immunodeficiency, condition associated with cell death, destructive bone disorder, thrombin-induced platelet aggregation and liver disease, and IRAK (especially IRAK4) is a target for treating such diseases.

Preferably, the inflammation includes, but is not limited to: rheumatic arthritis, rheumatoid arthritis, systemic lupus erythematosus, conjunctivitis, osteoarthritis, chronic gouty arthritis and synovitis.

Preferably, the cancer includes, but is not limited to: liver cancer, brain cancer, kidney cancer, stomach cancer, vaginal cancer, ovarian cancer, breast cancer, bladder cancer and colon cancer.

Cytokine TNF

TNF is abbreviated from tumor necrosis factor. TNF includes, but is not limited to: TNF-$\alpha$.

It has been demonstrated that the cytokine TNF, such as TNF-$\alpha$, is associated with diseases such as inflammation, tumor (e.g., cancer), autoimmune disease, metabolic disorder, genetic disorder, immunodeficiency, condition associated with cell death, destructive bone disorder, thrombin-induced platelet aggregation and liver disease. Cytokine TNF-$\alpha$ is a target for treating such diseases.

Use

The present disclosure further provides a method for inhibiting interleukin-1 receptor-associated kinase (especially IRAK4) and/or cytokine TNF-$\alpha$ and a method for preventing and/or treating a disease associated with interleukin-1 receptor-associated kinase (especially IRAK4) and/or cytokine TNF-$\alpha$ with the compound of the present disclosure.

The compound disclosed herein can be used for inhibiting interleukin-1 receptor-associated kinase (especially IRAK4) and/or cytokine TNF-$\alpha$, and further preventing or treating diseases associated with interleukin-1 receptor-associated kinase (especially IRAK4) and/or cytokine TNF-$\alpha$.

In the present disclosure, it is to be understood that a disease associated with interleukin-1 receptor-associated kinase (especially IRAK4) refers to a disease that is prevented and/or treated by inhibiting interleukin-1 receptor-associated kinase (especially IRAK4). The disease associated with cytokine TNF-$\alpha$ refers to a disease that can be prevented and/or treated by inhibiting cytokine TNF-$\alpha$.

In the present disclosure, the term "prevention" refers to a process of preventing the onset of a disease and/or its attendant symptoms or protecting a subject from the disease. As used herein, "preventing" also includes delaying the onset of a disease and/or its attendant symptoms and reducing the risk of developing the disease in a subject.

In the present disclosure, the term "treatment" refers to any therapy of a disease in a mammal, including but not limited to: (a) suppressing the disease, i.e., slowing or blocking the progression of symptoms; (b) relieving the disease, i.e., causing regression of symptoms, and/or (c) alleviating or eliminating the disease and/or its attendant symptoms.

In a preferred embodiment, the present disclosure provides an *in vitro* non-therapeutic and non-diagnostic method for inhibiting interleukin-1 receptor-associated kinase (IRAK) and/or inhibiting cytokine TNF-α, comprising, for example: contacting interleukin-1 receptor-associated kinase (IRAK) and/or cytokine TNF-α or a cell expressing interleukin-1 receptor-associated kinase (IRAK) and/or cytokine TNF-α with the compound, or the cis-trans isomer, the optical isomer or the racemate thereof, the pharmaceutically acceptable salt thereof, the prodrug thereof, the deuterated derivative thereof, the hydrate thereof or the solvate thereof disclosed herein, thereby inhibiting interleukin-1 receptor-associated kinase (IRAK) and/or cytokine TNF-α.

The present disclosure further provides a method for inhibiting interleukin-1 receptor-associated kinase (IRAK) and/or inhibiting cytokine TNF-α, or preventing and/or treating a disease associated with interleukin-1 receptor-associated kinase (IRAK) and/or cytokine TNF-α, comprising: administering the compound, or the cis-trans isomer, the optical isomer or the racemate thereof, the pharmaceutically acceptable salt thereof, the prodrug thereof, the deuterated derivative thereof, the hydrate thereof or the solvate thereof disclosed herein to a subject in need.

Preferably, the subject includes human and non-human mammals (rodents, rabbits, monkeys, domestic animals, dogs, cats, etc.).

Compositions and Route of Administration

The present disclosure provides a composition for inhibiting interleukin-1 receptor-associated kinase (especially IRAK4) and/or cytokine TNF-α. The composition includes, but is not limited to: pharmaceutical compositions, food compositions, dietary supplements, beverage compositions, and the like.

Typically, the composition is a pharmaceutical composition, comprising: the compound of formula I, or the cis-trans isomer, the optical isomer or the racemate thereof, the pharmaceutically acceptable salt thereof, the prodrug thereof, the deuterated derivative thereof, the hydrate thereof or the solvate thereof of the present disclosure; and a pharmaceutically acceptable carrier.

In the composition of the present disclosure, the amount of the compound of formula I is a therapeutically effective amount, wherein "therapeutically effective amount" refers to an amount that produces a function or activity and is acceptable in humans and/or animals. It should be understood by those skilled in the art that the "therapeutically effective amount" may vary depending on the form of the pharmaceutical composition, the route of administration, the excipients used, the severity of the disease, and combination with other medications.

In the present disclosure, the dosage form of the pharmaceutical composition includes, but is not limited to oral formulations, injections, and topical formulations.

Representative forms include, but are not limited to: tablet, injection, infusion, ointment, gel, solution, microsphere and film.

The term "pharmaceutically acceptable carrier" refers to: one or more compatible solid, semi-solid, liquid or gel fillers, which are suitable for human or animal use and must be of sufficient purity and sufficiently low toxicity. "Compatible" herein refers to that the components of the pharmaceutical composition and the therapeutically active ingredient are admixed with each other without significantly diminishing the efficacy of the composition.

It is to be understood that, in the present disclosure, the carrier is not specified, and may be prepared by materials commonly used in the art, or by a conventional method, or may be commercially available. Examples of pharmaceutically acceptable carrier are cellulose and its derivatives (e.g., methylcellulose, ethylcellulose, hydroxypropyl methylcellulose, sodium carboxymethyl cellulose), gelatin, talc, solid lubricants (e.g., stearic acid, magnesium stearate), calcium sulfate, vegetable oils (e.g., soybean oil, sesame oil, peanut oil, olive oil), polyols (e.g., propylene glycol, glycerin, mannitol, sorbitol), emulsifiers (e.g., Tween), wetting agents (e.g., sodium lauryl sulfate), buffering agents, chelating agents, thickening agents, pH adjusting agents, transdermal enhancers, coloring agents, flavoring agents, stabilizers, antioxidants, preservatives, bacteriostats, pyrogen-free water, and the like.

Typically, in addition to the active ingredient, a liquid dosage form may contain, inert diluents commonly employed in the art such as water or other solvents, solubilizers and emulsifiers such as ethanol, isopropanol, ethyl carbonate, ethyl acetate, propylene glycol, 1,3-butanediol, dimethylformamide, and oils, especially cottonseed, groundnut, corn germ, olive, castor, and sesame oils or mixtures of such materials. In addition to such inert diluents, the composition may also comprise adjuvants such as wetting agents, emulsifier and suspending agents, and the like.

The formulation shall match the route of administration. The agents of the present disclosure may also be used in combination with (including before, during or after) other concomitant medications. In using the pharmaceutical composition or formulation, a safe and effective amount of the agent, typically at least about 10 μg/kg body weight, and in most cases no more than about 8 mg/kg body weight, preferably from about 10 μg/kg body weight to about 1 mg/kg body weight, is administered to a subject in need (e.g., human or non-human mammals). For determining a specific dosage, such factors as the route of administration, the health condition of the patient and the like will also be considered, which are well known to skilled physicians.

The prominent advantages of the present disclosure include that:

(a) the present disclosure provides a compound of formula I with novel structure and excellent inhibitory activity against interleukin-1 receptor-associated kinase and/or cytokine TNF-$\alpha$;
(b) the compound has excellent *in vivo* anti-inflammation and anti-tumor efficacy in preventing and treating diseases such as rheumatoid arthritis;
(c) the compound features low toxicity, higher activity and thus a wider safety window;
(d) the compound has good druggability; and
(e) the compound has excellent pharmacokinetic properties.

The present disclosure will be further illustrated with reference to the following specific examples. It should be understood that these examples are merely intended to illustrate the present disclosure rather than limit the scope of the present disclosure. Experimental procedures without specified conditions in the following examples, are generally conducted according to conventional conditions, or according to conditions recommended by the manufacturer. Unless otherwise indicated, percentages and parts are by weight.

The compounds 1, 2, 3, 4, 5, 6 and 6a, 7, 8, 9, 4-1, 4-2, etc. described in the following examples all refer to the compounds indicated using Arabic numerals in the reaction flowchart of the corresponding examples.

Example 1. Synthesis of Compound MY-004-017 (18)

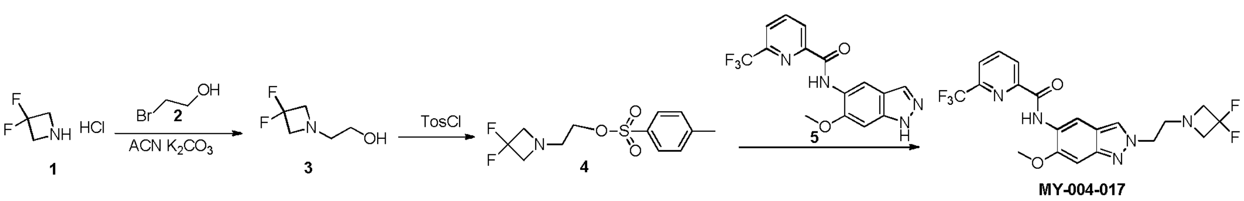


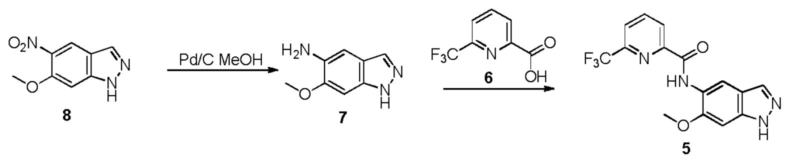


1. Preparation of compound 3 in Example 1

Compound 1 (1.5 g), compound 2 (1.74 g) and potassium carbonate (4.80 g) were dissolved in 40 mL of acetonitrile at room temperature. The reaction mixture was stirred at 80 °C overnight, and was filtered and concentrated after reaction completion to give a colorless oily crude product of 1.4 g in 87% yield. The crude product was used in the next reaction without further purification.

2. Preparation of compound 4 in Example 1

To a solution of compound 3 (1.0 g) and DMAP (1.08 g) in dichloromethane (20 mL) at room temperature were sequentially added p-toluenesulfonyl chloride (1.66 g) and triethylamine (1.18 g). The reaction mixture was stirred overnight at room temperature and concentrated under reduced pressure. The residue was purified on a silica gel column with dichloromethane/methanol=50:1 in gradient to give a colorless oil of 310 mg in 15% yield.

3. Preparation of compound 7 in Example 1

Compound 8 (1 g) and Pd/C (100 mg) were dissolved in methanol (100 mL). The reaction mixture was stirred overnight at room temperature in hydrogen atmosphere, filtered, and concentrated under reduced pressure to give a brown solid of 830 mg in 99% yield. The crude product was used directly in the next step without purification.

4. Preparation of compound 5 in Example 1

To a solution of compound 7 (830 mg) and compound 6 (1.08 g) in tetrahydrofuran (100 mL) were sequentially

added TBTU (1.97 g) and DIPEA (0.79 g) at room temperature. The reaction mixture was stirred overnight at room temperature and concentrated under reduced pressure. The residue was purified by silica gel column chromatography with dichloromethane/methanol = 100:1 in gradient to give a white solid of 1.0 g in 58% yield.

5. Synthesis of compound MY-004-017 (18) in Example 1

A solution of compound 5 (100 mg) in toluene (20 mL) was stirred at 110 °C for 15 min before compound 4 (130 mg) and DIPEA (230 mg) were sequentially added. The reaction mixture was stirred at 110 °C for 48 h and concentrated under reduced pressure. The residue was purified by preparative high performance liquid chromatography ($CH_3CN$:$H_2O$ (0.1% $NH_4HCO_3$)=5-95%, UV: 214 nm, flowrate: 15 mL/min) to give a white solid of 15 mg in 7% yield.

Compound 18: $^1$H NMR (400 MHz, DMSO-$d_6$): δ 10.5 (s, 1H), 8.69 (s, 1H), 8.47-8.40 (m, 2H), 8.32 (s, 1H), 8.22 (d, *J* = 6.4 Hz, 1H), 7.15 (s, 1H), 4.37 (s, 2H), 3.99 (s, 3H), 3.54 (t, *J* = 12.0 Hz, 4H), 3.14 (s, 2H). LCMS: Rt = 3.967 min, $[M+H]^+$= 456.1.

Example 2. Synthesis of Compounds MY-004-069 (158) and MY-004-040 (160)

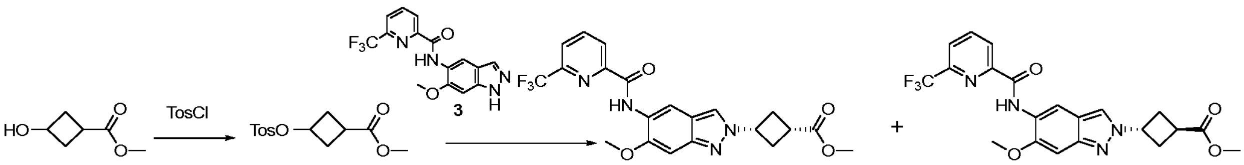


1 2 4-1 4-2

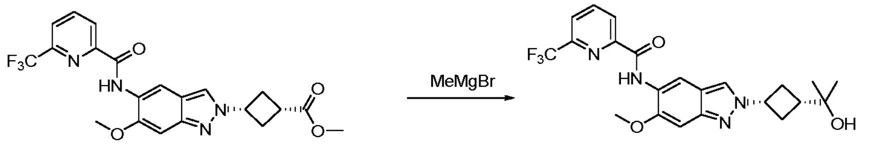


4-1 MY-004-040

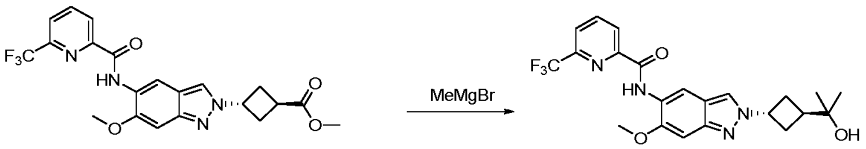


4-2 MY-004-069

1. Preparation of compound 2 in Example 2

Compound 1 (4.0 g), TosCl (7.62 g), DMAP (4.54 g) and triethylamine (5.57 mL) were sequentially added to 250 mL of dichloromethane at 0 °C. After that, the mixture was stirred at room temperature overnight, concentrated, and purified on a silica gel column with petroleum ether/ethyl acetate=3:1 in gradient to give a colorless oil of 4.9 g in 56% yield.

2. Preparation of compound 4-1 and compound 4-2 in Example 2

Compound 3 (200 mg), compound 2 (418 mg), and cesium carbonate (582 mg) were sequentially added to DMF (7 mL) at room temperature. The mixture was stirred at 80 °C for 16 h. At room temperature, the reaction was quenched by slowly adding water and stirring. Dichloromethane was added for extraction, and the organic phases were combined, dehydrated and concentrated. The residue was purified by preparative high performance liquid chromatography ($CH_3CN$:$H_2O$ (0.1% $NH_4HCO_3$)=5-95%, UV: 214 nm, flowrate: 15 mL/min) to give a white solid of compound 4-1 at Rt=8.65 min (28 mg, 27% yield) and a white solid of compound 4-2 at Rt=6.17 min (30 mg, 29% yield).

Compound 4-1 NMR: $^1$H NMR (400 MHz, $CDCl_3$): δ 10.71 (s, 1H), 8.81 (s, 1H), 8.50 (d, *J* = 8 Hz, 1H), 8.12 (t, *J* = 8 Hz, 1H), 7.93 (s, 1H), 7.86 (d, *J* = 7.6 Hz, 1H), 7.07 (s, 1H), 4.96-4.92 (m, 1H), 4.02 (s, 3H), 3.75 (s, 3H), 3.07-2.84 (m, 4H).

Compound 4-2 NMR: $^1$H NMR (400 MHz, $CDCl_3$): δ 10.71 (s, 1H), 8.81 (s, 1H), 8.50 (d, *J* = 8 Hz, 1H), 8.12 (t,

*J* = 8.4 Hz, 1H), 7.86 (t, *J* = 3.6 Hz, 2H), 7.09 (s, 1H), 5.29-5.21 (m, 1H), 4.03 (s, 3H), 3.78 (s, 3H), 3.35-3.28 (m, 1H), 3.13-3.05 (m, 2H), 2.88-2.82 (m, 2H).

3. Preparation of compounds MY-004-069 (158) and MY-004-040 (160) in Example 2

Compound 4-2 (30 mg) was dissolved in tetrahydrofuran (3 mL). At -30 °C, methylmagnesium bromide (0.67 mL, 0.67 mmol) was added slowly, and the mixture was stirred at -30 °C for 20 min and at room temperature for 0.5 h. The mixture was cooled to 0 °C and the reaction was quenched with saturated ammonium chloride. Ethyl acetate was added for extraction. The organic phases were combined, washed with water and saturated saline, and concentrated under reduced pressure. The residue was subjected to preparative high performance liquid chromatography ($CH_3CN$:$H_2O$ (0.1% $NH_4HCO_3$)=5-95%, UV: 214 nm, flowrate: 15 mL/min) to give a white solid of compound MY-004-069 (22 mg, 73% yield).

Compound 4-1 (28 mg) was dissolved in tetrahydrofuran (3 mL). At -30 °C, methylmagnesium bromide (0.62 mL, 0.62 mmol) was added slowly, and the mixture was stirred at -30 °C for 20 min and at room temperature for 0.5 h. The mixture was cooled to 0 °C and the reaction was quenched with saturated ammonium chloride. Ethyl acetate was added for extraction. The organic phases were combined, washed with water and saturated saline, and concentrated under reduced pressure. The residue was subjected to preparative high performance liquid chromatography ($CH_3CN$:$H_2O$ (0.1% $NH_4HCO_3$)=5-95%, UV: 214 nm, flowrate: 15 mL/min) to give a white solid of compound MY-004-040 (18 mg, 64% yield).

Compound of formula 160: $^1H$ NMR (400 MHz, $CDCl_3$): δ 10.71 (s, 1H), 8.81 (s, 1H), 8.50 (d, *J* = 8 Hz, 1H), 8.11 (t, *J* = 8 Hz, 1H), 7.89 (s, 1H), 7.86 (d, *J* = 8 Hz, 1H), 7.11 (s, 1H), 5.02 (t, *J* = 6 Hz, 1H), 4.03 (s, 3H), 2.76-2.73 (m, 2H), 2.67-2.61 (m, 3H), 1.21 (s, 6H). LCMS: Rt =2.412 min, $[M+H]^+$= 449.0.

Compound of formula 158: $^1H$ NMR (400 MHz, $CDCl_3$): δ 10.70 (s, 1H), 8.80 (s, 1H), 8.49 (d, *J* = 8 Hz, 1H), 8.11 (t, *J* = 8 Hz, 1H), 7.90 (s, 1H), 7.86 (d, *J* = 8 Hz, 1H), 7.06 (s, 1H), 4.84-4.78 (m, 1H), 4.01 (s, 3H), 2.64 (t, *J* = 8.4 Hz, 4H), 2.46 (br s, 1H), 2.31-2.24 (m, 1H), 1.21 (s, 6H). LCMS: Rt =2.475 min, $[M+H]^+$= 449.0.

Example 3. Synthesis of Compound MY-004-041 (112)

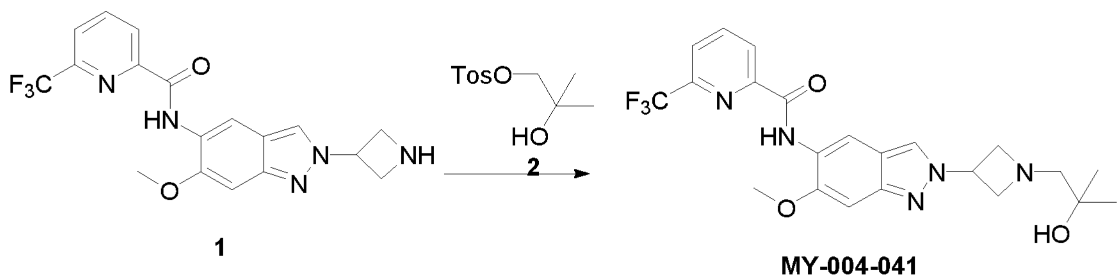

1

2

MY-004-041

1. Synthesis of compound MY-004-041 (112) in Example 3

Compound 1 (130 mg) and NaH (53 mg) were dissolved in DMF (5 mL). The mixture was stirred at room temperature for 1 h before compound 2 (325 mg) was added. The mixture was stirred overnight at 120 °C.After completion, the reaction was quenched by adding water. Ethyl acetate was added for extraction, and the organic phase was washed with saturated saline, dehydrated and concentrated under reduced pressure. The residue was purified by high performance liquid chromatography ($CH_3CN$:$H_2O$ (0.1% $NH_4HCO_3$)=20-60%, UV: 214 nm, flowrate: 15 mL/min) to give a white solid (19 mg, 12% yield).

Compound 112: $^1H$ NMR (400 MHz, DMSO): δ 10.51 (s, 1H), 8.69 (s, 1H), 8.47-8.41 (m, 3H), 8.22 (d, *J* = 7.6 Hz, 1H), 7.21(s, 1H), 5.23-5.21 (m, 1H), 4.11 (s, 1H), 3.99 (s, 3H), 3.87(t, *J* = 6.8 Hz, 2H), 3.64 (t, *J* = 6.8 Hz, 2H), 2.49 (s, 2H), 1.09 (s, 6H). LCMS: Rt = 3.714 min, [M+H]+ =464.1.

Example 4. Synthesis of Compound MY-004-042 (113)

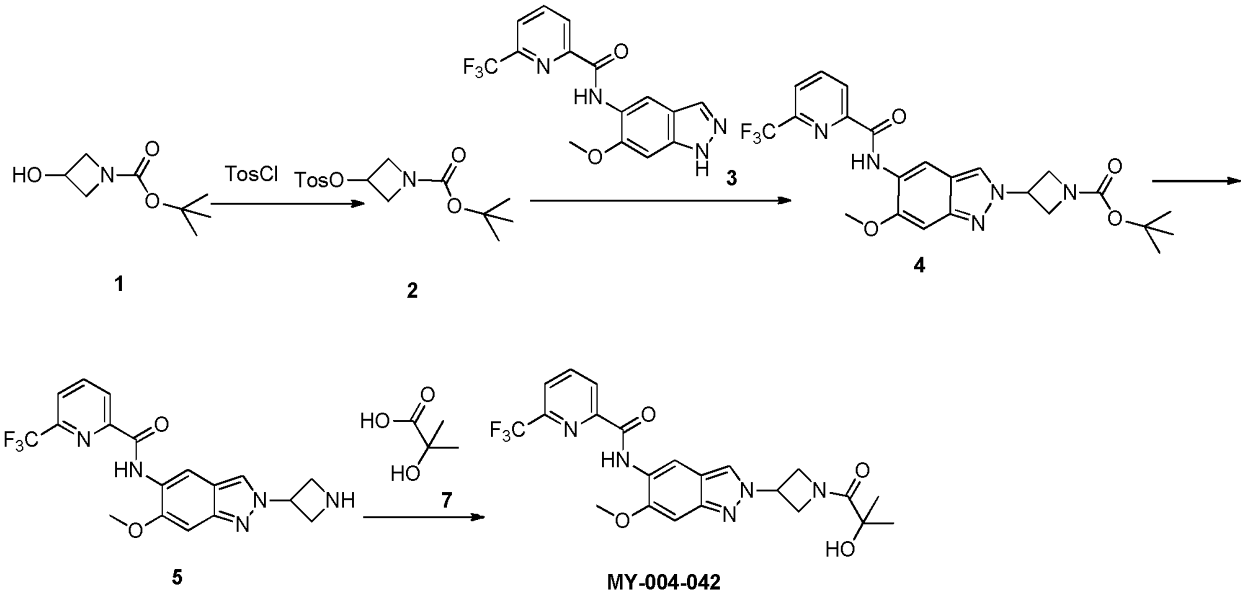


1. Preparation of compound 2 in Example 4

DMAP (2.4 g) and DIPEA (7.6 g) were sequentially added to a solution of compound 1 (3.4 g) and TosCl (4.1 g) in DCM (200 mL) at room temperature. The reaction mixture was stirred overnight at room temperature and concentrated under reduced pressure. The residue was purified on a silica gel column (petroleum ether/ethyl acetate=5:1) to give a colorless oil of 5.5 g in 86% yield.

2. Preparation of compound 4 in Example 4

To a solution of compound 2 (0.93 g) and compound 3 (0.64 g) in DMF (20 mL) at room temperature was added cesium carbonate (0.93 g). The reaction mixture was stirred at 80 °C overnight and concentrated under reduced pressure. The residue was purified on a silica gel column (PE/EA=1:1) to give a yellow solid of 290 mg in 31% yield.

3. Preparation of compound 5 in Example 4

Compound 4 (450 mg) was dissolved in HCl/EA (20 mL) at room temperature. The reaction mixture was stirred at room temperature for 1 h. After the reaction was completed, the mixture was concentrated under reduced pressure to give a white solid of 350 mg in 100% yield.

4. Synthesis of compound MY-004-042 (113) in Example 4

HOBT (45 mg), EDCI (64 mg) and DIPEA (99 mg) were sequentially added to a solution of compound 5 (100 mg) and compound 7 (40 mg) in DMF (5 mL) at room temperature. The reaction mixture was stirred overnight at room temperature and concentrated under reduced pressure. The residue was purified by preparative chromatography ($CH_3CN$:$H_2O$ (0.1% $NH_4HCO_3$)=20-60%, UV: 214 nm, flowrate 15 mL/min) to give a white solid of 27 mg in 22% yield.

Compound 113: $^1$H NMR (400 MHz, DMSO-$d_6$): δ 10.52 (s, 1H), 8.70 (s, 1H), 8.48-8.39 (m, 3H), 8.22 (d, *J* = 8.0 Hz, 1H), 7.24 (s, 1H), 5.48-5.46 (m, 1H), 5.19 (s, 1H), 4.89 (t, *J* = 8.0 Hz, 1H), 4.79-4.77 (m, 1H), 4.37(t, *J* = 8.8 Hz, 1H), 4.29-4.27 (m, 1H), 4.00 (s, 3H), 1.31 (d, *J* = 4.4Hz, 6H). LCMS: Rt = 3.801 min, $[M+H]^+$= 478.0.

Example 5. Synthesis of Compound MY-004-075 (159)

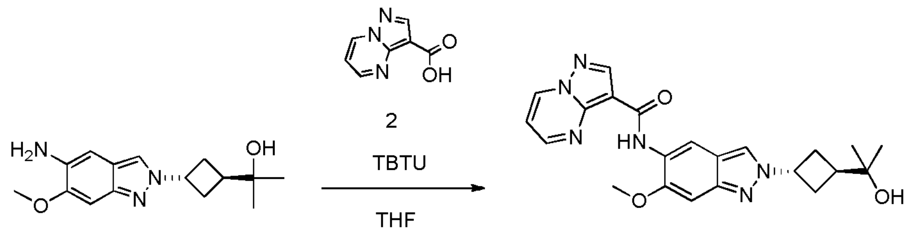


1

MY-004-075

1. Synthesis of compound MY-004-075 (159) in Example 5

Compound 1 (20 mg), compound 2 (15 mg), TBTU (35 mg) and DIEA (37 mg) were sequentially added to 7 mL of THF at room temperature. The mixture was stirred at room temperature overnight and concentrated. The residue was subjected to preparative high performance liquid chromatography ($CH_3CN:H_2O$ (0.1% $NH_4HCO_3$)=5-95%, UV: 214 nm, flowrate 15 mL/min) to give a white solid of 6 mg in 20% yield.

Compound 159: $^1$H NMR (400 MHz, $CDCl_3$): δ 10.46 (s, 1H), 8.83-8.74 (m, 4H), 7.88 (s, 1H), 7.11 (s, 1H), 7.06-7.03 (m, 1H), 5.03-4.99 (m, 1H), 4.05 (s, 3H), 2.77-2.72 (m, 2H), 2.67-2.60 (m, 3H), 1.25 (s, 6H). LCMS: Rt = 3.827 min, $[M+H]^+$= 421.2.

Example 6. Synthesis of Compound MY-004-079 (161)

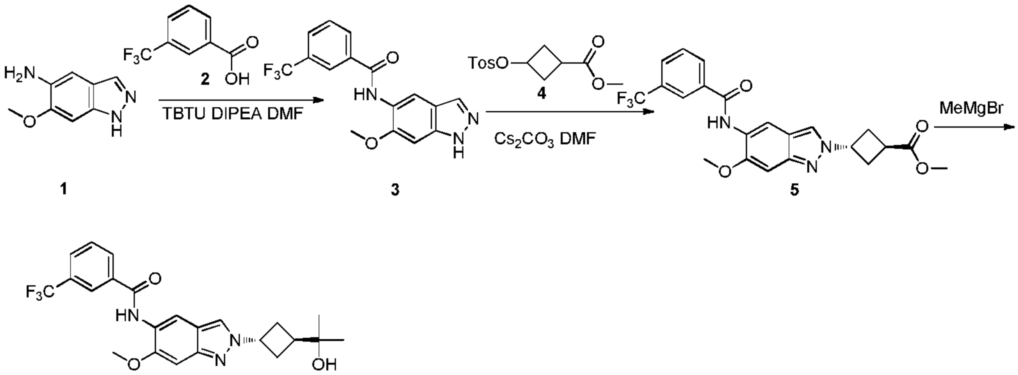


1 3 5

MY-004-079

1. Synthesis of compound 3 in Example 6

Compound 1 (800 mg), Compound 2 (1.03 g), TBTU (1.88 g) and DIEA (758 mg) were sequentially added to 10 mL of DMF at 0 °C. After the addition, the mixture was stirred at room temperature overnight, concentrated and purified on a silica gel column (dichloromethane/methanol=100:1) to give a colorless oil of 1.04 g in 63% yield.

2. Synthesis of compound 5 in Example 6

Compound 3 (500 mg), compound 4 (636 mg), and cesium carbonate (1.45 g) were sequentially added to DMF (50 mL) at room temperature. The mixture was stirred at 80 °C for 16 h. At room temperature, the reaction was quenched by slowly adding water and stirring. Dichloromethane was added for extraction and the organic phases were combined, dehydrated and concentrated. The residue was purified by preparative high performance liquid chromatography ($CH_3CN:H_2O$ (0.1% $NH_4HCO_3$)=5-95%, UV: 214 nm, flowrate 15 mL/min) and by silica gel preparative thin layer chromatography (DCM/MeOH=20:1) to give a crude white solid product of compound 5 (28 mg, 7% yield).

3. Synthesis of compound MY-004-079 (161) in Example 6

The crude product of compound 5 (18 mg) was dissolved in tetrahydrofuran (4 mL). At -30 °C, methylmagnesium bromide (0.8 mL, 0.8 mmol) was added slowly, and the mixture was stirred at -30 °C for 20 min. At 0 °C, the reaction

was quenched with saturated ammonium chloride. Ethyl acetate was added for extraction. The organic phases were combined, washed with water and saturated saline, and concentrated under reduced pressure. The residue was subjected to preparative high performance liquid chromatography ($CH_3CN:H_2O$ (0.1% $NH_4HCO_3$)=5-95%, UV: 214 nm, flowrate: 15 mL/min) to give a white solid (5 mg, 18% yield).

Compound 161: $^1$H NMR (400 MHz, $CDCl_3$): δ 8.77 (s, 1H), 8.72 (s, 1H), 8.18 (s, 1H), 8.08 (d, *J* = 8 Hz, 1H), 7.90 (s, 1H), 7.82 (d, *J* = 8 Hz, 1H), 7.65 (t, *J* = 8 Hz, 1H), 7.12 (s, 1H), 5.04-5.00 (m, 1H), 4.01 (s, 3H), 2.79-2.71 (m, 2H), 2.68-2.59 (m, 3H), 1.23 (s, 6H). LCMS: Rt =10.000 min, $[M+H]^+$= 448.0.

Example 7. Synthesis of Compounds MY-004-080 (162) and MY-004-098 (174)

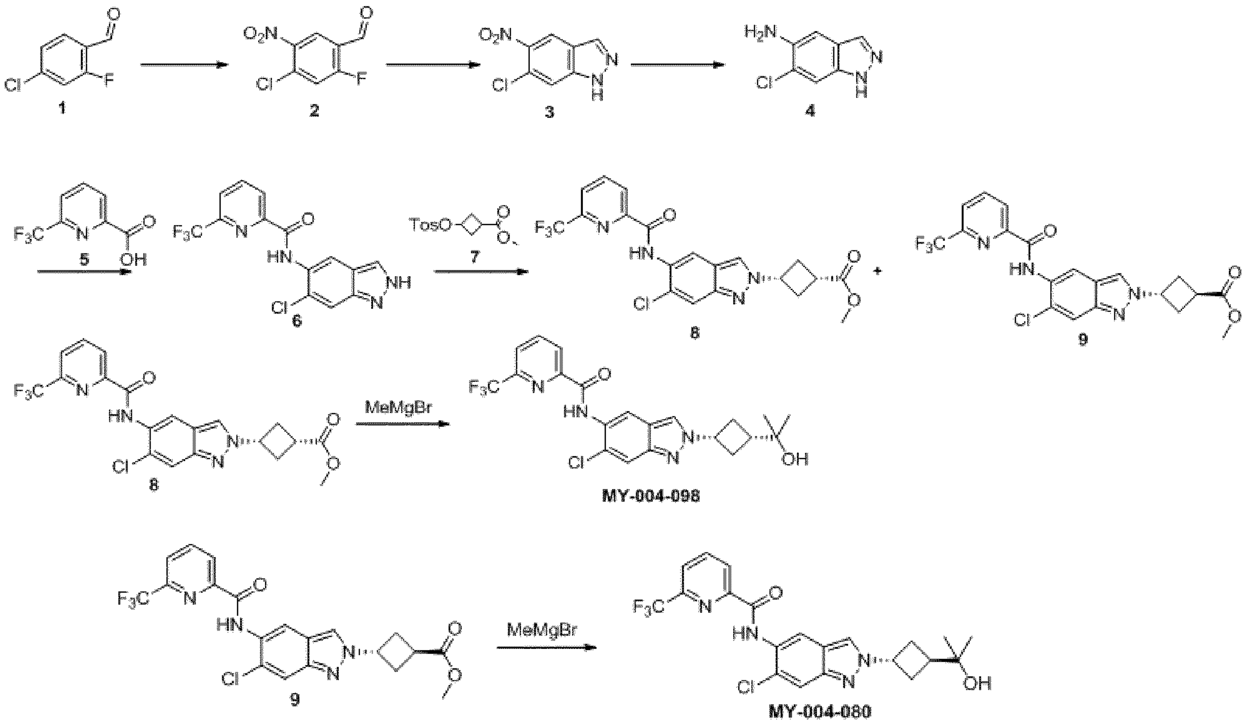


MY-004-098

MY-004-080

1. Synthesis of compound 2 in Example 7

At -15 °C, a mixture of 1.5 mL of sulfuric acid and 1.5 mL of nitric acid was added dropwise to a solution of compound 1 (1.58 g) in concentrated sulfuric acid (10 mL). The resultant mixture was stirred at -15 °C for 1 h. The reaction mixture was adjusted to a pH > 7 with saturated aqueous sodium bicarbonate before ethyl acetate was added for extraction. The organic phases were combined, concentrated under reduced pressure and purified on a silica gel column (PE/EA=10/1) to give a colorless oily product of 2 g in 99% yield.

2. Synthesis of compound 3 in Example 7

0.4 mL of hydrazine hydrate was added into a solution of compound 2 (0.2 g) in DMF (4 mL) at room temperature. The mixture was stirred at 100 °C overnight, cooled, added with 50 mL of ice water, stirred and filtered. The filter cake was dried to give an off-white solid of 150 mg in 78% yield without purification.

3. Synthesis of compound 4 in Example 7

Compound 3 (1.1 g) and Raney nickel were dissolved in methanol (100 mL) at room temperature. The reaction mixture was stirred overnight at 65 °C in hydrogen atmosphere, and was then cooled, filtered and dried by rotary evaporation to give a brown solid of 0.92 g in 99% yield without purification.

4. Synthesis of compound 6 in Example 7

Compound 4 (0.92 g), compound 5 (1.05 g) and EDCI (1.59 g) were dissolved in pyridine (50 mL) at room temperature and the reaction mixture was stirred overnight at room temperature. After the reaction, the mixture was dried by rotary evaporation and purified on a silica gel column (DCM/$CH_3OH$=100:1) to give a white solid of 1.5 g in 80% yield.

5. Synthesis of compound 8 and compound 9 in Example 7

Compound 6 (1.4 g), compound 7 (1.29 g) and $Cs_2CO_3$ (1.61 g) were dissolved in DMF (50 mL) at room temperature and the reaction mixture was stirred overnight at 80 °C. After the reaction, the mixture was cooled and dried by rotary evaporation. The residue was purified by preparative high performance liquid chromatography ($CH_3CN$:$H_2O$ (0.1% $NH_4HCO_3$)=5-95%, UV: 214 nm, flowrate: 15 mL/min) to give a white solid of compound 8 at Rt=9.83 min (120 mg, 6% yield) and a white solid of compound 9 at Rt=7.54 min (200 mg, 11% yield).

Compound 8: $^1$H NMR (400 MHz, DMSO-*d*$_6$): δ 10.52 (s, 1H), 8.63 (s, 1H), 8.56 (s, 1H), 8.48-8.39 (m, 2H), 8.23 (d, *J* = 7.6 Hz, 1H), 7.99 (s, 1H), 5.19-5.11 (m, 1H), 3.67 (s, 3H), 3.15-3.08 (m, 1H), 2.86-2.74 (m, 4H).

Compound 9: $^1$H NMR (400 MHz, DMSO-*d*$_6$): δ 10.53 (s, 1H), 8.63 (s, 1H), 8.60 (s, 1H), 8.45-8.42 (m, 2H), 8.24 (d, *J* = 7.6 Hz, 1H), 7.98 (s, 1H), 5.19-5.11 (m, 1H), 3.70 (s, 3H), 3.34-3.31 (m, 1H), 2.95-2.92 (m, 2H), 2.79-2.78 (m, 2H).

6. Synthesis of compound MY-004-098 (174) in Example 7

Compound 8 (80 mg) was dissolved in THF (8 mL) at -30 °C in nitrogen atmosphere. Methylmagnesium bromide (5.3 mL, 5.3 mmol) was added dropwise, and the reaction mixture was stirred at -30 °C for 30 min. After that, the reaction was quenched with saturated ammonium chloride. Ethyl acetate was added for extraction, and the organic phases were combined, concentrated under reduced pressure and purified by preparative high performance liquid chromatography ($CH_3CN$:$H_2O$ (0.1% $NH_4HCO_3$)=5-95%, UV: 214 nm, flowrate 15 mL/min) to give a white solid of 48 mg in 48% yield.

Compound 174: $^1$H NMR (400 MHz, DMSO-*d*$_6$): δ 10.52 (s, 1H), 8.63 (s, 1H), 8.50 (s, 1H), 8.48-8.39 (m, 2H), 8.23 (d, *J* = 7.2 Hz, 1H), 7.94 (s, 1H), 4.95-4.91 (m, 1H), 4.35 (s, 1H), 2.54-2.50 (m, 2H), 2.44-2.37 (m, 2H), 2.16-2.11 (m, 1H), 1.06 (s, 6H). LCMS: Rt = 9.945 min, $[M+H]^+$= 453.2.

7. Synthesis of compound MY-004-080 (162) in Example 7

Compound 9 (100 mg) was dissolved in THF (10 mL) at -30 °C in nitrogen atmosphere. Methylmagnesium bromide (6.6 mL, 6.6 mmol) was added dropwise, and the reaction mixture was stirred at -30 °C for 30 min. After that, the reaction was quenched with saturated ammonium chloride. Ethyl acetate was added for extraction, and the organic phases were combined, concentrated under reduced pressure and purified by preparative high performance liquid chromatography ($CH_3CN$:$H_2O$ (0.1% $NH_4HCO_3$)=30-95%, UV: 214 nm, flowrate 15 mL/min) to give a white solid of 79 mg in 79% yield.

Compound 162: $^1$H NMR (400 MHz, DMSO-*d*$_6$): δ 10.52 (s, 1H), 8.63 (s, 1H), 8.58 (s, 1H), 8.48-8.39 (m, 2H), 8.23 (d, *J* = 7.6 Hz, 1H), 7.97 (s, 1H), 5.10-5.07 (m, 1H), 4.45 (s, 1H), 2.61-2.55 (m, 4H), 2.46-2.44 (m, 1H), 1.11 (s, 6H). LCMS: Rt = 4.223 min, $[M+H]^+$= 453.2.

Example 8. Synthesis of Compound MY-004-105 (178)

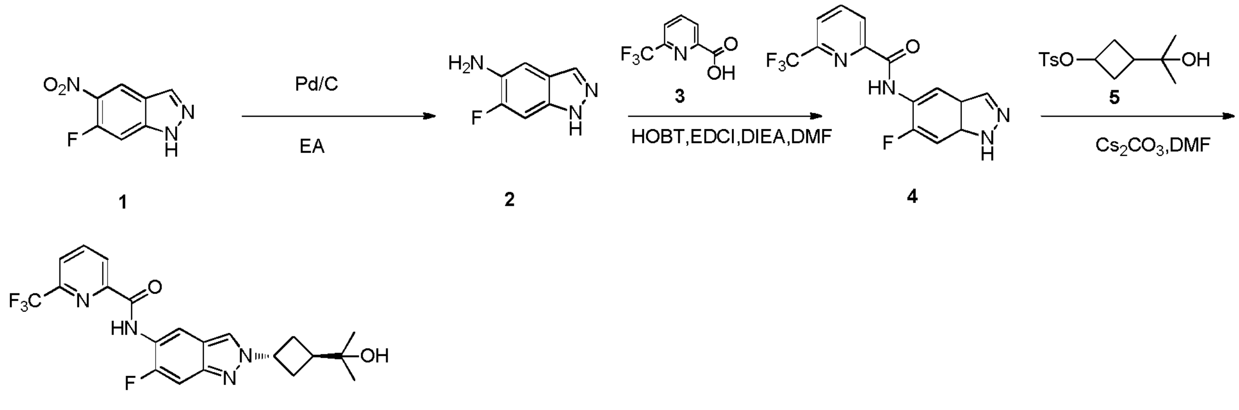


1 2 4

MY-004-105

1. Synthesis of compound 2 in Example 8

Compound 1 (500 mg) and palladium on carbon (250 mg) was sequentially added to 20 mL of EA at room temperature. The mixture was stirred overnight at room temperature, and filtered to remove palladium on carbon. The filtrate was dehydrated and concentrated to give a gray solid of 410 mg in 98% yield.

2. Synthesis of compound 4 in Example 8

Compound 2 (390 mg), compound 3 (508.5 mg), HOBT (527.5 mg), EDCI (743 mg) and DIEA (665.6 mg) were sequentially added to DMF (20 mL) at room temperature. The mixture was stirred at room temperature overnight. At room temperature, the reaction was quenched by slowly adding water and stirring. Ethyl acetate was added for extraction, and the organic phases were combined, dehydrated and concentrated. The residue was purified on a silica gel column (petroleum ether:ethyl acetate=1:1) to give a crude yellow solid of 500 mg in 57% yield.

3. Preparation of compound MY-004-105 (178) in Example 8

Compound 4 (120 mg), compound 5 (94.5 mg), and cesium carbonate (301 mg) were sequentially added to DMF (8 mL) and the mixture was stirred at 90 °C overnight. The reaction was quenched with water and ethyl acetate was added for extraction. The organic phases were combined, washed with water, and concentrated under reduced pressure. The residue was purified by preparative high performance liquid chromatography ($CH_3CN:H_2O$ (0.1% $NH_4HCO_3$)=5-95%, UV: 214 nm, flowrate: 15 mL/min) to give a white solid of compound MY-004-105 (28 mg, 19% yield).

Compound 178: $^1$H NMR (400 MHz, $CDCl_3$): δ 10.23 (s, 1H), 8.81 (d, *J* = 7.6 Hz, 1H), 8.51 (d, *J* = 7.6 Hz, 1H), 8.13 (t, *J* = 7.6 Hz, 1H), 7.98 (s, 1H), 7.89 (d, *J* = 7.2 Hz, 1H), 7.48 (d, *J* = 11.6 Hz, 1H), 5.08-5.03 (m, 1H), 2.79-2.72 (m, 2H), 2.70-2.59 (m, 3H), 1.25 (s, 6H). LCMS: Rt = 2.973 min, $[M+H]^+$ = 437.2.

Example 9. Synthesis of Compound MY-004-109 (179)

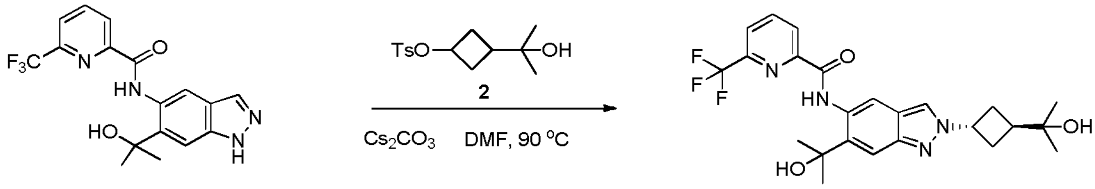


1 MY-004-109

1. Synthesis of compound MY-004-109 (179) in Example 9

Compound 1 (110 mg), compound 2 (103 mg), and cesium carbonate (245 mg) were sequentially added to DMF (7 mL). The mixture was stirred at 90 °C overnight. The reaction was quenched with water and ethyl acetate was added for extraction. The organic phases were combined, washed with water, and concentrated under reduced pressure.

The residue was purified by preparative high performance liquid chromatography ($CH_3CN:H_2O$ (0.1% $NH_4HCO_3$)=5-95%, UV: 214 nm, flowrate: 15 mL/min) and by silica gel preparative thin layer chromatography (DCM/MeOH=20:1) to give a white solid of compound MY-004-109 (20 mg, 14% yield).

Compound 179: $^1H$ NMR (400 MHz, DMSO-$d_6$): δ 12.38 (s, 1H), 8.71 (s, 1H), 8.46 (d, *J*= 2.8 Hz, 1H), 8.41 (s, 1H), 8.36 (t, *J* = 8 Hz, 1H), 8.17 (d, *J* = 7.6 Hz, 1H), 7.61 (s, 1H), 5.96 (s, 1H), 5.06-5.02 (m, 1H), 4.43 (s, 1H), 2.67-2.53 (m, 4H), 2.49-2.42 (m, 1H), 1.62 (s, 6H), 1.11 (s, 6H). LCMS: Rt = 3.702 min, $[M+H]^+$= 477.2.

Example 10. Synthesis of Compounds MY-004-081 (163) and MY-004-101 (177)

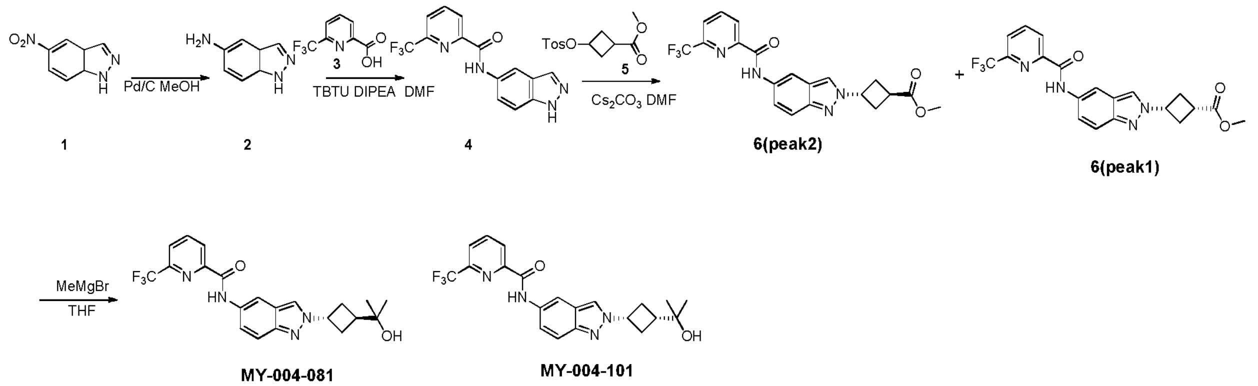


1 2 4 6(peak2) 6(peak1)

MY-004-081 MY-004-101

1. Synthesis of compound 2 in Example 10

Compound 1 (5 g) and Pd/C (500 mg, 10% w/w) were dissolved in methanol (50 mL) at room temperature. The reaction mixture was stirred at room temperature for 16 h in hydrogen atmosphere, filtered and dried by rotary evaporation to give a brown solid of 4.1 g in 99% yield without purification.

2. Synthesis of compound 4 in Example 10

Compound 2 (600 mg), Compound 3 (891 mg), TBTU (1.71 g) and DIEA (687 mg) were sequentially added to 8 mL of DMF at 0 °C. After the addition, the mixture was stirred at room temperature overnight, concentrated and purified on a silica gel column (dichloromethane/methanol=100:1) to give a yellow solid of 1.0 g in 74% yield.

3. Synthesis of compounds 6 and 6a in Example 10

Compound 4 (500 mg), compound 5 (694 mg), and cesium carbonate (1.59 g) were sequentially added to DMF (6 mL) at room temperature. The mixture was stirred at 80 °C for 16 h. At room temperature, the mixture was concentrated, and the residue was subjected to preparative high performance liquid chromatography ($CH_3CN:H_2O$ (0.1% $NH_4HCO_3$)=30-95%, UV: 214 nm, flowrate 15 mL/min) to give a white solid of compound 6a at Rt=8.92 min (150 mg, 22% yield) and compound 6 at Rt=7.31 min (41 mg, 6% yield).

Compound 6: $^1H$ NMR (400 MHz, DMSO-$d_6$): δ 10.37 (s, 1H), 8.48 (s, 1H), 8.41-8.34 (m, 2H), 8.29 (s, 1H), 8.18-8.16 (m, 1H), 7.67-7.64 (m, 1H), 7.59-7.57 (m, 1H), 5.34-5.30 (m, 1H), 3.34 (s, 3H), 2.97-2.90 (m, 2H), 2.79-2.73 (m, 2H), 2.42-2.37 (m, 1H).

Compound 6 a: $^1H$ NMR (400 MHz, DMSO-$d_6$): δ 10.39 (s, 1H), 8.45 (s, 1H), 8.40-8.33 (m, 2H), 8.29 (s, 1H), 8.18-8.17 (m, 1H), 7.67-7.64 (m, 1H), 7.59-7.56 (m, 1H), 5.13-5.09 (m, 1H), 3.66 (s, 3H), 3.14-3.07 (m, 1H), 2.83-2.75 (m, 4H).

4. Synthesis of compound MY-004-081 (163) in Example 10

Compound 6 (peak 2, 40 mg) was dissolved in tetrahydrofuran (4 mL). At -30°C, methylmagnesium bromide (1.4 mL, 1.4 mmol) was added slowly, and the mixture was stirred at -30 °C for 30 min. At 0 °C, the reaction was quenched with saturated ammonium chloride. Ethyl acetate was added for extraction. The organic phases were combined, washed with water and saturated saline, and concentrated under reduced pressure. The residue was subjected to preparative high performance liquid chromatography ($CH_3CN:H_2O$ (0.1% $NH_4HCO_3$)=30-95%, UV: 214 nm, flowrate: 15 mL/min)

to give a yellow solid of compound 163 (3.9 mg, 10% yield).

Compound 163: $^1$H NMR (400 MHz, $CDCl_3$): δ 9.83 (s, 1H), 8.52 (d, *J* = 8.0 Hz, 1H), 8.42 (s, 1H), 8.13 (t, *J* = 8.0 Hz, 1H), 7.89 (s, 1H), 7.87 (d, *J* = 8.0 Hz, 1H), 7.77 (d, *J* = 9.2 Hz, 1H), 7.37 (d, *J* = 10.4 Hz, 1H), 5.10-5.06 (m, 1H), 282-2.74 (m, 2H), 2.70-2.60 (m, 3H), 2.61 (s, 6H). LCMS: Rt = 3.956 min, $[M+H]^+$= 419.2.

5. Preparation of compound MY-004-101 (177) in Example 10

Compound 6a (peak 1, 31 mg) was dissolved in tetrahydrofuran (4 mL), before slowly adding methylmagnesium bromide (0.7 mL, 0.7 mmol) at -30 °C. The mixture was stirred at -30 °C for 30 min. The mixture was cooled to 0 °C and the reaction was quenched with saturated ammonium chloride. Ethyl acetate was added for extraction. The organic phases were combined, washed with water and saturated saline, and concentrated under reduced pressure. The residue was subjected to preparative high performance liquid chromatography ($CH_3CN$:$H_2O$ (0.1% $NH_4HCO_3$)=20-60%, UV: 214 nm, flowrate: 15 mL/min) to give a yellow solid of compound MY-004-101 (2.8 mg, 9% yield).

Compound 177: $^1$H NMR (400 MHz, $CDCl_3$): δ 8.48 (d, *J* = 8.0 Hz, 1H), 8.37 (s, 1H), 8.11 (t, *J* = 8.0 Hz, 1H), 8.01 (s, 1H), 7.86 (d, *J* = 8.0 Hz, 1H), 7.68 (d, *J* = 9.2 Hz, 1H), 7.37-7.34 (d, *J* = 10.4 Hz, 1H), 4.89-4.80 (m, 1H), 2.66-2.55 (m, 4H), 2.27-2.23 (m, 1H), 1.17 (s, 6H). LCMS: Rt = 3.692 min, $[M+H]^+$= 419.2.

Example 11. Synthesis of Compound MY-004-082 (164)

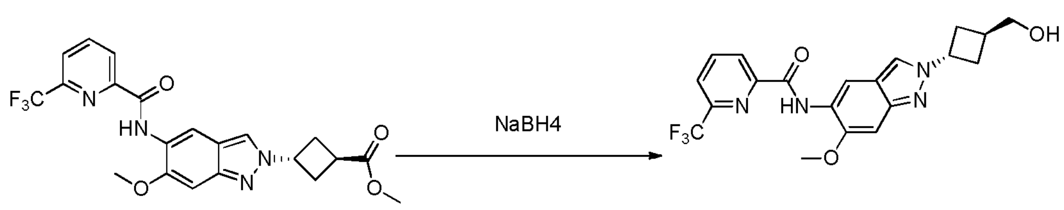


MY-004-100 MY-004-082

1. Synthesis of compound MY-004-082 (164) in Example 11

Sodium borohydride (25.3 mg) was added at 0 °C to a mixture of compound MY-004-100 (30 mg) in 0.5 mL of methanol and 4 mL of tetrahydrofuran. The mixture was stirred at room temperature overnight and concentrated. The residue was subjected to preparative high performance liquid chromatography ($CH_3CN$:$H_2O$ (0.1% $NH_4HCO_3$)=5-95%, UV: 214 nm, flowrate 15 mL/min) to give a white solid of 23 mg of compound MY-004-082 in 82% yield.

Compound 164: $^1$H NMR (400 MHz, DMSO-$d_6$): δ 10.51 (s, 1H), 8.68 (s, 1H), 8.47 (d, *J* = 7.6 Hz, 1H), 8.40 (t, *J* = 8 Hz, 1H), 8.37 (s, 1H), 8.21 (d, *J* = 6.8 Hz, 1H), 7.20 (s, 1H), 5.16-5.12 (m, 1H), 4.76 (t, *J* = 5.2 Hz, 1H), 3.98 (s, 3H), 3.56 (t, *J* = 6.0 Hz, 2H), 2.70-2.63 (m, 2H), 2.46-2.44 (m, 1H), 2.37-2.31 (m, 2H).

Example 12. Synthesis of Compounds MY-004-083 (165) and MY-004-091 (166)

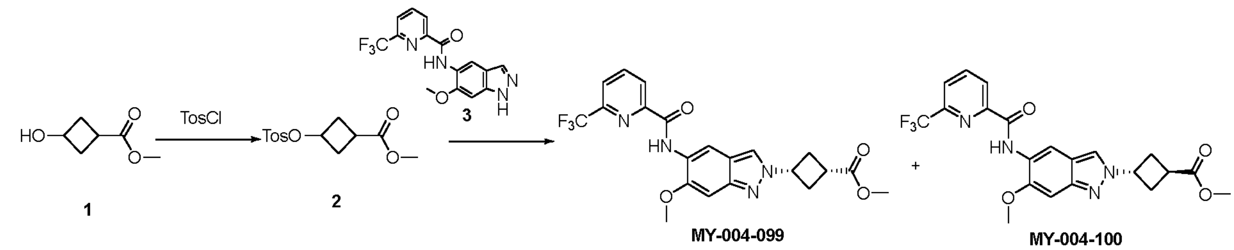


1 2 3 MY-004-099 + MY-004-100

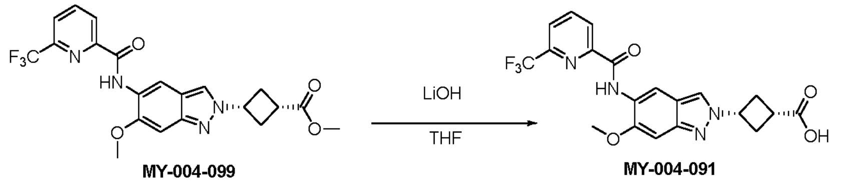


MY-004-099 MY-004-091

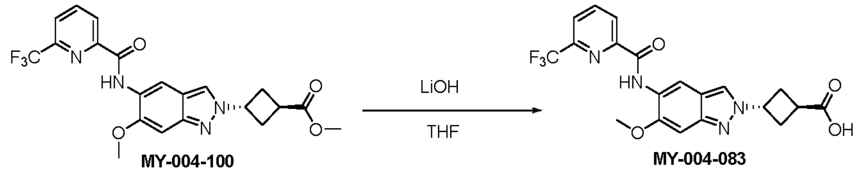


MY-004-100 MY-004-083

1. Synthesis of compound 2 in Example 12

Compound 1 (10 g), TosCl (19.06 g), DMAP (11.3 g) and triethylamine (21.5 mL) were sequentially added to 500 mL of dichloromethane at 0 °C. After that, the mixture was stirred at room temperature overnight, concentrated, and purified on a silica gel column (petroleum ether/ethyl acetate=5:1) to give of a colorless oil (16 g, 73.4% yield).

2. Synthesis of compounds MY-004-099 and MY-004-100 in Example 12

Compound 3 (6 g), compound 2 (7.31 g), and cesium carbonate (17.45 g) were sequentially added to DMF (300 mL) at room temperature. The mixture was stirred at 80 °C for 16 h. At room temperature, the reaction was quenched by slowly adding water and stirring. Ethyl acetate was added for extraction and the organic phases were combined, dehydrated and concentrated. The residue was subjected to preparative high performance liquid chromatography ($CH_3CN$:$H_2O$ (0.1% $NH_4HCO_3$)=30-95%, UV: 214 nm, flowrate 15 mL/min) to give a white solid of compound MY-004-099 at Rt=8.95 min (610 mg, 16% yield) and a yellow solid of compound MY-004-100 at Rt=6.64 min (520 mg, 14% yield).

Compound MY-004-099: $^1$H NMR (400 MHz, $CDCl_3$): δ 10.72 (s, 1H), 8.81 (s, 1H), 8.50 (d, *J* = 8 Hz, 1H), 8.12 (t, *J* = 7.6 Hz, 1H), 7.93 (s, 1H), 7.81 (d, *J* = 7.6 Hz, 1H), 7.07 (s, 1H), 4.96-4.92 (m, 1H), 4.03 (s, 3H), 3.75 (s, 3H), 3.07-2.85 (m, 5H).

Compound MY-004-100: $^1$H NMR (400 MHz, $CDCl_3$): δ 10.70 (s, 1H), 8.80 (s, 1H), 8.50 (d, *J* = 7.6 Hz, 1H), 8.11 (t, *J* = 7.6 Hz, 1H), 7.86-7.84 (m, 2H), 7.09 (s, 1H), 5.29-5.21 (m, 1H), 4.03 (s, 3H), 3.78 (s, 3H), 3.35-3.28 (m, 1H), 3.12-3.04 (m, 2H), 2.88-2.82 (m, 2H).

3. Preparation of compounds MY-004-083 (165) and MY-004-091 (166) in Example 12

Compound MY-004-099 (23 mg) and lithium hydroxide (4.9 mg) were added to tetrahydrofuran (3 mL)/water (1 mL) at room temperature. The mixture was stirred at room temperature overnight. The mixture was adjusted to pH 6 by slowly adding 1 N hydrochloric acid in an ice bath. Ethyl acetate was added for extraction, and the organic phases were combined, dehydrated and concentrated. The residue was subjected to preparative high performance liquid chromatography ($CH_3CN$:$H_2O$ (0.1% $NH_4HCO_3$)=5-95%, UV: 214 nm, flowrate: 15 mL/min) to give a white solid of compound MY-004-091 (16 mg, 72% yield).

Compound MY-004-100 (30 mg) and lithium hydroxide (6.5 mg) were added to tetrahydrofuran (3 mL)/water (1 mL) at room temperature. The mixture was stirred at room temperature overnight. The mixture was adjusted to pH 6 by slowly adding 1 N hydrochloric acid in an ice bath. Ethyl acetate was added for extraction, and the organic phases were combined, dehydrated and concentrated. The residue was subjected to preparative high performance liquid chromatography (ammonium bicarbonate) to give a white solid of compound MY-004-083 (22 mg, 75% yield).

Compound 166: $^1$H NMR (400 MHz, DMSO-$d_6$): δ 10.50 (s, 1H), 8.68 (s, 1H), 8.47 (d, *J* = 7.6 Hz, 1H), 8.40 (t,

$J$ = 7.6 Hz, 1H), 8.35 (s, 1H), 8.22 (d, $J$ = 7.6 Hz, 1H), 7.22 (s, 1H), 5.04-5.00 (m, 1H), 3.98 (s, 3H), 2.98-2.92 (m, 1H), 2.81-2.67 (m, 4H).

Compound 165: $^1H$ NMR (400 MHz, DMSO-$d_6$): δ 10.51 (s, 1H), 8.68 (s, 1H), 8.47 (d, $J$ = 8 Hz, 1H), 8.42-8.38 (m, 2H), 8.23 (d, $J$ = 7.6 Hz, 1H), 7.20 (s, 1H), 5.26-5.22 (m, 1H), 3.99 (s, 3H), 3.19-3.13 (m, 1H), 2.89-2.82 (m, 2H), 2.73-2.67 (m, 2H).

Example 13. Synthesis of Compound MY-004-084 (120)

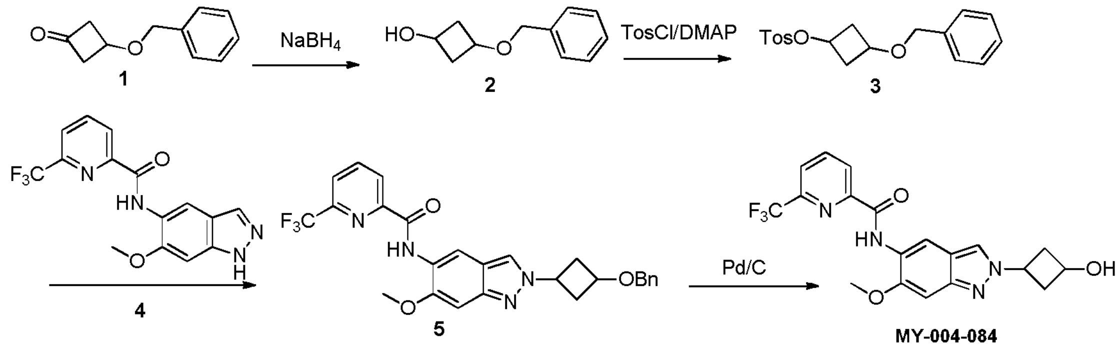


1. Synthesis of compound 2 in Example 13

$NaBH_4$ (32 mg) was added to compound 1 (100 mg) in methanol (5 mL) at 0 °C. The reaction mixture was stirred overnight at room temperature, and washed with water. Ethyl acetate was added for extraction and the organic phases were combined and dried by rotary evaporation to give a colorless oily solid of 100 mg in 99% yield without purification.

2. Synthesis of compound 3 in Example 13

Compound 2 (100 mg), TosCl (147 mg), DMAP (63 mg), and DIPEA (199 mg) were sequentially added to dichloromethane (10 mL) at room temperature. The reaction mixture was stirred overnight at room temperature, and then washed with water. Dichloromethane was added for extraction, and the organic phases were combined, dried by rotary evaporation, and purified by preparative thin layer chromatography (PE/EA=5:1) to give a colorless oily solid of 70 mg in 36% yield.

3. Synthesis of compound 5 in Example 13

Compound 3 (0.73 g), compound 4 (0.67 g), and cesium carbonate (0.78 g) were sequentially added to DMF (5 mL) at room temperature. The reaction mixture was stirred at 90 °C overnight and washed with water. Ethyl acetate was added for extraction. The organic phases were combined, dried by rotary evaporation and purified by preparative high performance liquid chromatography ($CH_3CN:H_2O$ (0.1% $NH_4HCO_3$)=5-95%, UV: 214 nm, flowrate: 15 mL/min) to give a pale yellow solid of 210 mg in 21% yield.

4. Preparation of compound MY-004-084 (120) in Example 13

Compound 5 (190 mg) and Pd/C (20 mg, 10% w/w) were added sequentially to methanol (20 mL) at room temperature. The reaction system was stirred overnight at 55 °C in hydrogen atmosphere, filtered, dried by rotary evaporation, and purified by preparative high performance liquid chromatography ($CH_3CN:H_2O$ (0.1% $NH_4HCO_3$)=5-95%, UV: 214 nm, flowrate: 15 mL/min) to give a white solid of 150 mg in 88% yield.

Compound 120: $^1H$ NMR (400 MHz, DMSO-$d_6$): δ 10.50 (s, 1H), 8.68 (s, 1H), 8.47-8.38 (m, 3H), 8.21 (d, $J$ = 7.6Hz, 1H), 7.20 (s, 1H), 5.27 (d, $J$ = 5.2Hz, 1H), 5.19-5.15 (m, 1H), 4.59-4.55 (m, 1H), 3.99 (s, 3H), 2.78-2.72 (m, 2H), 2.50-2.42 (m, 2H). LCMS: Rt = 3.602min, $[M+H]^+$= 407.1.

Example 14. Synthesis of Compound MY-004-085 (121)

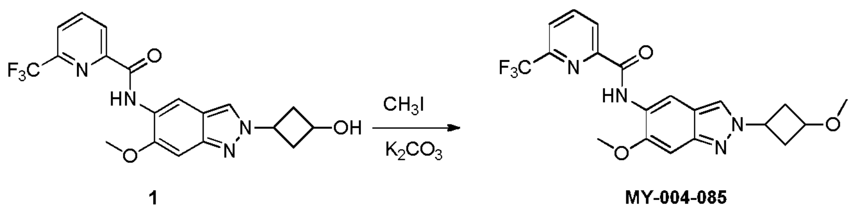


1. Synthesis of compound MY-004-085 (121) in Example 14

Compound 1 (i.e., compound 120 prepared in Example 13; 100 mg), potassium carbonate (102 mg), and methyl iodide (140 mg) were sequentially added to a closed system containing 5 mL of DMF at room temperature. The mixture was stirred at 80 °C overnight and washed with water. Ethyl acetate was added for extraction, and the organic phase was concentrated. The residue was subjected to preparative high performance liquid chromatography ($CH_3CN:H_2O$ (0.1% $NH_4HCO_3$)=5-95%, UV: 214 nm, flowrate: 15 mL/min) to give a white solid of 47 mg in 46% yield.

Compound 121: $^1H$ NMR (400 MHz, $CDCl_3$): δ 9.37 (s, 1H), 8.92 (s, 1H), 8.50-8.41 (m, 2H), 8.25 (d, *J* = 7.6 Hz, 1H), 7.68 (s, 1H), 5.52-5.49 (m, 1H), 4.49-4.43 (m, 1H), 4.18 (s, 3H), 4.06 (s, 3H), 3.01-2.92 (m, 2H), 2.66-2.60 (m, 2H). LCMS: Rt = 3.252 min, $[M+H]^+$= 421.2.

Example 15. Synthesis of Compound MY-004-089 (167)

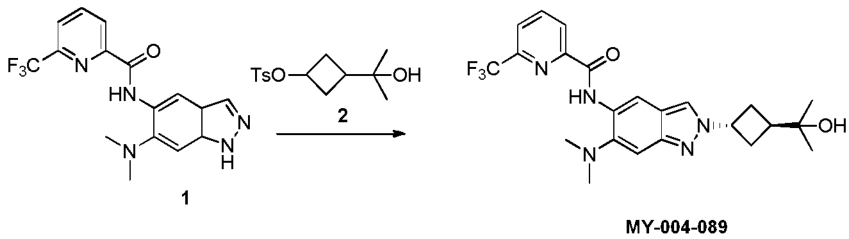

1. Preparation of compound MY-004-089 (167) in Example 15

Compound 1 (120 mg), compound 2 (98 mg), and cesium carbonate (247 mg) were dissolved in DMF (10 mL) at room temperature. The reaction mixture was stirred at 90 °C overnight and washed with water. Ethyl acetate was added for extraction. The organic phases were combined, dried by rotary evaporation and purified by preparative high performance liquid chromatography ($CH_3CN:H_2O$ (0.1% $NH_4HCO_3$)=5-95%, UV: 214 nm, flowrate: 15 mL/min) to give a white solid of 21 mg in 13% yield.

Compound 167: $^1H$ NMR (400 MHz, DMSO-$d_6$): δ 11.05 (s, 1H), 8.71 (s, 1H), 8.47 (d, *J* = 7.6 Hz, 1H), 8.40 (t, *J* = 8.4 Hz, 2H), 8.20 (d, *J* = 6.8 Hz, 1H),7.49 (s, 1H), 5.03-4.99 (m, 1H), 4.41 (s, 1H), 2.75 (s, 6H), 2.58-2.54 (m, 3H), 2.49-2.41 (m, 2H), 1.06 (s, 6H), LCMS: Rt = 3.475 min, $[M+H]^+$= 462.3.

Example 16. Synthesis of Compound MY-004-090 (168)

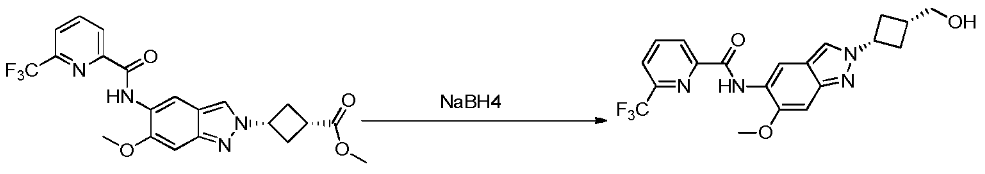


MY-004-099 MY-004-090

1. Preparation of compound MY-004-090 (168) in Example 16

Sodium borohydride (13 mg) and 0.4 mL of methanol were added to a solution of compound MY-004-099 (30 mg) in THF (4 mL) at 0 °C. The mixture was stirred at room temperature overnight and concentrated. The residue was subjected to preparative high performance liquid chromatography ($CH_3CN$:$H_2O$ (0.1% $NH_4HCO_3$)=5-95%, UV: 214 nm, flowrate 15 mL/min) to give a white solid of 12 mg in 40% yield.

Compound 168: $^1$H NMR (400 MHz, DMSO-$d_6$): δ 10.51 (s, 1H), 8.68 (s, 1H), 8.46 (d, $J$ = 7.6 Hz, 1H), 8.42-8.37 (m, 1H), 8.35 (s, 1H), 8.22 (d, $J$ = 6.8 Hz, 1H), 7.20 (s, 1H), 4.97-4.91 (m, 1H), 4.66 (t, $J$ = 5.2 Hz, 1H), 3.98 (s, 3H), 3.48 (t, $J$ = 4.8 Hz, 2H), 2.54-2.50 (m, 2H), 2.38-2.45 (m, 3H). LCMS: Rt = 3.263 min, $[M+H]^+$= 421.0.

Example 17. Synthesis of Compound MY-004-093 (169)

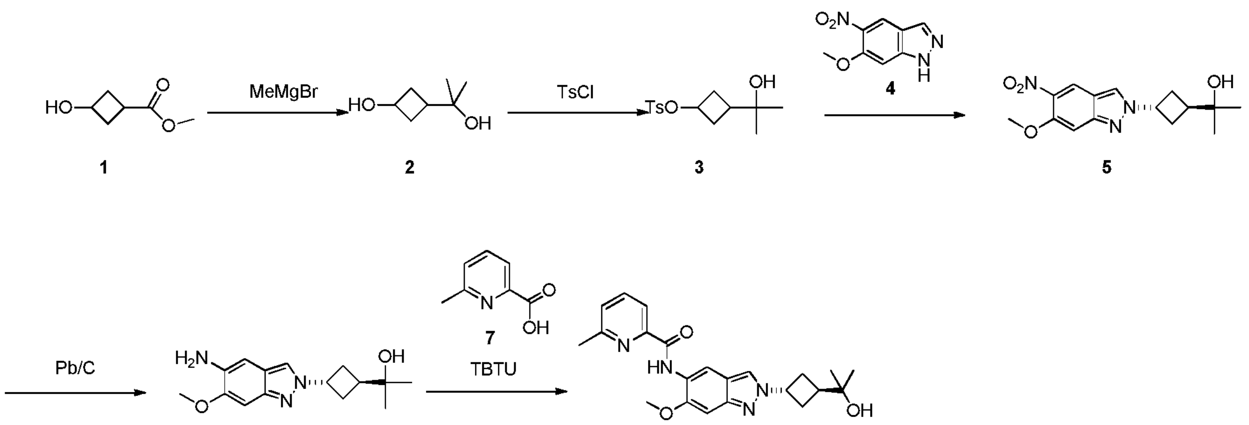


1 2 3 4 5

7

6 MY-004-093

1. Synthesis of compound 2 in Example 17

Methylmagnesium bromide (46 mL, 46 mmol) was dissolved in diethyl ether (15 mL) before a solution of compound 1 (1 g) in diethyl ether (5 mL) was added slowly at -20 °C. The mixture was stirred at -20 °C for 6 h. At 0 °C. The reaction was quenched with 4 N hydrochloric acid (14 mL). The mixture was dehydrated over anhydrous sodium sulfate, filtered, concentrated under reduced pressure, and purified on a silica gel column with petroleum ether/ethyl acetate=1.5:1 in gradient to give a yellow oil of 0.76 g in 76% yield.

2. Synthesis of compound 3 in Example 17

Compound 2 (0.76 g), TosCl (1.26 g), DMAP (0.79 g) and triethylamine (0.65 mL) were sequentially added to 5 mL of dichloromethane at 0 °C. After that, the mixture was stirred at room temperature overnight, concentrated, and purified on a silica gel column with petroleum ether/ethyl acetate=4:1 in gradient to give a colorless oil of 1.2 g in 73% yield.

3. Synthesis of compound 5 in Example 17

Cesium carbonate (3.4 g) was added to 15 mL of DMF containing compound 3 (1.0 g) and compound 4 (680 mg) at room temperature. The reaction mixture was stirred at 100 °C for 12 h, cooled to room temperature, and added with 20 mL of water. Ethyl acetate was added for extraction (15 mL × 4), and the organic phases were combined, washed

with water (8 mL × 3) and concentrated under reduced pressure. The residue was purified by HPLC ($CH_3CN$:$H_2O$ (0.1% $NH_4HCO_3$)=5-95%, UV: 214 nm, flowrate 15 mL/min) to give a white solid of 250 mg in 25% yield.

4. Synthesis of compound 6 in Example 17

Pd/C (100 mg, 10% w/w) was added to a solution of compound 5 (200 mg) in methanol (10 mL) at room temperature. The reaction mixture was stirred at room temperature under 50 psi in hydrogen atmosphere for 16 h, filtered, and concentrated under reduced pressure to give a yellow solid of 150 mg in 83% yield.

5. Preparation of compound MY-004-093 (169) in Example 17

Compound 6 (20 mg), compound 7 (13 mg), TBTU (35 mg) and DIEA (37 mg) were sequentially added to 6 mL of THF at room temperature. The mixture was stirred at room temperature overnight and concentrated. The residue was subjected to preparative high performance liquid chromatography ($CH_3CN$:$H_2O$ (0.1% $NH_4HCO_3$)=5-95%, UV: 214 nm, flowrate 15 mL/min) to give a white solid of 12 mg in 43% yield.

Compound 169: $^1$H NMR (400 MHz, DMSO-$d_6$): δ 10.83 (s, 1H), 8.84 (s, 1H), 8.10 (d, $J$ = 7.6 Hz, 1H), 7.87 (s, 1H), 7.77 (t, $J$ = 7.2 Hz, 1H), 7.31 (d, $J$ = 7.2 Hz, 1H), 7.11 (s, 1H), 5.02-4.96 (m, 1H), 4.04 (s, 3H), 2.78-2.74 (m, 2H), 2.66-2.61 (m, 6H). 1.25 (s, 6H). LCMS: Rt = 3.757 min, $[M+H]^+$= 395.2.

Example 18. Synthesis of Compound MY-004-094 (170)

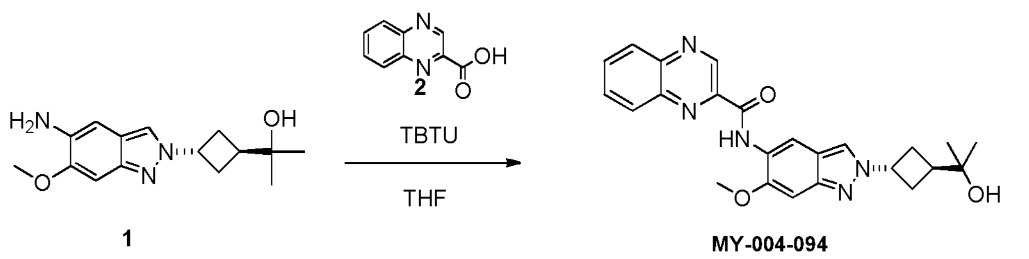


1. Preparation of compound MY-004-094 (170) in Example 18

Compound 1 (15 mg), compound 2 (12 mg), TBTU (26 mg) and DIEA (21 mg) were sequentially added to 5 mL of THF at room temperature. The mixture was stirred at room temperature overnight and concentrated. The residue was subjected to preparative high performance liquid chromatography ($CH_3CN$:$H_2O$ (0.1% $NH_4HCO_3$)=5-95%, UV: 214 nm, flowrate 15 mL/min) to give a white solid of 12 mg in 52% yield.

Compound 170: $^1$H NMR (400 MHz, $CDCl_3$): δ 10.65 (s, 1H), 9.79 (s, 1H), 8.89 (s, 1H), 8.25-8.22 (m, 2H), 7.93-7.88 (m, 3H), 7.15 (s, 1H), 5.05-5.01 (m, 1H), 4.09 (s, 3H), 2.80-2.74 (m, 2H), 2.69-2.62 (m, 3H), 1.37 (s, 6H). LCMS: Rt = 3.808 min, $[M+H]^+$= 432.2.

Example 19. Synthesis of Compound MY-004-095 (199)

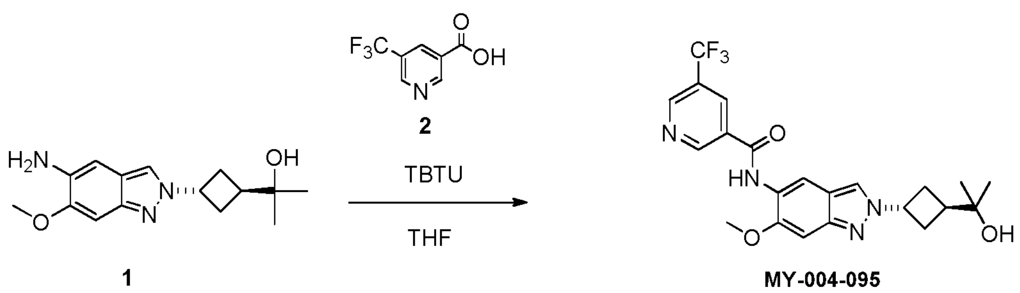

1. Preparation of compound MY-004-095 (199) in Example 19

Compound 1 (20 mg), compound 2 (18 mg), TBTU (35 mg) and DIEA (37 mg) were sequentially added to 5 mL of THF at room temperature. The mixture was stirred at room temperature overnight and concentrated. The residue was subjected to preparative high performance liquid chromatography ($CH_3CN$:$H_2O$ (0.1% $NH_4HCO_3$)=20-60%, UV: 214 nm, flowrate 15 mL/min) to give a white solid of 12 mg in 37% yield.

Compound 199: $^1H$ NMR (400 MHz, $CDCl_3$): δ 9.29 (s, 1H), 9.05 (s, 1H), 8.76 (s, 2H), 8.49 (s, 1H), 7.92 (s, 1H), 7.13 (s, 1H), 5.05-5.01 (m, 1H), 4.02 (s, 3H), 2.77-2.71 (m, 2H), 2.69-2.61 (m, 3H), 1.27 (s, 6H). LCMS: Rt = 3.538 min, $[M+H]^+$= 449.2.

Example 20. Synthesis of Compound MY-004-096 (172)

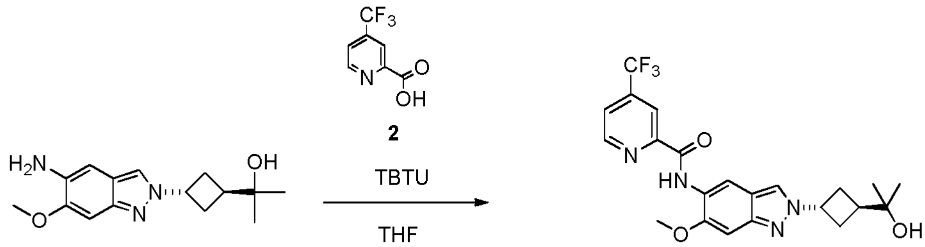

1 MY-004-096

1. Preparation of compound MY-004-096 (172) in Example 20

Compound 1 (15 mg), compound 2 (14 mg), TBTU (26 mg) and DIEA (28 mg) were sequentially added to 5 mL of THF at room temperature. The mixture was stirred at room temperature overnight and concentrated. The residue was subjected to preparative high performance liquid chromatography ($CH_3CN$:$H_2O$ (0.1% $NH_4HCO_3$)=5-95%, UV: 214 nm, flowrate 15 mL/min) to give a white solid of 20 mg in 80% yield.

Compound 172: $^1H$ NMR (400 MHz, $CDCl_3$): δ 10.66 (s, 1H), 8.86-8.84 (m, 2H), 8.55 (s, 1H), 7.91 (s, 1H), 7.70-7.69 (m, 1H), 7.13 (s, 1H), 5.04-5.00 (m, 1H), 4.05 (s, 3H), 2.79-2.74 (m, 2H), 2.68-2.60 (m, 3H), 1.25 (s, 6H). LCMS: Rt = 4.049 min, $[M+H]^+$= 449.2.

Example 21. Synthesis of Compound MY-004-097 (173)

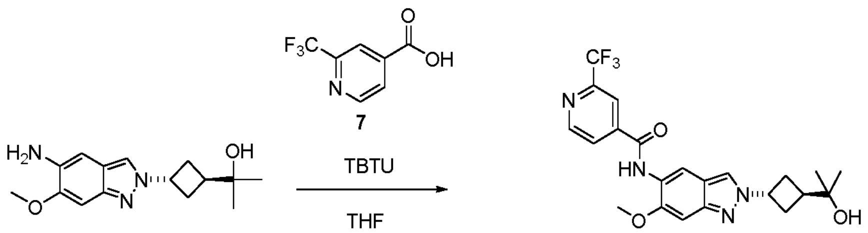

6 MY-004-097

1. Preparation of compound MY-004-097 (173) in Example 21

Compound 6 (20 mg), Compound 7 (20 mg), TBTU (35 mg) and DIEA (37 mg) were sequentially added to 8 mL of THF at 0 °C. The mixture was stirred at room temperature overnight and concentrated. The residue was subjected to preparative high performance liquid chromatography ($CH_3CN$:$H_2O$ (0.1% $NH_4HCO_3$)=5-95%, UV: 214 nm, flowrate 15 mL/min) to give a yellow solid of compound MY-004-097 (22 mg, 68% yield).

Compound 173: $^1H$ NMR (400 MHz, DMSO-$d_6$): δ 8.94-8.93 (d, $J$ = 4.8 Hz, 1H), 8.77-8.76 (d, $J$ = 3.2 Hz, 2H), 8.15 (s, 1H), 7.93-7.92 (d, $J$ = 4.4 Hz, 2H), 7.13 (s, 1H), 5.05-4.99 (m, 1H), 4.01 (s, 3H), 2.78-2.74 (m, 2H), 2.66-2.59 (m, 3H), 1.25 (s, 6H). LCMS: Rt = 3.614 min, $[M+H]^+$= 449.2.

Example 22. Synthesis of Compounds MY-004-099 (175) and MY-004-100 (176)

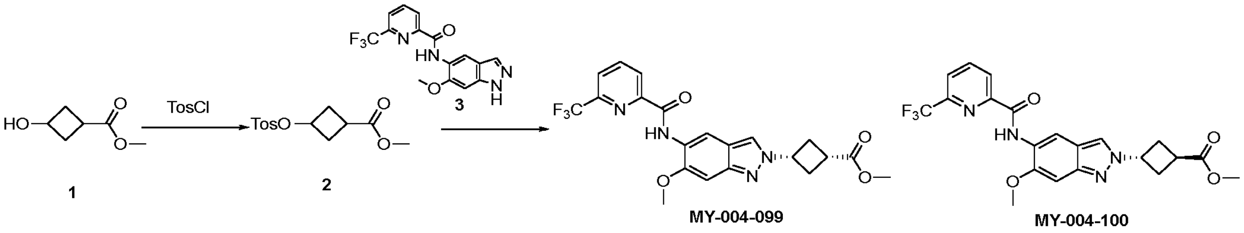


1 2 3 MY-004-099 MY-004-100

1. Synthesis of compound 2 in Example 22

Compound 1 (10 g), TosCl (19.06 g), DMAP (11.3 g) and triethylamine (21.5 mL) were sequentially added to 500 mL of dichloromethane at 0 °C. After that, the mixture was stirred at room temperature overnight, concentrated, and purified on a silica gel column (petroleum ether/ethyl acetate=5:1) to give a colorless oil of 16 g in 73.4% yield.

2. Preparation of compounds MY-004-099 (175) and MY-004-100 (176) in Example 22

Compound 3 (6 g), compound 2 (7.31 g), and cesium carbonate (17.45 g) were sequentially added to DMF (300 mL) at room temperature. The mixture was stirred at 80 °C for 16 h. At room temperature, the reaction was quenched by slowly adding water and stirring. Ethyl acetate was added for extraction and the organic phases were combined, dehydrated and concentrated. The residue was subjected to preparative high performance liquid chromatography ($CH_3CN:H_2O$ (0.1% $NH_4HCO_3$)=5-95%, UV: 214 nm, flowrate: 15 mL/min) to give a white solid of compound MY-004-099 at Rt=8.95 min (610 mg, 16% yield) and a yellow solid of compound MY-004-100 at Rt=6.64 min (520 mg, 14% yield).

Compound 175: $^1H$ NMR (400 MHz, $CDCl_3$): δ 10.72 (s, 1H), 8.81 (s, 1H), 8.50 (d, *J* = 8 Hz, 1H), 8.12 (t, *J* = 7.6 Hz, 1H), 7.93 (s, 1H), 7.81 (d, *J* = 7.6 Hz, 1H), 7.07 (s, 1H), 4.96-4.92 (m, 1H), 4.03 (s, 3H), 3.75 (s, 3H), 3.07-2.85 (m, 5H).

Compound 176: $^1H$ NMR (400 MHz, $CDCl_3$): δ 10.70 (s, 1H), 8.80 (s, 1H), 8.50 (d, *J* = 7.6 Hz, 1H), 8.11 (t, *J*= 7.6 Hz, 1H), 7.86-7.84 (m, 2H), 7.09 (s, 1H), 5.29-5.21 (m, 1H), 4.03 (s, 3H), 3.78 (s, 3H), 3.35-3.28 (m, 1H), 3.12-3.04 (m, 2H), 2.88-2.82 (m, 2H).

Example 23. Synthesis of Compound MY-004-110 (180)

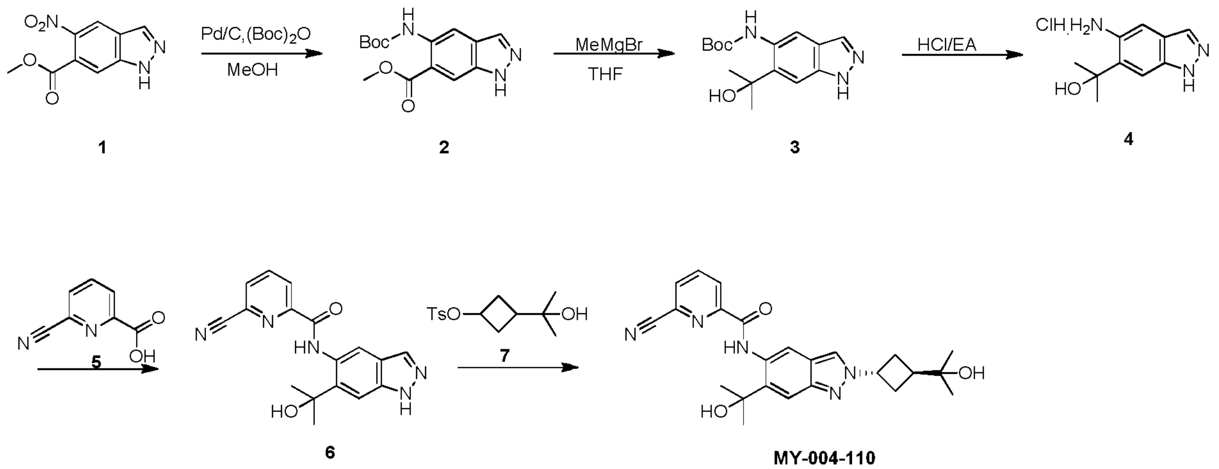


1 2 3 4 6 MY-004-110

1. Synthesis of compound 2 in Example 23

At room temperature, $(Boc)_2O$ (4.1 g) and 1 g of palladium on carbon were added into a solution of compound 1 (2.8 g) in methanol (50 mL) at room temperature. The reaction mixture was stirred at room temperature in hydrogen

atmosphere (one atm) overnight, filtered to remove palladium on carbon, and concentrated. The residue was resuspended and purified with a 40-mL mixture containing equal volumes of petroleum ether and tert-butyl methyl ether to give a yellow solid (3.2 g, 89% yield).

2. Synthesis of compound 3 in Example 23

At -70 °C, methylmagnesium bromide (17 mL, 51 mmol) was dropwise added to a solution of compound 2 (1.0 g) in tetrahydrofuran (30 mL). The mixture was stirred at room temperature for 16 h. At 0 °C, 5 mL of aqueous ammonium chloride solution was added dropwise to the reaction system. Ethyl acetate was added for extraction (10 mL × 4), and the organic phases were combined and concentrated under reduced pressure. The residue was purified by silica gel column chromatography (PE/EA=3:1) to give a yellow solid of 250 mg in 25% yield.

3. Synthesis of compound 4 in Example 23

Compound 3 (240 mg) was added to a solution of 4 M hydrochloric acid in ethyl acetate (20 mL) at room temperature. The mixture was stirred at room temperature for 3 h and concentrated under reduced pressure to give a white solid of 180 mg in 83% yield.

4. Synthesis of compound 6 in Example 23

Compound 5 (80 mg) and EDCI (157 mg) were added to a solution of compound 4 (144 mg) in pyridine (8 mL) at 25 °C. The reaction mixture was stirred overnight at room temperature and concentrated under reduced pressure. The residue was purified by silica gel column chromatography (PE/EA=3:1) to give a white solid of 100 mg in 57% yield.

5. Synthesis of compound MY-004-110 (180) in Example 23

Cesium carbonate (220 mg) was added to a solution of compound 6 (87 mg) and compound 7 (100 mg) in DMF (34 mL) at room temperature. The reaction mixture was stirred at 90 °C for 16 h. The mixture was cooled and added with water (15 mL). Ethyl acetate was added for extraction (15 mL × 4) and the organic phases were combined and concentrated under reduced pressure. The residue was purified by preparative high performance liquid chromatography ($CH_3CN:H_2O$ (0.1% $NH_4HCO_3$)=5-95%, UV: 214 nm, flowrate: 15 mL/min) to give a yellow solid of 7 mg in 5% yield.

Compound 180: $^1$H NMR (400 MHz, DMSO-$d_6$): δ 12.19 (s, 1H), 8.80 (s, 1H), 8.49 (d, $J$ = 8.0 Hz, 1H), 8.04 (t, $J$ = 8.0 Hz, 1H), 7.89 (s, 1H), 7.81 (d, $J$ = 8.0 Hz, 1H), 7.73 (s, 1H), 5.07-5.03 (m, 1H), 2.80-2.72 (m, 2H), 2.69-2.58 (m, 3H), 2.07 (s, 6H), 1.25 (s, 6H). LCMS: Rt = 2.761 min, $[M+H]^+$= 434.2.

Example 24. Synthesis of Compound MY-004-115 (181)

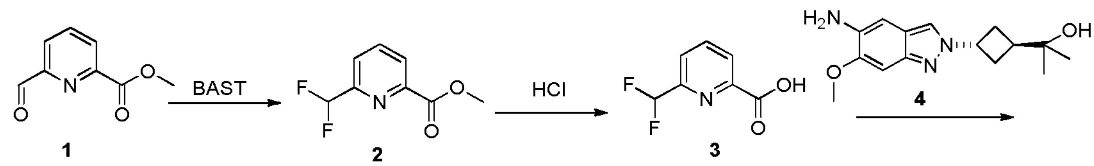

1 2 3 4

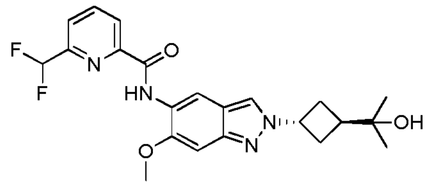

MY-004-115

1. Synthesis of compound 2 in Example 24

Compound 1 (165 mg) was added to a mixture of 6 mL of DCM and 0.02 mL of ethanol at room temperature. BAST (0.884 g) was added at 0 °C. 3 h later, the reaction was transferred to an ice bath and quenched by adding water.

Dichloromethane was added for extraction, and the extracts were combined and dehydrated over anhydrous sodium sulfate and concentrated under reduced pressure to give a colorless oily product of 180 mg in 96% yield.

2. Synthesis of compound 3 in Example 24

Compound 2 (180 mg) was added to concentrated hydrochloric acid (3 mL) at room temperature, and the mixture was stirred overnight at 90 °C in nitrogen atmosphere. The mixture was then concentrated under reduced pressure to give a white solid of 100 mg in 60% yield.

3. Synthesis of compound MY-004-115 (181) in Example 24

Compound 3 (13 mg), compound 4 (20 mg), and EDCI (21 mg) were sequentially added to pyridine (5 mL), and the mixture was stirred at room temperature overnight. Water was added to the mixture, followed by extraction with ethyl acetate. The organic phases were combined, washed with water, and concentrated under reduced pressure. The residue was purified by preparative high performance liquid chromatography ($CH_3CN$:$H_2O$ (0.1% $NH_4HCO_3$)=30-95%, UV: 214 nm, flowrate: 15 mL/min) to give a white solid of 16 mg in 41% yield.

Compound 181: $^1H$ NMR (400 MHz, DMSO-d6): δ 10.56 (s, 1H), 8.69 (s, 1H), 8.38-8.28 (m, 3H), 7.99 (d, *J* = 6.8 Hz, 1H), 7.18 (d, *J* = 17.2 Hz, 2H), 4.99 (t, *J* = 7.2 Hz, 1H), 4.41 (s, 1H), 4.01 (s, 3H), 2.55-2.50 (m, 4H), 2.44-2.43 (m, 1H), 1.11 (s, 6H). LCMS: Rt =3.757min, $[M+H]^+$= 431.2.

Example 25. Synthesis of Compound MY-004-118 (183)

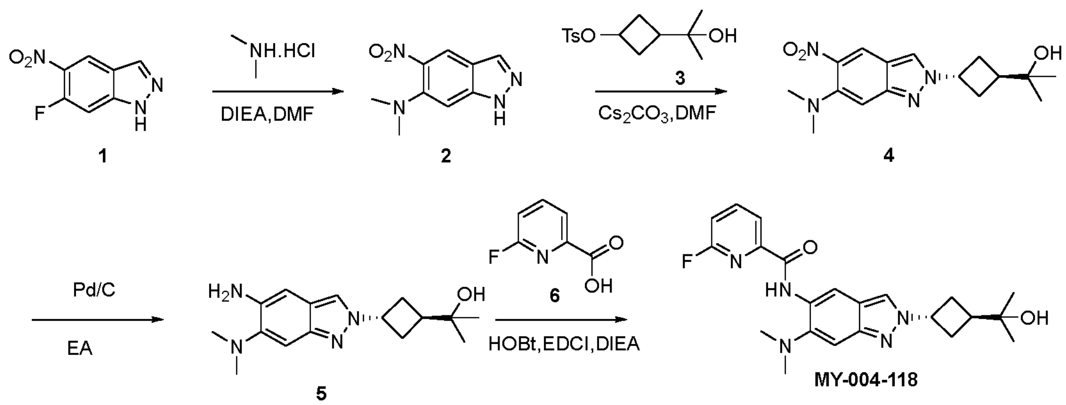


1. Synthesis of compound 2 in Example 25

Compound 1 (400 mg), dimethylamine hydrochloride (1.81 g) and DIPEA (2.86 g) were added sequentially to 20 mL of DMF at room temperature. The reaction mixture was stirred overnight at 80 °C in a closed vessel. After the reaction, water was added, followed by three extractions with ethyl acetate. The extracts were concentrated under reduced pressure and purified on a silica gel column (DCM/$CH_3OH$=200:1) to give a yellow solid of 570 mg in 83% yield.

2. Synthesis of compound 4 in Example 25

Compound 2 (100 mg), compound 3 (205 mg), and cesium carbonate (245 mg) were sequentially added to DMF (7 mL) and the mixture was stirred at 90 °C overnight. The reaction was quenched with water and ethyl acetate was added for extraction. The organic phases were combined, washed with water, and concentrated under reduced pressure. The residue was purified on a silica gel column (petroleum ether:ethyl acetate=3:1) to give a white solid of 60 mg in 39% yield.

3. Synthesis of compound 5 in Example 25

Compound 4 (50 mg) and palladium on carbon (25 mg) were sequentially added to 7 mL of ethyl acetate at room temperature. The reaction system was stirred overnight in hydrogen atmosphere at room temperature. The palladium on carbon was removed by filtration. The filtrate was concentrated to give a crude colorless oil of 43 mg in 95% yield.

4. Synthesis of compound MY-004-118 (183) in Example 25

Compound 5 (33 mg), compound 6 (17.7 mg), HOBT (23 mg), EDCI (32.8 mg), and DIEA (29.5 mg) were sequentially added to DMF (5 mL), and the system was stirred at room temperature overnight. The reaction was quenched with water and ethyl acetate was added for extraction. The organic phases were combined, washed with water, and concentrated under reduced pressure. The residue was purified by preparative high performance liquid chromatography ($CH_3CN$:$H_2O$ (0.1% $NH_4HCO_3$)=5-95%, UV: 214 nm, flowrate: 15 mL/min) to give a white solid (30 mg, 57% yield).

Compound 183: $^1H$ NMR (400 MHz, $CDCl_3$): δ 10.80 (s, 1H), 8.81 (s, 1H), 8.22 (dd, $J_1$ = 7.6 Hz, $J_2$ = 2 Hz, 1H), 8.03-7.98 (m, 1H), 7.89 (s, 1H), 7.49 (s, 1H), 7.13 (dd, $J_1$ = 8.4 Hz, $J_2$ = 2.4 Hz, 1H), 5.04-5.01 (m, 1H), 2.81 (m, 6H), 2.78-2.71 (m, 2H), 2.68-2.60 (m, 3H), 1.25 (s, 6H). LCMS: Rt =3.136 min, $[M+H]^+$= 412.2.

Example 26. Synthesis of Compound MY-004-119 (184)

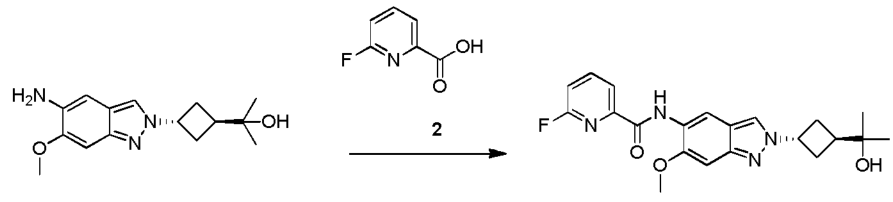


1 MY-004-119

1. Synthesis of compound MY-004-119 (184) in Example 26

Compound 1 (20 mg), compound 2 (13 mg), TBTU (34 mg) and DIEA (37 mg) were sequentially added to 8 mL of THF at room temperature. The mixture was stirred at room temperature overnight and concentrated. The residue was purified by preparative high performance liquid chromatography ($CH_3CN$:$H_2O$ (0.1% $NH_4HCO_3$)=5-95%, UV: 214 nm, flowrate 15 mL/min) to give a white solid of 13 mg in 45% yield.

Compound 184: $^1H$ NMR (400 MHz, $CDCl_3$): δ 10.33 (s, 1H), 8.90 (s, 1H), 8.18 (d, $J$ = 7.2 Hz, 1H), 7.99 (q, $J$ = 8.0 Hz, 1H), 7.87 (s, 1H), 7.12-7.09 (m, 2H), 5.01-4.98 (m, 1H), 4.03 (s, 3H), 2.74-2.70 (m, 2H), 2.65-2.58 (m, 3H), 1.39 (s, 6H). LCMS: Rt = 3.633 min, $[M+H]^+$= 399.2.

Example 27. Synthesis of Compound MY-004-128 (136)

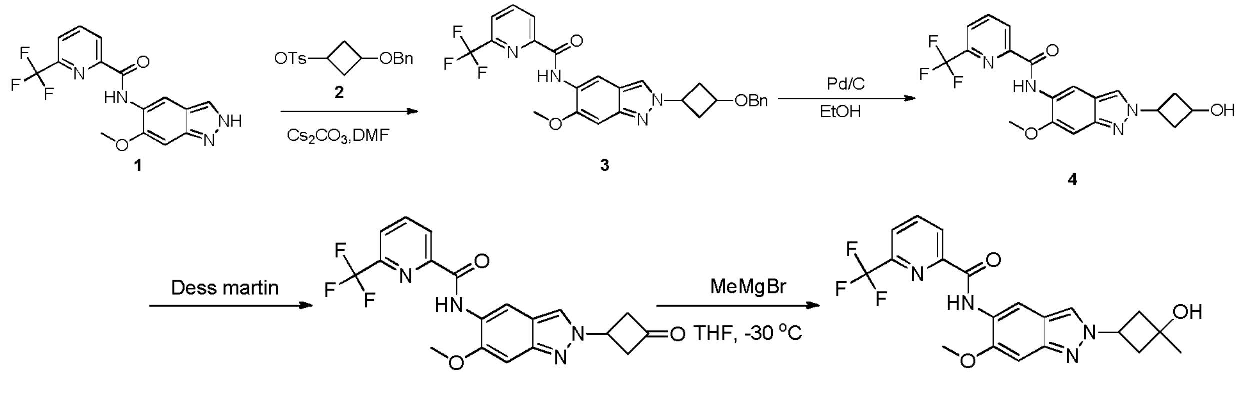


5 MY-004-128

1. Synthesis of compound 3 in Example 27

Compound 1 (500 mg), compound 2 (593 mg), and cesium carbonate (976 mg) were sequentially added to 20 mL of DMF at room temperature. The mixture was stirred at 90 °C overnight. The reaction mixture was concentrated under reduced pressure. The residue was purified by preparative high performance liquid chromatography ($CH_3CN$:$H_2O$

(0.1% $NH_4HCO_3$)=5-95%, UV: 214 nm, flowrate: 15 mL/min) to give a white solid of 115 mg (peak 1, target product) and a white solid of 220 mg (peak 2, 16% yield).

2. Synthesis of compound 4 in Example 27

Compound 3 (115 mg) and palladium on carbon (60 mg) were sequentially added to 8 mL of ethanol at room temperature. The mixture was stirred overnight at room temperature in hydrogen atmosphere. Palladium on carbon was removed from the reaction mixture by filtration, and the filtrate was concentrated under reduced pressure to give a crude white solid product of 93 mg in 99% yield, which was directly used for the next reaction. LCMS: Rt = 1.52 min, $[M+H]^+$ = 407.1.

3. Synthesis of compound 5 in Example 27

Compound 4 (73 mg) was dissolved in 7 mL of dichloromethane before Dess-Martain periodinane (99.1 mg) was added at 0 °C. The mixture was stirred at room temperature for 2 h. The reaction was monitored by LCMS, and was quenched by adding 10 mL of dichloromethane. The organic phase was washed with saturated sodium bicarbonate and concentrated. The residue was purified on a silica gel column (petroleum ether:ethyl acetate=3:1) to give a white solid of 90 mg in 99% yield.

4. Synthesis of compound MY-004-128 (136) in Example 27

Compound 5 (80 mg) was dissolved in 4 mL of tetrahydrofuran before MeMgBr (1.32 mL, 3.96 mmol) was dropwise added to the flask at -30 °C. The mixture was stirred at -30 °C for 2 h. The reaction was quenched with saturated aqueous ammonium chloride. Dichloromethane was added for extraction, and the organic phase was concentrated under reduced pressure. The residue was purified by preparative high performance liquid chromatography ($CH_3CN$:$H_2O$ (0.1% $NH_4HCO_3$)=5-95%, UV: 214 nm, flowrate 15 mL/min) to give a white solid of 20 mg in 22% yield.

Compound 136: $^1H$ NMR (400 MHz, $CDCl_3$): δ 10.70 (s, 1H), 8.80 (s, 1H), 8.49 (d, *J* = 8.0 Hz, 1H), 8.11 (t, *J* = 8.0 Hz, 1H), 7.87 (s, 1H), 7.84 (d, *J* = 9.2 Hz, 1H), 7.07 (s, 1H), 4.73-4.70 (m, 1H), 4.20 (s, 1H), 4.03 (s, 3H), 2.85-2.80 (m, 2H), 2.76-2.71 (m, 2H), 1.48 (s, 3H). LCMS: Rt = 3.909 min, $[M+H]^+$= 421.1.

Example 28. Synthesis of Compound MY-004-168 (146)

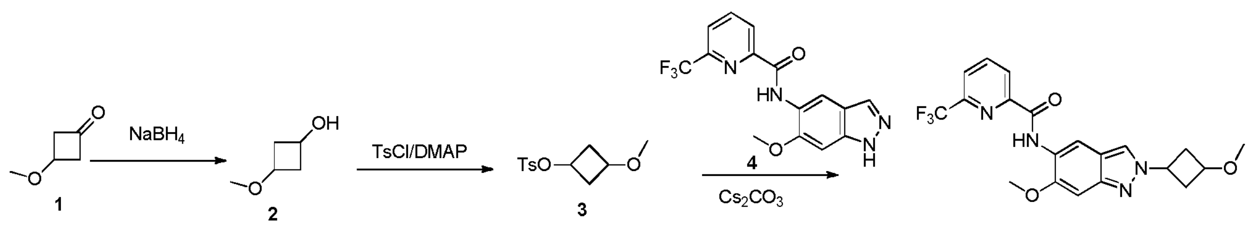


MY-004-168

1. Synthesis of compound 2 in Example 28

To compound 1 (20 mg) in 2 mL of methanol at 0 °C was added sodium borohydride (15 mg). The mixture was stirred at room temperature for 3 h. After the reaction, the mixture was washed with water. Ethyl acetate was added for extraction, and the organic phase was concentrated under reduced pressure. The residue was purified on a silica gel column (dichloromethane:methanol=20:1) to give a colorless oily product of 120 mg in 29% yield.

2. Synthesis of compound 3 in Example 28

Compound 2 (120 mg), DMAP (215 mg) and DIPEA (228 mg) were sequentially added to 5 mL of dichloromethane. The reaction mixture was added with TsCl (335 mg) and stirred overnight at 0 °C. The resultant solution was concentrated under reduced pressure. The residue was purified on a silica gel column (petroleum ether:ethyl acetate=5:1) to give a colorless oily product of 310 mg in 99% yield.

3. Synthesis of compound MY-004-168 (146) in Example 28

Compound 3 (55 mg), compound 4 (60 mg), and cesium carbonate (116 mg) were sequentially added to 5 mL of DMF in nitrogen atmosphere. The mixture was stirred at 80 °C overnight. The mixture was washed with water. Ethyl acetate was added for extraction, and the extracts were concentrated under reduced pressure. The residue was purified by preparative chromatography to give a white solid of 20 mg in 20% yield.

Compound 146: $^1H$ NMR (400 MHz, DMSO-$d_6$): δ 10.51 (s, 1H), 8.68 (s, 1H), 8.47-8.37 (m, 3H), 8.21 (d, *J* = 7.6 Hz, 1H), 7.20 (s, 1H), 5.20-5.16 (m, 1H), 4.29-4.25 (m, 1H), 3.99 (s, 3H), 3.22 (s, 3H), 2.78-2.72 (m, 2H), 2.58-2.50 (m, 2H). LCMS: Rt = 3.264 min, $[M+H]^+$= 421.2.

Example 29. Synthesis of Compounds MY-004-126-1 (189) and MY-004-126-2 (190)

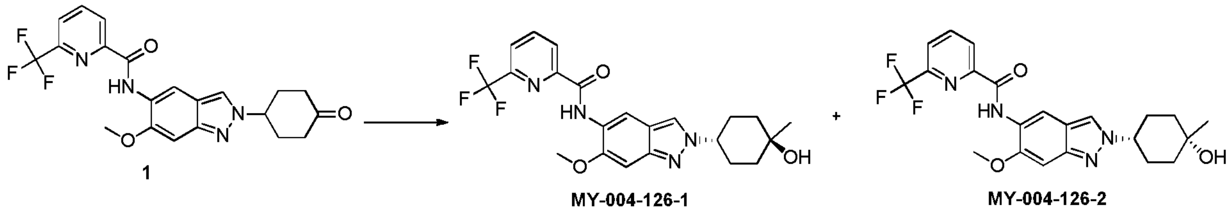

1. Synthesis of compounds MY-004-126-1 (189) and MY-004-126-2 (190) in Example 29

Compound 1 (100 mg) was dissolved in 10 mL of THF at room temperature. A solution of methylmagnesium bromide in tetrahydrofuran (2.78 mL, 2.78 mmol) was added dropwise at -30 °C. The mixture was stirred for 1 h at -30 °C, and added with an aqueous ammonium chloride solution for quenching the reaction. Ethyl acetate was added for extraction, and the extracts were purified by preparative chromatography ($CH_3CN$:$H_2O$ (0.1% $NH_4HCO_3$)=5-95%, UV: 214 nm, flowrate 15 mL/min) to give a white product of MY-004-126-1 at Rt=7.13 min (21 mg, 9% yield) and MY-004-126-2 at Rt=8.94 min (31 mg, 14% yield).

MY-004-126-1: $^1H$ NMR (400 MHz, $CDCl_3$): δ 10.70 (s, 1H), 8.82 (s, 1H), 8.49 (d, *J* = 8.0 Hz, 1H), 8.12 (t, *J* = 8.0 Hz, 1H), 7.86 (t, *J* = 8.0 Hz, 2H), 7.08 (s, 1H), 4.42-4.40 (m, 1H), 4.03 (s, 3H), 2.24-2.14 (m, 4H), 1.89-1.87 (m, 2H), 1.76-1.72 (m, 2H), 1.40 (s, 3H). LCMS: Rt = 2.349, $[M+H]^+$= 449.2.

MY-004-126-2: $^1H$ NMR (400 MHz, $CDCl_3$): δ 10.70 (s, 1H), 8.82 (s, 1H), 8.49 (d, *J* = 8.0 Hz, 1H), 8.11 (t, *J* = 8.0 Hz, 1H), 7.91 (s, 1H), 7.85 (d, *J* = 7.6 Hz, 1H), 7.07 (s, 1H), 4.37-4.31(m, 1H), 4.03 (s, 3H), 2.34-2.26 (m, 2H), 2.14-2.11 (m, 2H), 1.89-1.86 (m, 2H), 1.69-1.63 (m, 2H), 1.33 (s, 3H). LCMS: Rt = 2.349, $[M+H]^+$= 449.2. LCMS: Rt =2.611, $[M+H]^+$= 449.2.

Example 30. Synthesis of Compounds MY-004-127-1 (191) and MY-004-127-2 (192)

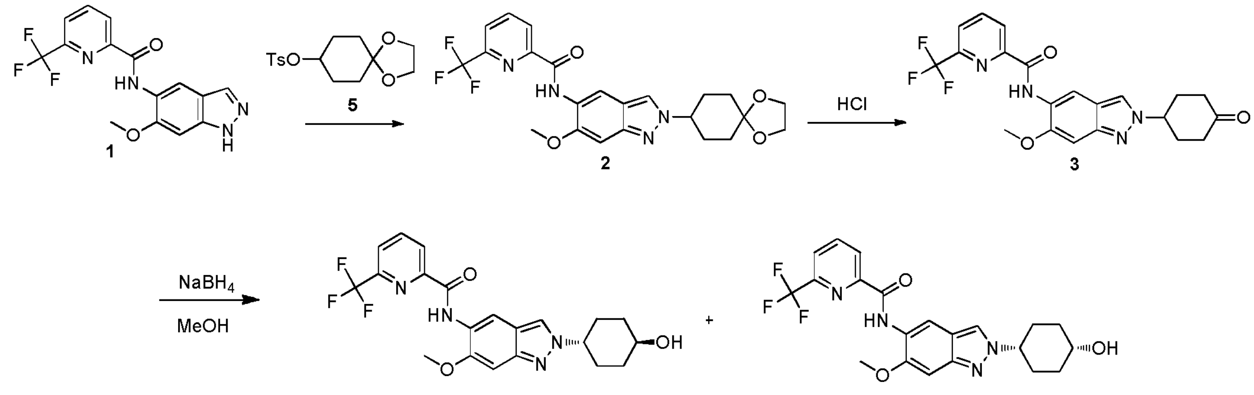


1 2 3

MY-004-127-1 MY-004-127-2

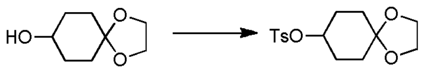

4 5

1. Synthesis of compound 2 in Example 30

Compound 1 (750 mg), compound 5 (836 mg), and cesium carbonate (2.18 g) were added to 20 mL of DMF at room temperature. The reaction mixture was stirred overnight at 90 °C before water was added. Ethyl acetate was added for extraction, and the extracts were concentrated by preparative chromatography ($CH_3CN:H_2O$ (0.1% $NH_4HCO_3$)=5-95%, UV: 214 nm, flowrate 15 mL/min) to give a white product of 300 mg in 28.3% yield.

2. Synthesis of compound 3 in Example 30

Compound 2 (280 mg) was dissolved in 8 mL of THF and 16 mL of 1 N HCl at room temperature. The mixture was stirred at room temperature overnight. After the reaction, a saturated aqueous sodium bicarbonate solution was added. Ethyl acetate was added for extraction, and the extracts were dehydrated over sodium sulfate, dried by rotary evaporation, and concentrated to give a pale yellow solid of 240 mg in 95% yield without purification.

3. Synthesis of compounds MY-004-127-1 (191) and MY-004-127-2 (192) in Example 30

Compound 3 (74 mg) was dissolved in 2 mL of methanol before sodium borohydride (13 mg) was added at 0 °C. The mixture was stirred at room temperature for 1 h. The reaction was quenched by adding water. Then ethyl acetate was added for extraction, and the extracts were concentrated under reduced pressure. The residue was purified by preparative high performance liquid chromatography ($CH_3CN:H_2O$ (0.1% $NH_4HCO_3$)=5-95%, UV: 214 nm, flowrate 15 mL/min) to give a white solid of compound MY-004-127-1 at Rt=8.46 min (30 mg, 41% yield) and compound MY-004-127-2 at Rt=10.56 min (6 mg, 8% yield).

$^1H$ NMR (400 MHz, $CDCl_3$) (MY-004-127-1) : δ 10.70 (s, 1H), 8.81 (s, 1H), 8.50 (d, *J* = 7.6 Hz, 1H), 8.11 (t, *J* = 7.6 Hz, 1H), 7.86 (t, *J* = 5.2 Hz, 2H), 7.06 (s, 1H), 4.39-4.31 (m, 1H), 4.02 (s, 3H), 3.85-3.78 (m, 1H), 2.30-2.27 (m, 2H), 2.20-2.17 (m, 2H), 2.11-2.00 (m, 2H), 1.60-1.50 (m, 3H).LCMS: Rt =3.674, $[M+H]^+$= 435.2

$^1H$ NMR (400 MHz, $CDCl_3$)(MY-004-127-2): δ 10.70 (s, 1H), 8.82(s, 1H), 8.49 (d, *J* = 7.6 Hz, 1H), 8.11 (t, *J* = 8.0 Hz, 1H), 7.92 (s, 1H), 7.86 (d, *J* = 8 Hz, 1H), 7.07 (s, 1H), 4.42-4.35 (m, 1H), 4.15 (s, 1H), 4.03 (s, 3H), 2.42-2.32 (m, 2H), 2.10-1.97 (m, 4H), 1.80-1.73 (m, 2H), 1.53 (s, 1H).LCMS: Rt =3.076, $[M+H]^+$= 435.2

4. Synthesis of compound 5 in Example 30

Compound 4 (1 g) was dissolved in 100 mL of dichloromethane. *p*-Toluenesulfonyl chloride (1.8 g), triethylamine (0.96 g) and DMAP (1.16 g) were added sequentially and the mixture was stirred at room temperature overnight. The mixture was concentrated under reduced pressure. The residue was purified on a silica gel column (PE/EA=2:1) to give a yellow solid of 2 g in 99% yield.

Example 31. Synthesis of Compounds MY-004-153 (193) and MY-004-154 (194)

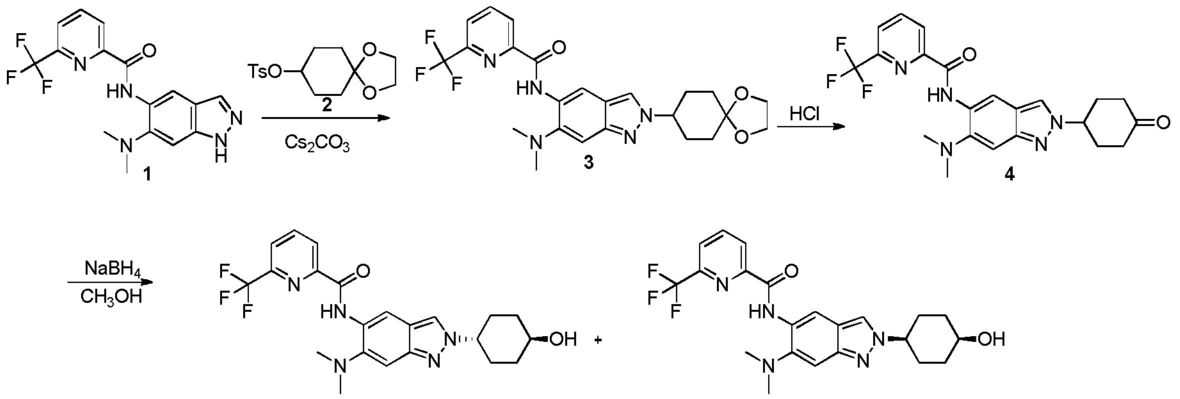


MY-004-153 MY-004-154

1. Synthesis of compound 3 in Example 31

Compound 1 (400 mg), compound 2 (429 mg), and cesium carbonate (486 mg) were sequentially added to 20 mL of DMF in nitrogen atmosphere. The mixture was stirred at 90 °C for 18 h. The reaction was quenched by adding water at room temperature. Ethyl acetate was added for extraction, and the organic phases were combined and concentrated under reduced pressure. The residue was purified by preparative chromatography ($CH_3CN$:$H_2O$ (0.1% $NH_4HCO_3$)=5-95%, UV: 214 nm, flowrate: 15 mL/min) to give a white solid (170 mg, 30% yield).

2. Synthesis of compound 4 in Example 31

Compound 3 (150 mg) was added to 5 mL of THF and 20 mL of 1 N HCl. The mixture was stirred at 25 °C for 18 h. After the reaction, the mixture was washed with saturated sodium bicarbonate. Ethyl acetate was added for extraction, and the extracts were dehydrated over sodium sulfate and concentrated under reduce pressure to give a pale yellow solid of 140 mg in 99% yield.

3. Preparation of compounds MY-004-153 (193) and MY-004-154 (194)

Compound 4 (120 mg) was added to 10 mL of methanol before sodium borohydride (31 mg) was added at 0 °C. The mixture was stirred at 25 °C for 18 h. After the reaction, ammonium chloride was added for quenching. The mixture was concentrated under reduced pressure and purified by preparative chromatography ($CH_3CN$:$H_2O$ (0.1% $NH_4HCO_3$)=20-60%, UV: 214 nm, flowrate: 15 mL/min) to give a yellow solid of compound MY-004-153 (85 mg, 70% yield) at Rt=8.08 min and a yellow solid of compound MY-004-154 (9 mg, 7% yield) at Rt=10.43 min.

$^1$H NMR (400 MHz, $CDCl_3$) MY-004-153: δ 11.23 (s, 1H), 8.82 (s, 1H), 8.50 (d, *J* = 8.0 Hz, 1H), 8.12 (t, *J* = 8.0 Hz, 1H), 7.87 (t, *J* = 8.0 Hz, 2H), 7.46 (s, 1H), 4.38-4.36 (m, 1H), 3.83-3.81 (m, 1H), 2.81 (s, 6H), 2.30-2.27 (m, 2H), 2.21-2.17 (m, 2H), 2.09-2.05 (m, 2H), 1.57-1.54 (m, 2H). LCMS: Rt =3.452 min, $[M+H]^+$= 448.2.

$^1$H NMR (400 MHz, $CDCl_3$) MY-004-154: δ 11.24 (s, 1H), 8.83 (s, 1H), 8.50 (d, *J* = 7.6 Hz, 1H), 8.12 (t, *J* = 8.0 Hz, 1H), 7.93 (s, 1H), 7.85 (d, *J* = 7.6 Hz, 1H), 7.46 (s, 1H), 4.42-4.40 (m, 1H), 4.15(s, 1H), 2.82 (s, 6H), 2.40-2.36 (m, 2H), 2.10-1.98 (m, 4H), 1.81-1.77 (m, 2H), 1.52 (s, 1H). LCMS: Rt =3.507 min, $[M+H]^+$= 448.2.

Example 32. Synthesis of Compounds MY-004-227 (1100) and MY-004-228 (1101)

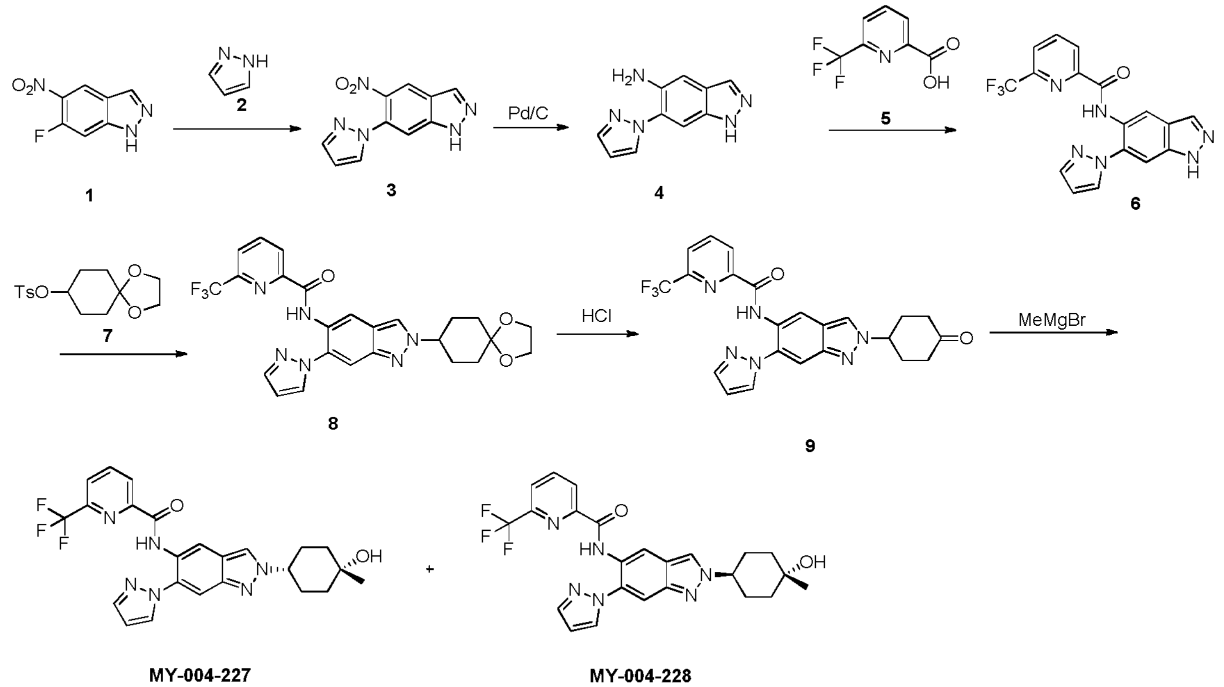


1, 3, 4, 6, 8, 9

MY-004-227 + MY-004-228

1. Synthesis of compound 3 in Example 32

Cesium carbonate (4.5 g) was added to a solution of compound 1 (1.0 g) and compound 2 (7.5 g) in DMSO (30 mL) at 15 °C. The reaction system was stirred at 120 °C for 16 h, cooled and added with 200 mL of water. Ethyl acetate was added for extraction (80 mL × 4), and the organic phases were combined and concentrated under reduced pressure. The residue was purified by silica gel column chromatography (PE/EA=2:1) to give a yellow solid of 850 mg in 67% yield.

2. Synthesis of compound 4 in Example 32

60 mg of Pd/C was added to a solution of compound 3 (600 mg) in ethyl acetate (200 mL). The reaction system was stirred in hydrogen atmosphere at 25 °C for 16 h. The mixture was filtered and the filtrate was concentrated under reduced pressure to give a yellow solid of 520 mg in 100% yield. LCMS: Rt =1.14, $[M+H]^+$= 200.0.

3. Synthesis of compound 6 in Example 32

EDCI (752 mg) was added to a solution of compound 4 (520 mg) and compound 5 (600 mg) in 30 mL of pyridine. The mixture was stirred at 25 °C for 16 h. The resultant solution was concentrated, and the residue was purified by preparative high performance liquid chromatography ($CH_3CN$:$H_2O$ (0.1% $NH_4HCO_3$)=5-95%, UV: 214 nm, flowrate: 15 mL/min) to give a brown solid (556 mg, 57% yield).

4. Synthesis of compound 8 in Example 32

Cesium carbonate (1.3 g) was added to a solution of compound 6 (588 mg) and compound 7 (739 mg) in DMF (15 mL) at 15 °C. In nitrogen atmosphere, the mixture was stirred at 90 °C for 36 h. At 15 °C, the reaction was quenched by adding water (500 mL). Ethyl acetate was added for extraction (50 mL × 4)., and the organic phases were combined, dehydrated over anhydrous $MgSO_4$, filtered under vacuum and concentrated under reduced pressure. The residue was purified by preparative high performance liquid chromatography ($CH_3CN$:$H_2O$ (0.1% $NH_4HCO_3$)=5-95%, UV: 214 nm, flowrate: 15 mL/min) to give a yellow solid (144 mg, 18% yield).

5. Synthesis of compound 9 in Example 32

Compound 8 (144 mg) was dissolved in 15 mL of THF before 15 mL of 2 M aqueous hydrochloric acid was added. The mixture was stirred at 15 °C for 16 h before the reaction was quenched by adding 20 mL of ethyl acetate and 20 mL of saturated aqueous sodium bicarbonate. Ethyl acetate (20 mL × 4) was added for extraction, and the

extracts were dehydrated over anhydrous $MgSO_4$ and concentrated under reduced pressure. The residue was purified by preparative high performance liquid chromatography ($CH_3CN$:$H_2O$ (0.1% $NH_4HCO_3$)=5-95%, UV: 214 nm, flowrate: 15 mL/min) to give a yellow solid of compound 9 (98 mg, 74% yield).

6. Preparation of compounds MY-004-227 (1100) and MY-004-228 (1101)

Compound 8 (88 mg) was dissolved in 15 mL of THF at 15 °C. The mixture was added with methylmagnesium bromide (0.63 mL, 1.9 mmol) at -30 °C, and was stirred at 20 °C for 5 h. The reaction was quenched by adding aqueous ammonium chloride solution (20 mL). Ethyl acetate (20 mL × 4) was added for extraction, and the extracts were dehydrated over anhydrous $MgSO_4$ and concentrated under reduced pressure. The residue was purified by prep-TLC to give crude yellow solids of compound MY-004-227 (20 mg) and compound MY-004-228 (20 mg). The crude products were then purified by preparative high performance liquid chromatography ($CH_3CN$:$H_2O$ (0.1% $NH_4HCO_3$)=5-95%, UV: 214 nm, flowrate: 15 mL/min) to give white solids of compound MY-004-227 (12 mg, 13% yield) at Rt=11.36 min and MY-004-228 (10 mg, 11% yield) at Rt=9.57 min.

$^1H$ NMR (400 MHz, $CDCl_3$) (MY-004-227): δ 12.06 (s, 1H), 9.04 (s, 1H), 8.44 (d, *J* = 8.0 Hz, 1H), 8.09-8.05 (m, 2H), 7.97 (s, 1H), 7.84-7.81 (m, 2H), 7.71 (s, 1H), 6.54 (s, 1H), 4.46-4.38 (m, 1H), 2.40-2.30 (m, 2H), 2.14 (d, *J* = 16.0 Hz, 2H), 1.88 (d, *J* = 12.0 Hz, 2H), 1.69-1.61 (m, 2H), 1.34 (s, 3H), 1.29 (s, 1H). LCMS: Rt =3.216, $[M+H]^+$= 485.2.

$^1H$ NMR (400 MHz, $CDCl_3$) (MY-004-228) : δ 12.09 (s, 1H), 9.03 (s, 1H), 8.44 (d, *J* = 8.0 Hz, 1H), 8.09-8.03 (m, 2H), 7.97 (s, 1H), 7.85-7.81 (m, 2H), 7.73 (s, 1H), 6.54 (s, 1H), 4.53-4.45 (m, 1H), 2.32-2.23 (m, 2H), 2.22-2.13 (m, 2H), 1.91-1.88 (m, 2H), 1.77-1.69 (m, 2H), 1.45 (s, 1H), 1.41 (s, 3H). LCMS: Rt =10.574, $[M+H]^+$= 485.2.

Example 33. Synthesis of Compound MY-004-144 (1102)

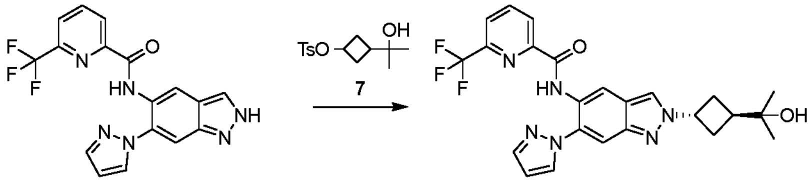


6

MY-004-144

Cesium carbonate (6.99 g) was added to a solution of compound 6 (3.2 g) and compound 7 (2.93 g) in DMF (100 mL) at 25 °C. The mixture was stirred at 90 °C for 12 h. The mixture was cooled and added with water (500 mL). Ethyl acetate was added for extraction (200 mL × 3), and the organic phase was concentrated under reduced pressure. The residue was purified on a silica gel column (DCM/$CH_3OH$=100:1), and the resultant crude product was resuspended in 20 mL of acetonitrile, filtered and then concentrated to give a white solid of MY-004-144 (1.184 g, 16% yield).

$^1H$ NMR (400 MHz, $CDCl_3$): δ 12.08 (s, 1H), 9.03 (s, 1H), 8.44 (d, *J* = 8.0 Hz, 1H), 8.07 (t, *J* = 8.0 Hz, 1H), 8.03 (s, 1H), 7.97 (d, *J* = 0.8 Hz, 1H), 7.86-7.81 (m, 2H), 7.76 (s, 1H), 6.56 (d, *J* = 2.0 Hz, 1H), 5.12-5.08 (m, 1H), 2.82-2.75 (m, 2H), 2.72-2.61 (m, 3H), 1.41 (s, 1H), 1.26 (s, 6H). LCMS: Rt = 3.999 min, $[M+H]^+$= 485.2.

Example 34. Synthesis of Compound MY-004-146 (1103)

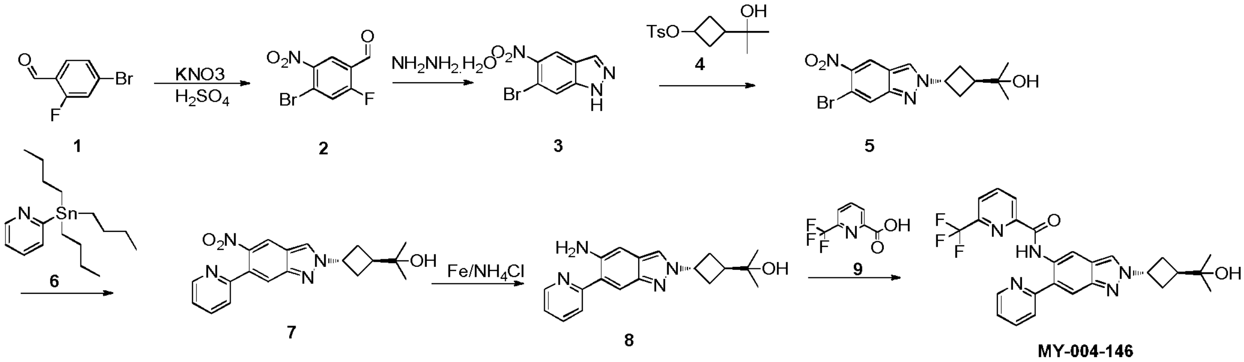


1 2 3 5

7 8

MY-004-146

1. Synthesis of compound 2 in Example 34

At -15 °C, $KNO_3$ (3.9 g) was dissolved in concentrated sulfuric acid (100 mL, 96%) and the mixture was stirred for 20 min. Compound 1 (7 g) was added in portions while the internal temperature was controlled between -15 °C and -12 °C. The mixture was stirred for 3 h at 15 °C. The mixture was added into ice water, stirred for 5 min and filtered under vacuum. The filter cake was washed with ice water, and the solid was collected and dried under reduced pressure to give a yellow solid of 7.2 g in 92% yield.

2. Synthesis of compound 3 in Example 34

Hydrazine hydrate (4.2 mL) was added dropwise to a solution of compound 2 (7.7 g) in THF (150 mL). The reaction system was stirred at 15 °C for 3 days. Water (100 mL) and ethyl acetate (150 mL) were added to the mixture. The organic phase was washed with water (150 mL × 4) and saline (150 mL), dehydrated over anhydrous $MgSO_4$, and concentrated under reduced pressure to give a crude product. The crude product was resuspended in MTBE (40 mL) to give a yellow solid of 6.6 g in 86% yield.

3. Synthesis of compound 5 in Example 34

Cesium carbonate (3.3 g) was added to a solution of compound 3 (1 g) and compound 4 (1.8 g) in DMF (40 mL). In nitrogen atmosphere, the mixture was stirred at 90 °C for 36 h and then cooled to 25 °C. To the reaction mixuter, water (200 mL) was added, and ethyl acetate was added for extraction (80 mL × 4), and the extracts were combined and dehydrated over anhydrous $MgSO_4$, filtered under vacuum and concentrated under reduced pressure. The residue was purified by preparative high performance liquid chromatography ($CH_3CN$:$H_2O$ (0.1% $NH_4HCO_3$)=5-95%, UV: 214 nm, flowrate: 15 mL/min) to give a yellow solid (162 mg, 11% yield).

4. Synthesis of compound 7 in Example 34

$Pd(PPh_3)_4$ (26 mg) and LiCl (75 mg) were added to a solution of compound 5 (160 mg) and compound 6 (332 mg) in DMF (10 mL). The mixture was stirred at 85 °C for 16 h in nitrogen atmosphere. The mixture was cooled, added with saturated KF solution (30 mL), and filtered under vacuum. The filtrate was added with ethyl acetate (50 mL × 4) for extraction, and the extracts were combined and dehydrated over anhydrous $MgSO_4$, filtered, and concentrated under reduced pressure. The residue was purified by preparative high performance liquid chromatography ($CH_3CN$:$H_2O$ (0.1% $NH_4HCO_3$)=5-95%, UV: 214 nm, flowrate: 15 mL/min) to give a white solid (50 mg, 31% yield).

5. Synthesis of compound 8 in Example 34

Iron powder (25 mg) and $NH_4Cl$ (32 mg) were added to a solution of compound 7 (27 mg) in EtOH/$H_2O$ (6 mL/2 mL). In nitrogen atmosphere, the mixture was stirred at 60 °C for 4 h. The mixture was cooled and added with water (30 mL). Ethyl acetate was added for extraction (20 mL × 7), and the organic phases were combined, washed with saline (50 mL), dehydrated over anhydrous $MgSO_4$, filtered under vacuum and concentrated under reduced pressure to give a crude yellow oil (25 mg, 100% yield) The crude product was directly used without further purification. LCMS: Rt = 1.33 min, $[M+H]^+$ = 323.2.

6. Preparation of compound MY-004-146 (1103)

EDCI (22 mg) was added to a solution of compound 8 (25 mg) and compound 9 (17 mg) in 5 mL of pyridine. The mixture was stirred at 15 °C for 16 h and concentrated. The residue was purified by preparative high performance liquid chromatography ($CH_3CN$:$H_2O$ (0.1% $NH_4HCO_3$)=20-60%, UV: 214 nm, flowrate: 15 mL/min) to give a yellow solid (27 mg, 71% yield).

Compound 1103: $^1H$ NMR (400 MHz, $CDCl_3$): δ 13.22 (s, 1H), 9.02 (s, 1H), 8.87 (d, *J* = 4.8 Hz, 1H), 8.49 (d, *J* = 8.0 Hz, 1H), 8.09-8.03 (m, 2H), 7.98 (s, 1H), 7.88-7.76 (m, 3H), 7.35-7.32 (m, 1H), 5.12-5.08 (m, 1H), 2.83-2.77 (m, 2H), 2.72-2.61 (m, 3H), 1.43 (s, 1H), 1.25 (s, 6H). LCMS: Rt =3.688, $[M+H]^+$= 496.2.

Example 35. Synthesis of Compounds MY-004-164 (195) and MY-004-165 (196)

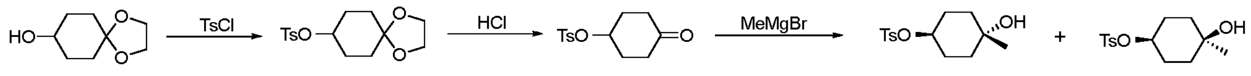


1 2 3 4 5

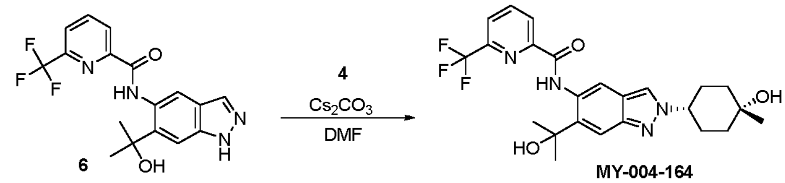


6 MY-004-164

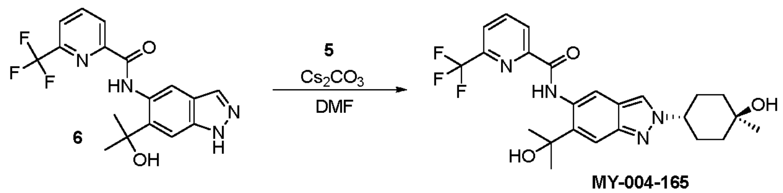


6 MY-004-165

1. Synthesis of compound 2 in Example 35

DMAP (4.25 g), TsCl (6.34 g) and triethylamine (6.39 g, 63.3 mmol) were added sequentially to a solution of compound 1 (5.0 g) in dichloromethane (100 mL) at 15 °C. The reaction system was stirred at 25 °C for 18 h. After the reaction, the mixture was added with dichloromethane (200 mL), and washed with water (100 mL × 2) and 1 M hydrochloric acid (100 mL × 3). The organic phase was dehydrated over anhydrous sodium sulfate, filtered and concentrated under reduced pressure. The residue was purified by silica gel column chromatography (petroleum ether:ethyl acetate=10:1) to give a white solid (18.0 g, 87% yield).

2. Synthesis of compound 3 in Example 35

1 M hydrochloric acid (100 mL) was added to a solution of compound 2 (5.0 g) in tetrahydrofuran (25 mL) at 15 °C. The reaction system was stirred at 25 °C for 20 h. At 0 °C, the mixture was adjusted to pH 9 with 1 M sodium hydroxide solution. Ethyl acetate (200 mL × 3) was added for extraction. The extracts were washed with saturated sodium chloride solution (100 mL), dehydrated over anhydrous sodium sulfate, filtered and concentrated under reduced pressure. The residue was purified by silica gel column chromatography (petroleum ether:ethyl acetate=5:1) to give a white solid (7.5 g, 87% yield).

3. Synthesis of compounds 4 and 5 in Example 35

To a solution of methylmagnesium bromide (12.4 mL, 37.31 mmol) in tetrahydrofuran (80 mL) was dropwise added a solution of compound 3 (5.0 g) in tetrahydrofuran (50 mL) at -40 °C. The reaction system was stirred at -40 °C for 4 h. After the reaction was quenched with a saturated ammonium chloride solution (50 mL), ethyl acetate (250 mL × 3) was added for extraction, and the extracts were washed with saturated saline (50 mL), dehydrated over anhydrous sodium sulfate, filtered and concentrated under reduced pressure. The residue was purified by silica gel column chromatography (petroleum ether:ethyl acetate=5:1) to give colorless oils of compound 4 (1.4 g, 14% yield) and compound 5 (2.3 g, 23% yield).

Compound 4: $^1$H NMR (400 MHz, $CDCl_3$) δ 7.79 (d, *J* = 8.4 Hz, 2H), 7.33 (d, *J* = 8.0 Hz, 2H), 4.74-4.64 (m, 1H), 2.44 (s, 3H), 1.92-1.79 (m, 2H), 1.77-1.62 (m, 4H), 1.49-1.38 (m, 2H), 1.23 (s, 3H).

Compound 5: $^1$H NMR (400 MHz, $CDCl_3$) δ 7.79 (d, *J* = 8.0 Hz, 2H), 7.32 (d, *J* = 8.4 Hz, 2H), 4.52-4.41 (m, 1H), 2.44 (s, 3H), 1.95-1.80 (m, 2H), 1.77-1.61 (m, 4H), 1.46-1.35 (m, 2H), 1.19 (s, 3H).

4. Preparation of compound MY-004-164 (195)

Compound 4 (500 mg), compound 6 (427 mg) and cesium carbonate (957 mg) were sequentially added to DMF (10 mL) at 20 °C. The reaction system was stirred at 90 °C for 18 h before the reaction was quenched with water (10 mL) at room temperature. Ethyl acetate was added for extraction (20 mL × 3), and the extracts were washed with

saturated sodium chloride solution (20 mL), dehydrated over anhydrous sodium sulfate, filtered and concentrated under reduced pressure. The residue was purified on a silica gel column (petroleum ether:ethyl acetate=2:1) to give a crude product (120 mg). The crude product was further purified by high performance liquid chromatography ($CH_3CN$:$H_2O$ (0.1% $NH_4HCO_3$)=5-95%, UV: 214 nm, flowrate: 15 mL/min) to give a white solid of compound MY-004-164 (22 mg, 3% yield).

Compound 195: $^1H$ NMR (400 MHz, $CDCl_3$): δ 12.27 (s, 1H), 8.86 (s, 1H), 8.49 (d, *J* = 7.6 Hz, 1H), 8.09 (t, *J* = 7.6 Hz, 1H), 7.94 (s, 1H), 7.82 (d, *J* = 7.2 Hz, 1H), 7.73 (s, 1H), 4.44-4.32 (m, 1H), 2.41-2.24 (m, 3H), 2.16-2.04 (m, 2H), 1.91-1.84 (m, 2H), 1.79 (s, 6H), 1.71-1.63 (m, 2H), 1.38-1.28 (m, 4H). LCMS: Rt = 9.525 min, $[M+H]^+$= 477.3.

5. Preparation of compound MY-004-165 (196)

Compound 5 (500 mg), compound 6 (427 mg) and cesium carbonate (957 mg) were sequentially added to DMF (10 mL) at 18 °C. The reaction system was stirred at 90 °C for 18 h before the reaction was quenched with water (10 mL) at room temperature. Ethyl acetate was added for extraction (15 mL × 3), and the extracts were washed with saturated sodium chloride solution (20 mL), dehydrated over anhydrous sodium sulfate, filtered and concentrated under reduced pressure. The residue was purified by preparative high performance liquid chromatography ($CH_3CN$:$H_2O$ (0.1% $NH_4HCO_3$)=20-70%, UV: 214 nm, flowrate: 15 mL/min) to give a white solid of compound MY-004-165 (70 mg, 9% yield).

Compound 196: $^1H$ NMR (400 MHz, $CDCl_3$): δ 12.28 (s, 1H), 8.86 (s, 1H), 8.49 (d, *J* = 8.0 Hz, 1H), 8.09 (t, *J* = 7.8 Hz, 1H), 7.93 (s, 1H), 7.83 (d, *J* = 7.6 Hz, 1H), 7.74 (s, 1H), 4.47-4.42 (m, 1H), 2.27-2.16 (m, 5H), 1.92-1.83 (m, 2H), 1.80 (s, 6H), 1.76-1.66 (m, 2H), 1.44 (s, 1H), 1.40 (s, 3H). LCMS: Rt = 3.030 min, $[M+H]^+$= 477.3.

Example 36. Synthesis of Compound MY-004-167 (198)

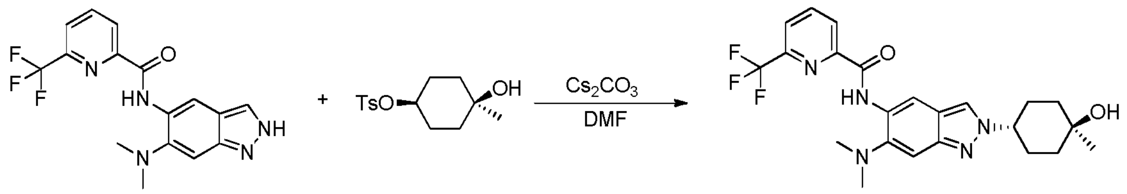


1 2 MY-004-167

Compound 1 (4.5 g), compound 2 (5.5 g), and cesium carbonate (10.5 g) were sequentially added to DMF (100 mL) at 25 °C. The reaction system was stirred at 90 °C for 18 h before the reaction was quenched by adding water (200 mL) at 0 °C. Ethyl acetate was added for extraction (200 mL × 3), and the extracts were washed with saturated saline (200 mL), dehydrated over anhydrous sodium sulfate, filtered and concentrated under reduced pressure. The residue was purified on a silica gel column (petroleum ether:ethyl acetate=1:1) to give a crude product of 2.0 g. The crude product was further purified by preparative high performance liquid chromatography ($CH_3CN$:$H_2O$ (0.1%$NH_4HCO_3$)=20-60%, UV: 214 nm, flowrate: 15 mL/min) to give a yellow solid of compound MY-004-167 (1.34 g, 23% yield).

Compound 198: $^1H$ NMR (400 MHz, $CDCl_3$): δ 11.23 (s, 1H), 8.83 (s, 1H), 8.50 (d, *J* = 8.0 Hz, 1H), 8.12 (t, *J* = 8.0 Hz, 1H), 7.91 (s, 1H), 7.85 (d, *J* = 8.0 Hz, 1H), 7.47 (s, 1H), 4.49-4.35 (m, 1H), 2.81 (s, 6H), 2.30-2.09 (m, 4H), 1.93-1.84 (m, 2H), 1.78-1.73 (m, 2H), 1.51 (s, 1H), 1.40 (s, 3H). LCMS: Rt = 3.977 min, $[M+H]^+$= 462.2.

Example 37. Synthesis of Compound MY-004-229 (1104)

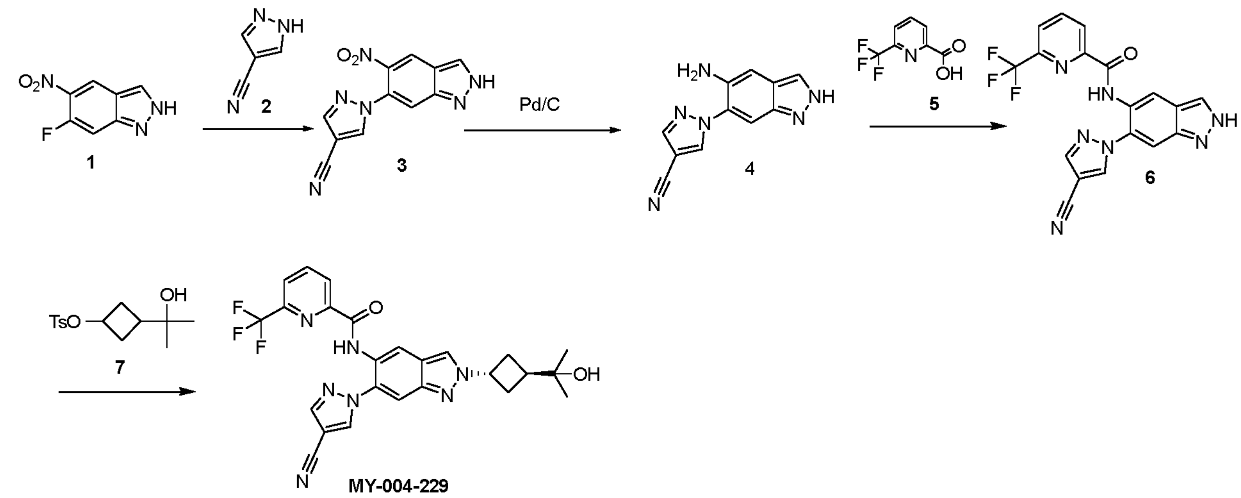


1. Synthesis of compound 3 in Example 37

Compound 1 (650 mg), compound 2 (1.68 g) and cesium carbonate (2.96 g) were sequentially added to 15 mL of DMSO at 15 °C. The reaction system was stirred at 120 °C for 18 h. The reaction was monitored by LCMS until a product peak of about 85%. The reaction was quenched with 10 mL of water and ethyl acetate was added for extraction (30 mL × 2). The organic phases were combined and concentrated and the residue was purified by preparative high performance liquid chromatography ($CH_3CN$:$H_2O$ (0.1% $NH_4HCO_3$)=5-95%, UV: 214 nm, flowrate: 15 mL/min) to give a crude yellow solid (570 mg, 62% yield).

2. Synthesis of compound 4 in Example 37

Compound 3 (570 mg) and palladium on carbon (320 mg, 10%) were added sequentially to 150 mL of ethyl acetate at 15 °C. The reaction system was stirred at 15 °C for 18 h. The mixture was filtered through celite to remove palladium on carbon, and the filtrate was concentrated to give a yellow solid (410 mg, 78% yield).

3. Synthesis of compound 6 in Example 37

EDCI (390 mg), compound 4 (300 mg) and compound 5 (262 mg) were added sequentially to pyridine (20 mL) at 15 °C. The reaction system was stirred for 18 h at 15 °C. The mixture was concentrated and the residue was resuspended in 40 mL of methanol/water (1:1) and purified to give a yellow solid (400 mg, 72% yield).

4. Preparation of compound MY-004-229 (1104)

Cesium carbonate (368 mg) was added to a solution of compound 6 (180 mg) and compound 7 (142 mg) in DMF (10 mL) at 15 °C. The mixture was stirred at 85 °C for 6 h. The reaction was quenched with 10 mL of water. Ethyl acetate was added for extraction (10 mL × 4), and the organic phases were combined and concentrated under reduced pressure. The residue was purified by preparative high performance liquid chromatography ($CH_3CN$:$H_2O$ (0.1% $NH_4HCO_3$)=5-95%, UV: 214 nm, flowrate: 15 mL/min) to give a crude product of 23 mg, which was purified by preparative silica gel thin layer chromatography (dichloromethane:methanol=20:1) to give a white solid (17 mg, 7% yield).

Compound 1104: $^1H$ NMR (400 MHz, $CDCl_3$): δ 11.45 (s, 1H), 8.95 (s, 1H), 8.45 (d, *J* = 7.6 Hz, 1H), 8.24 (d, *J* = 7.6 Hz, 2H), 8.13-8.07 (m, 2H), 7.86 (d, *J* = 7.6 Hz, 1H), 7.77 (s, 1H), 5.14-5.10 (m, 1H), 2.78-2.61 (m, 5H), 1.25 (s, 6H). LCMS: Rt =3.782 min, $[M+H]^+$= 510.2.

Example 38. Synthesis of Compounds MY-004-322 (1105) and MY-004-326 (1106)

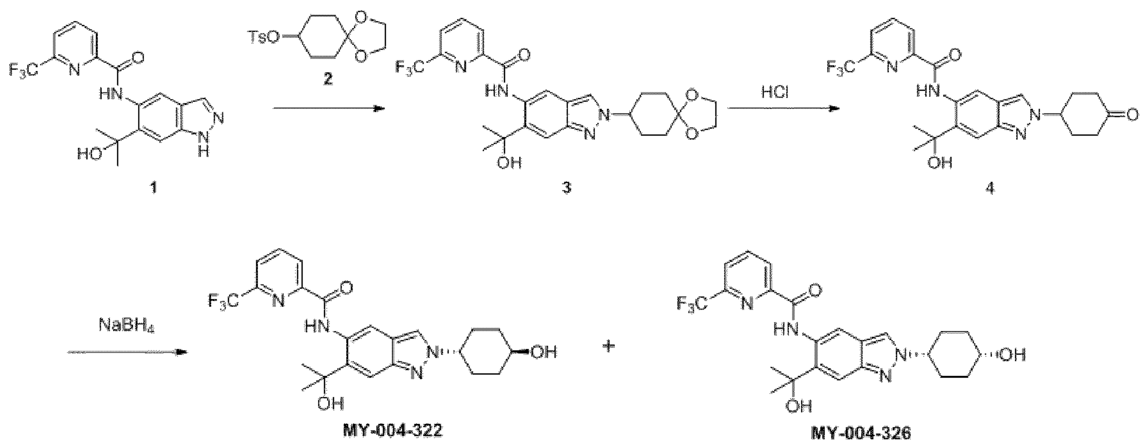

1 3 4

MY-004-322 MY-004-326

1. Synthesis of compound 3 in Example 38

Compound 1 (1.5 g), compound 2 (3.21 g), and $Cs_2CO_3$ (4.03 g) were sequentially added to DMF (60 mL) at 25 °C. The reaction system was stirred at 90 °C for 18 h. At 0 °C, water (50 mL) was added to quench the reaction. Ethyl acetate was added for extraction (100 mL × 3), and the extracts were washed with saturated saline (50 mL), dehydrated over anhydrous sodium sulfate, filtered and concentrated under reduced pressure. The residue was purified on a silica gel chromatographic column (petroleum ether:ethyl acetate=2:1) to give a pale yellow solid (550 mg, 26% yield).

2. Synthesis of compound 4 in Example 38

1 M hydrochloric acid (15 mL) was added dropwise to a solution of compound 3 (500 mg) in dioxane (15 mL) at 0 °C and the reaction system was stirred at 25 °C for 18 h. Ethyl acetate was added for extraction (30 mL × 3), and the extracts were washed with saturated saline (30 mL), dehydrated over anhydrous sodium sulfate, filtered and concentrated under reduced pressure. The residue was purified by silica gel column chromatography (petroleum ether:ethyl acetate=2:1) to give a pale yellow solid (340 mg, 75% yield).

3. Preparation of compounds MY-004-322 (1105) and MY-004-326 (1106)

Sodium borohydride (50 mg) was added to a solution of compound 4 (200 mg) in methanol (10 mL) at 0 °C and the reaction system was stirred at 25 °C for 2 h. At 0 °C, the reaction was quenched with 1 M hydrochloric acid. Ethyl acetate was added for extraction (30 mL×3), and the extracts were washed with saturated saline (30 mL), dehydrated over anhydrous sodium sulfate, filtered and concentrated under reduced pressure. The residue was purified by preparative high performance liquid chromatography ($CH_3CN$:$H_2O$ (0.1% $NH_4HCO_3$)=20-80%, UV: 214 nm, flowrate: 15 mL/min) to give pale yellow solids of MY-004-322 (110 mg, 55% yield) at Rt=8.60 min and MY-004-326 (19 mg, 9% yield) at Rt=10.03 min.

$^1$H NMR (400 MHz, DMSO-*d*6) (MY-004-322) : δ 12.36 (s, 1H), 8.70 (s, 1H), 8.45 (d, *J* = 8.0 Hz, 1H), 8.40-8.30 (m, 2H), 8.15 (d, *J* = 7.6 Hz, 1H), 7.57 (s, 1H), 5.93 (s, 1H), 4.70 (d, *J* = 8.0 Hz, 1H), 4.49-4.37 (m, 1H), 3.60-3.48 (m, 1H), 2.13-2.03 (m, 2H), 2.02-1.92 (m, 4H), 1.61 (s, 6H), 1.47-1.32 (m, 2H). LCMS: Rt =3.440 min, $[M+H]^+$= 463.2.

$^1$H NMR (400 MHz, DMSO-*d*6) (MY-004-326): δ 12.35 (s, 1H), 8.71 (s, 1H), 8.45 (d, *J* = 8.0 Hz, 1H), 8.36 (t, *J* = 8.0 Hz, 2H), 8.15 (d, *J* = 7.6 Hz, 1H), 7.58 (s, 1H), 5.93 (s, 1H), 4.53-4.30 (m, 2H), 3.92-3.85 (m, 1H), 2.37-2.22 (m, 2H), 1.90-1.73 (m, 4H), 1.70-1.57(m, 8H). LCMS: Rt =3.480 min, $[M+H]^+$= 463.2.

Example 39. Synthesis of Compounds MY-004-217 (1107) and MY-004-218 (1108)

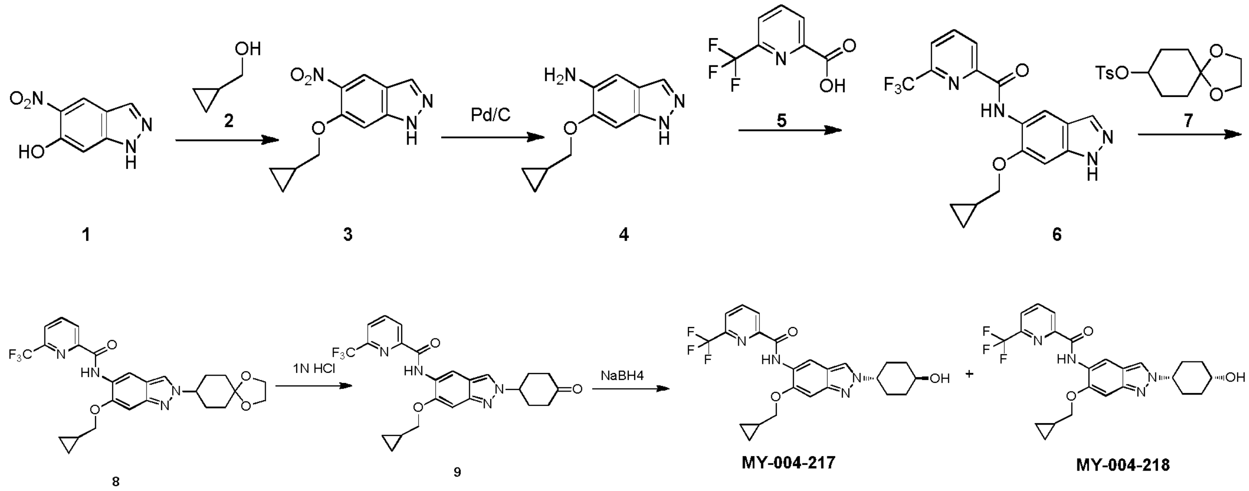


1 3 4 6

8 9 MY-004-217 MY-004-218

1. Synthesis of compound 3

$PPh_3$ (1.3 g) was added to a solution of compound 1 (600 mg) and compound 2 (289 mg) in THF (20 mL) in ice bath. The reaction system was stirred for 5 min. DIAD (1.0 g) was dropwise added to the reaction, and the system was stirred for 30 min at 0 °C and for 18 h at 30 °C. The reaction was quenched with 30 mL of water. Ethyl acetate was added for extraction (15 mL × 3), and the organic phase was concentrated under reduced pressure. The residue was purified by silica gel column chromatography (PE/EA=1:1) to give a yellow solid (500 mg, 64% yield).

2. Synthesis of compound 4

Pd/C (150 mg, 10%) was added to a solution of compound 3 (440 mg) in ethyl acetate (30 mL) at 15 °C and the reaction system was stirred at 25 °C for 18 h in hydrogen atmosphere at 760 Torr. The mixture was filtered and concentrated under reduced pressure to give a yellow solid (343 mg, 90% yield).

3. Synthesis of compound 6

EDCI (587 mg) was added to a solution of compound 4 (100 mg) and compound 5 (191 mg) in pyridine (10 mL) at 15 °C. The reaction system was stirred at 15 °C for 18 h. The mixture was concentrated under reduced pressure, and the crude product was purified by silica gel column chromatography (PE/EA=5/1 to PE/EA=2/1) to give a white solid (400 mg, 52% yield).

4. Synthesis of compound 8

Cesium carbonate (108 g) was added to a solution of compound 6 (500 mg) and compound 7 (622 mg) in DMF (20 mL) at 15 °C. The mixture was stirred at 90 °C for 18 h. The mixture was cooled and added with water (15 mL), and ethyl acetate was added for extraction (15 mL × 4). The organic phase was concentrated under reduced pressure, and the residue was purified by preparative high performance liquid chromatography ($CH_3CN$:$H_2O$ (0.1% $NH_4HCO_3$)=5-95%, UV: 214 nm, flowrate: 15 mL/min) to give a yellow solid (120 mg, 18% yield).

5. Synthesis of compound 9

Compound 8 (120 mg) was added to a mixture of 24 mL of 1 N HCl and 6 mL of THF at 15 °C. The reaction was stirred for 16 h at 30 °C, and adjusted to pH >7 with saturated sodium bicarbonate. Dichloromethane was added for extraction (15 mL × 4), and the organic phase was concentrated under reduced pressure. The crude product was purified by preparative high performance liquid chromatography ($CH_3CN$:$H_2O$ (0.1% $NH_4HCO_3$)=5-95%, UV: 214 nm, flowrate: 15 mL/min) to give a white solid (80 mg, 73% yield).

6. Synthesis of compounds MY-004-217 (1107) and MY-004-218 (1108)

$NaBH_4$ (18 mg) was added to a solution of compound 9 (75 mg) in MeOH (15 mL) at 15 °C, and the reaction was stirred for 3 h at 15 °C. The mixture was concentrated under reduced pressure, and the residue was purified by

preparative high performance liquid chromatography ($CH_3CN:H_2O$ (0.1% $NH_4HCO_3$)=5-95%, UV: 214 nm, flowrate: 15 mL/min) to give yellow solids of compound MY-004-217 (33 mg, 27% yield) at Rt=10.10 min and compound MY-004-218 (5 mg, 6% yield) at Rt=11.85 min.

$^1H$ NMR (400 MHz, $CDCl_3$) (MY-004-217) : δ 10.88 (s, 1H), 8.85 (s, 1H), 8.50 (d, *J* = 8.0 Hz, 1H), 8.12 (t, *J*= 8.0 Hz, 1H), 7.86 (s, 1H), 7.85 (s, 1H), 6.99 (s, 1H), 4.37-4.31 (m, 1H), 3.96 (d, *J* = 6.4 Hz, 2H), 3.84-3.78 (m, 1H), 2.30-2.17 (m, 4H), 2.10-1.99 (m, 2H), 1.62-1.49 (m, 2H), 1.46-1.42 (m, 1H), 0.77-0.72 (m, 2H), 0.46-0.41 (m, 2H). LCMS: Rt = 4.023 min, $[M+H]^+$= 475.2.

$^1H$ NMR (400 MHz, $CDCl_3$) (MY-004-218) : δ 10.88 (s, 1H), 8.85 (s, 1H), 8.50 (d, *J* = 8.0 Hz, 1H), 8.11 (t, *J* = 8.0 Hz, 1H), 7.91 (s, 1H), 7.85 (d, *J* = 7.6 Hz, 1H), 6.99 (s, 1H), 4.41-4.31 (m, 1H), 4.15 (s, 1H), 3.97 (d, *J* = 7.2 Hz, 2H), 2.41-2.31 (m, 2H), 2.10-1.97 (m, 4H), 1.81-1.72 (m, 2H), 1.47-1.41 (m, 1H), 0.77-0.72 (m, 2H), 0.46-0.41 (m, 2H). LCMS: Rt = 3.306 min, $[M+H]^+$= 475.2.

In addition, with reference to the above examples, the following compound Compound MYA (MY-004-001) was synthesized:

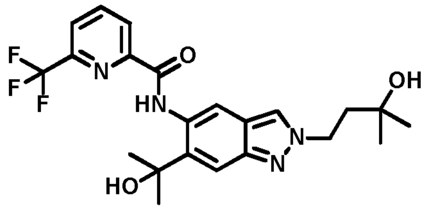

MYA (MY-004-001)

## Efficacy Examples

Example 40. Inhibition of human IRAK4 by the compounds according to the present disclosure Major experimental materials:

ATP (Sigma), DMSO (Sigma), EDTA (Sigma), HEPES (Sigma), DTT (Sigma), and Brij-35 (Sigma).

Procedures:

The inhibitory activities of the compounds on IRAK4 at the Km concentration of ATP were measured in the IRAK4 MSA (Mobility-Shift Assay, a mobility detection of microfluidic chip technology) as described below.

A recombinant fusion protein of N-terminal GST (glutathione-S-transferase) and human IRAK4 was used as enzyme (GST-IRAK4, kinase IRAK4 (Carna)) at a final concentration of 1 nM; ATP (Sigma) was at a final concentration of 37 μM; the substrates used for the kinase reaction were a 5-FAM (5-carboxyfluorescein)-labeled polypeptide (5-FAM-IPTSPITTTYFFFKKK-COOH) and a substrate peptide FAM-P8 (GL Biochem) at final concentrations of 5 μM.

In this assay, 500 μM solutions of the compounds were prepared in 100% DMSO, and serially 4-fold diluted to the 10th concentration gradient with 100% DMSO, followed by a 10-fold dilution in the compound buffer (50 mM HEPES, pH 7.5, 0.00015% Brij-35) to give intermediate dilutions of the compounds at a final concentration of 10 μM-0.04 nM containing 10% DMSO resectively. 5 μL of the dilutions were transferred into a black 384-well plate respectively.

IRAK4 was diluted to 2.5 nM in the kinase buffer (50 mM HEPES, pH 7.5, 0.00015% Brij-35, 2 mM DTT). 10 μL of the IRAK4 dilution was transferred to the 384-well plate and co-incubated with the compound for 10-15 min.

The substrate and ATP were diluted to 12.5 μM and 92.5 μM with reaction buffer (50 mM HEPES, pH 7.5, 0.00015% Brij-35, 10 mM $MgCl_2$), respectively. 10 μL of the dilutions were transferred to a 384-well plate and incubated at 28 °C for 1 h respectively. The reaction was terminated by adding 25 μL of 50 mM EDTA to the 384-well plate.

The inhibition rates of IRAK4 by the compounds were calculated by measuring the rate of phosphorylated substrate using a Caliper EZ Reader (PerkinElmer) and the $IC_{50}$ values were calculated by XL-fit software.

Results

$IC_{50}$ values of the compounds according to the present disclosure inhibiting human IRAK4 kinase activity are shown in the following Table 1.

Table 1. $IC_{50}$ for inhibiting human IRAK4 kinase

| Compound No. | $IC_{50}$ (nM) | Compound No. | $IC_{50}$ (nM) |
|---|---|---|---|
| MY-004-126-1 | 2.7 | MY-004-126-2 | 1.0 |
| MY-004-017 | 36 | MY-004-127-1 | 5.3 |
| MY-004-040 | 13 | MY-004-127-2 | 4.3 |
| MY-004-041 | 26 | MY-004-128 | 6.6 |
| MY-004-069 | 1.1 | MY-004-153 | 1.9 |
| MY-004-075 | 3.6 | MY-004-154 | 1.6 |
| MY-004-082 | 5 | MY-004-168 | 6.3 |
| MY-004-084 | 9.5 | MY-004-144 | 4.4 |
| MY-004-089 | 6.8 | MY-004-146 | 6.4 |
| MY-004-090 | 12.3 | MY-004-164 | 3.1 |
| MY-004-093 | 13 | MY-004-165 | 4.2 |
| MY-004-094 | 41 | MY-004-167 | 2.7 |
| MY-004-099 | 35 | MY-004-217 | 2.2 |
| MY-004-100 | 11 | MY-004-218 | 5.1 |
| MY-004-109 | 5.8 | MY-004-227 | 1.7 |
| MY-004-110 | 48 | MY-004-228 | 2 |
| MY-004-115 | 2.0 | MY-004-229 | 8.4 |
| MY-004-118 | 16.7 | MY-004-322 | 2.4 |
| MY-004-119 | 14.0 | MY-004-326 | 6.9 |

As can be seen from Table 1, the compounds according to the present disclosure have a significant inhibitory effect on human IRAK4 activity.

Example 41. Inhibitory effect on human IRAK1 activity and selectivity for IRAK4 of the compounds according to the present disclosure

This assay evaluated the inhibitory effect of the compounds on human IRAK1 activity with the same major materials as in Example 39.

The inhibitory activities of the compounds on IRAK1 at the Km concentration of ATP were measured in IRAK1 MSA (Mobility-Shift Assay, a mobility detection of microfluidic chip technology) as described below. A recombinant fusion protein of N-terminal GST (glutathione-S-transferase) and human IRAK1 were used as enzyme (GST-IRAK1, kinase IRAK1, Carna) at a final concentration of 3 nM; ATP (Sigma) was at a final concentration of 97 μM; the substrates used for the kinase reaction were 5-FAM (5-carboxyfluorescein)-labeled polypeptide (5-FAM-IPTSPITTTYFFFKKK-COOH) and substrate peptide FAM-P8 (GL Biochem) at final concentrations of 5 μM.

In this assay, 500 μM stock solutions of the compounds were prepared in 100% DMSO, and serially 4-fold diluted to the 10th concentration gradient with 100% DMSO, followed by a 10-fold dilution in the compound buffer (50 mM HEPES, pH 7.5, 0.00015% Brij-35) to give intermediate dilutions of the compounds at a final concentration of 10 μM-0.04 nM containing 10% DMSO, respectively. 5 μL of the intermediate dilutions were transferred into a black 384-well plate.

IRAK1 was diluted to 7.5 nM in the kinase buffer (50 mM HEPES, pH 7.5, 0.00015% Brij-35, 2 mM DTT). 10 μL of the IRAK1 dilution was transferred to the 384-well plate and co-incubated with the compound for 10-15 min.

The substrate and ATP were diluted to 12.5 μM and 242.5 μM with reaction buffer (50 mM HEPES, pH 7.5, 0.00015% Brij-35, 10 mM $MgCl_2$), respectively. 10 μL of the dilutions was transferred to a 384-well plate and incubated at 28 °C for 1 h. The reaction was terminated by adding 25 μL of 50 mM EDTA to the 384-well plate.

The inhibition rates of IRAKI by the compounds were calculated by measuring the rate of phosphorylated substrate using a Caliper EZ Reader (PerkinElmer) and the $IC_{50}$ values were calculated by XL-fit software.

Results show that the compounds according to the present disclosure have significant selective inhibitory activity on IRAK4, and the $IC_{50}$ (nM) ratio of IRAKI to IRAK4 is more than 500, preferably more than 200. In particular, some exemplary compound activity values are shown below: $IC_{50}$ values for the compounds disclosed herein inhibiting human IRAK1 activity are shown in the following Table 2.

Table 2. $IC_{50}$ for inhibiting human IRAK1 activity and selectivity for IRAK4

| Compound no. | IRAKI $IC_{50}$ (nM) | $IC_{50}$ (IRAK1)/ $IC_{50}$ (IRAK4) |
|---|---|---|
| MYA | 2820 | 470 |
| MY-004-109 | 4319 | 745 |
| MY-004-164 | 4127 | 1331 |

As can be seen from Table 2, the compounds disclosed herein have good selectivity for human IRAK4.

Example 42. Inhibition of cytokine TNF-α in LPS-induced human PBMCs by the compounds according to the present disclosure

Major experimental materials: fresh human PBMCs (peripheral blood mononuclear cells); RPMI 1640 medium (Gibco, 10% fetal bovine serum, 100 U/mL penicillin, 100 μg/mL streptomycin)

Major materials: fetal bovine serum, penicillin, streptomycin, LPS (lipopolysaccharide), Human TNF-α ELISA Kit (Beyotime Biotech), and DMSO

Procedures: Cytokine production in human PBMCs (peripheral blood mononuclear cells) was induced by LPS (lipopolysaccharide) *in vitro,* and the inhibitory effect of the compounds on induced cytokine production in human PBMCs was investigated.

Fresh human PBMC was centrifuged for 10 min at room temperature at 450× g and the supernatant was discarded. The PBMC was resuspended in complete RPMI 1640 medium.

The assay was performed in complete medium. PBMC was seeded at a density of $1\times10^5$ cells/well into a 96-well plate. The compounds according to the present disclosure were serially diluted in an isotonic 100% DMSO solution to 8 concentrations from 20 μM to 0.002 nM respectively, with the final DMSO concentration being 0.25%. Cells were pre-incubated with the formulated compounds for 30 min at 37 °C prior to stimulation. To induce cytokine secretion, cells were stimulated with 0.1 μg/mL LPS (Sigma, *Escherichia coli* O111:B4) for 4 h at 37 °C. The cell culture supernatant was then separated by centrifugation at 450× g for 10 min at room temperature.

The amount of secreted TNF-α in the supernatants was determined using the human TNF-α ELISA kit according to the manufacturer's instruction.

The readings of absorbance at A450 were measured with a microplate reader SpectraMax i3x (Molecular Device) for calculating the inhibition of the compounds, and $IC_{50}$ values were calculated with GraphPad Prism 7.0 software respectively.

Table 3. Inhibition of cytokine TNF-α in LPS-induced human PBMC

| Compound No. | $IC_{50}$ (nM, TNF-α inhibition) | Compound No. | $IC_{50}$ (nM, TNF-α inhibition) |
|---|---|---|---|
| MYA | 208.3 | MY-004-126-1 | 71.84 |
| MY-004-069 | 74.98 | MY-004-153 | 190.60 |
| MY-004-089 | 164.60 | MY-004-164 | 145.90 |

Results show that the compounds have significant inhibitory effect on cytokine TNF-α in LPS-induced human PBMCs.

Example 43. Inhibition of cytokine TNF-α release in LPS-induced Balb/c female mice by the compounds according to the present disclosure (administered 5 h before LPS induction)

Female Balb/c mice were randomized into several groups, of which each group contained 4 mice, and these groups including a normal control + vehicle group, a model + vehicle group, a model + positive drug group (the positive drug was the compound MYA) and a model + test compound group. In the normal control groups, the animals received an intraperitoneal injection of normal saline (10 mL/kg) and in the model groups, the animals received LPS stimulation

(Sigma CAT# L2630, i.p., 10 mL/kg, 0.2 mg/kg). During the assay, to the test compounds, DMSO, Solutol and 10 mM PBS were sequentially added to prepare a solution or suspension of required concentration for administration. For the vehicle, DMSO, Solutol and 10 mM PBS were mixed in a volume ratio of 5:15:80. In each of the groups, the animals were administered through oral gavage (10 mL/kg) 5 h before LPS (or saline) stimulation at predetermined doses, and euthanized with $CO_2$ at 1.5 h after the stimulation for cardiac blood collection. The whole blood was not anticoagulated, incubated in wet ice for 1.5 h, and centrifuged at 2000× g at 4 °C for 10 min to separate the serum. The serum was frozen at -80 °C for TNF-α assay. Quantification of the TNF-α was done with TNF-α ELISA kit according to the manufacturer's instruction.

Table 4. Inhibition of cytokine TNF-α release in LPS-induced Balb/c female mice (administered 5 h before LPS induction)

| Compound No. | Inhibition rate of TNF-α | Compound No. | Inhibition rate of TNF-α |
|---|---|---|---|
| MYA | 86.25% | MY-004-127-1 | 89.29% |
| MY-004-126-1 | 94.43% | MY-004-228 | 88.46% |
| MY-004-126-2 | 94.11% | MY-004-153 | 88.42% |
| MY-004-075 | 90.15% | MY-004-154 | 87.82% |
| MY-004-089 | 91.27% | MY-004-164 | 94.00% |
| MY-004-115 | 89.59% | MY-004-165 | 88.99% |

As shown in Table 4, the compounds according to the present disclosure have significant inhibitory effect on TNF-α release in LPS-induced Balb/c female mice.

Example 44. Inhibition of IL-6 release in LPS-induced Balb/c female mice by the compounds according to the present disclosure (administered 5 h before LPS induction)

Objective of the test: To determine the inhibitory effect of the compounds by measuring the plasma IL-6 concentration in a Balb/c mouse model with LPS-induced inflammation.

2. Experimental Materials:

2.1 Prepared mouse serum samples (sample batch no. 20181218)
2.2 Mouse IL-6 ELISA kit (Beyotime, CAT No. PI326, Lot No.: 040918181104)

3. Equipment and instruments: microplate reader: SpectraMax i3x (Molecular Device)

4. Procedures:

4.1 Reagent kit was equilibrated at room temperature (25-28 °C) for 20 min. After each detection, the remaining reagents were stored at 4 °C.
4.2 Preparing washing buffer of appropriate amount: The stock washing buffer (20×) was diluted to 1× with double distilled water or deionized water, for example, 10 mL of stock washing buffer (20×) is mixed with 190 mL of water to give the 1× washing buffer.
4.3 Preparing reference standard: 1 vial of reference standard substance was added with labeled volume of reference standard diluent. The mixtures were incubated for 15 min at room temperature. Then the mixtures were gently mixed to completely dissolve the substance to prepare a reference standard stock at a final concentration of 1000 pg/mL. The stock was serially diluted in 1.5-mL centrifuge tubes to give seven standard dilutions at concentrations of 1000, 500, 250, 125, 62.5, 31.25 and 0 pg/mL.
4.4 Dilution of samples: The sample in each group wa diluted by 8 fold, i.e., a 2-fold dilution with sample assay buffer followed by another two-fold dilution with standard diluent.
4.5 The diluted samples or standards of different concentrations were added into corresponding wells at 100 μL/well

respectively, and a background correcting well (or a blank well) was set by adding only TMB buffer and terminating solution. The wells were sealed with a transparent film and incubated at room temperature for 120 min.
4.6 The plate was subjected to 5 washes, each with 300 μL of washing buffer for 15-30 sec. The plate was dried on thick absorbent paper after the last wash.
4.7 Biotinylated antibody was added at 100 μL/well (Note: the biotinylated antibody was prepared in advance and could be used directly without further dilution). The wells were sealed with a transparent film and incubated at room temperature for 60 min.
4.8 The plate was subjected to 5 washes, each with 300 μL of washing buffer for 15-30 sec. The plate was dried on thick absorbent paper after the last wash.
4.9 Horseradish peroxidase-labeled streptavidin was added at 100 μL/well. The wells were sealed with a white film and incubated away from light at room temperature for 20 min.
4.10 The plate was subjected to 5 washes, each with 300 μL of washing buffer for 15-30 sec. The plate was dried on thick absorbent paper after the last wash.
4.11 The chromogenic reagent TMB was added 100 μL/well. The wells were sealed with a white film and incubated away from light at room temperature for 20 min. At a lower room temperature, the incubation time should be prolonged appropriately until the reference standards and the samples demonstrated remarkable color change.
4.12 The terminating solution was added at 50 μL/well and A450 value was measured right after mixing.

The results are shown in the followiin Table 5.

Table 5. Inhibition of IL-6 release in LPS-induced Balb/c female mice (administered 5 h before LPS induction)

| Compound no. | Inhibition rate of IL-6 | Compound no. | Inhibition rate of IL-6 |
|---|---|---|---|
| MYA | 79.50% | MY-004-115 | 93.35% |
| MY-004-041 | 81.65% | MY-004-126-1 | 97.05% |
| MY-004-089 | 94.33% | MY-004-126-2 | 93.39% |
| MY-004-075 | 96.88% | MY-004-127-1 | 83.28% |

As shown in the table, the compounds according to the present disclosure have significant inhibitory effect on IL-6 release in LPS-induced Balb/c female mice.

Example 45. Inhibition of hERG by the compounds disclosed herein

This study evaluated the cardiac safety of the compounds according to the present disclosure, and an HEK-293 cell line stably expressing hERG potassium ion channel was used for detection.
Instrument Information:

Amplifier: purchased from HEKA (Germany), EPC10
Micromanipulator: purchased from Sutter Instruments (USA), MP225
Micropipette puller: purchased from Sutter Instruments (USA), P97
Microscope: purchased from Nikon, TE300
Capillary glass tubing: purchased from Sutter Instruments (USA), BF150-86-10
Data acquisition and analysis software: PatchMaster, Igor Pro 6.0 and GraphPad Prism 5.0

Procedures: Test compound stocks were diluted in DMSO into 0.3 mM, 1 mM, and 3 mM dilutions respectively. Test compound stocks were diluted in an extracellular buffer (140 mM NaCl, 3.5 mM KCl, 1 mM $MgCl_2$, 2 mM $CaCl_2$, 10 mM glucose, 10 mM HEPES, 1.25 mM $NaH_2PO_4$, adjusted to pH 7.4 with NaOH) to give working solutions of the test compounds at concentrations of 0.3 μM, 1 μM, 3 μM, 10 μM and 30 μM respectively. The working solutions of the test compounds were ultrasonically treated for 20 min.
Patch clamping: Under an inverted microscope, recording electrodes were controlled by micromanipulator to contact with the cell. A negative voltage was applied to create a G-omega shaped connection. After forming the G-omega shaped connection, a rapid capacitance compensation was given. Under the continuous negative voltage, the cell membrane was ruptured to form a whole-cell recording configuration. In the whole-cell recording configuration, slow capacitance compensation was given, and the values of membrane capacitance and series resistance were recorded.
Voltage stimulation scheme for cellular hERG potassium current: The cell membrane clamping voltage was -80 mV; the voltage was first elevated from -80 mV to +30 mV, held for 2.5 sec, and rapidly raised to and held at -50

mV for 4 sec, thus exciting the hERG channel tail current. Data acquisition was performed every 10 sec. -50 mV was used for drain current detection.

The cover glasses planted with cells were placed in the recording chamber of an inverted microscope. Negative control and test compounds flowed through the recording chamber in an ascending order of concentration by gravity perfusion to quickly act on the cells. During the recording, the extracellular buffer was continuously circulated by a vacuum pump. The current detected in a cell in the negative control was used as the background for the cell. Each concentration was allowed to act for 5 min or until the current was stabilized. All experiments were performed at room temperature.

Data analysis:

The current of each concentration was normalized first $\left(\frac{\textit{Peak tail current of compound}}{\textit{Peak tail current of vehicle}}\right)$, and then the inhibition rate was calculated $\left(\left(1 - \frac{\textit{Peak tail current of compound}}{\textit{Peak tail current of vehicle}}\right)\right)$. Basic statistics were calculated for each concentration, including mean, standard deviation (SD), standard error (SE), and replicates (n). The dose-dependent curve was fitted with the following equation and the half maximal inhibitory concentration ($IC_{50}$) of the test compounds was calculated:

$$\text{inhibition} = \frac{1}{1 + \left(\frac{IC50}{C}\right)^h}$$

wherein C represents the test compound concentration, $IC_{50}$ represents the half maximal inhibitory concentration, and h represents the Hill coefficient. Curve fitting and calculation of $IC_{50}$ were done by GraphPad Prism 5.0 software.

The results demonstrate low inhibition rates of the compounds according to the present disclosure for human hERG, which are even significantly superior to the competitor MYA. The inhibition rate of the compounds at 30 μM for hERG is less than 50%, preferably less than 30% of that of the competitor. In particular, some exemplary compound inhibition rate values are as follows:

Table 6. inhibition for hERG at 30 μM concentration

| Compound No. | $IC_{50}$ for hERG inhibition | Compound No. | $IC_{50}$ for hERG inhibition |
|---|---|---|---|
| MYA | 27.10% ± 1.74% | MY-004-127-1 | 11.64% ± 1.12% |
| MY-004-069 | 24.14% ± 2.14% | MY-004-153 | 17.73% ± 0.23% |
| MY-004-126-1 | 12.82% ± 2.57% | MY-004-164 | 24.67% ± 2.93% |
| MY-004-126-2 | 10.11% ± 1.00% | | |

From the above results, the compounds according to the present disclosure have no significant inhibitory effect on human hERG.

Example 46. Mini-Ames assay of the compounds disclosed herein in *Salmonella typhimurium* Test strains and genotypes thereof

The strains used in this assay (Ames et al., 1975) are described in the following table:

| Test strains | Histidine mutations | Other mutations | | Resistance factor plasmids |
|---|---|---|---|---|
| | | DNA repair mutations | Membrane mutations | |
| TA98 | *his*D3052 | *uvr*B | *rfa* | pKM101 |
| TA100 | *his*G46 | *uvr*B | *rfa* | pKM101 |

Metabolic activation system

S9 homogenate: β-napthoflavone and phenobarbital induced rat liver microsome homogenate S9 fraction was used as the metabolic activation system for this assay. S9 was purchased from CHI Scientific, Inc. and stored in a freezer at -75 °C before use. Preparation of S9 homogenate: Male Sprague Dawley rats were intraperitoneally injected with a single dose of β-napthoflavone and phenobarbital. The tissue was collected for preparing the sample. According to the certificate of analysis for the S9 fraction provided by the CHI Scientific, Inc., the S9 had been subjected to bacterial reverse mutation test (*S. typhimurium* TA97, TA98 and TA100) for activating 2-aminofluorene at a concentration of 10% in S9 mixture. The ability of S9 to activate cyclophosphamide at a concentration of 1.5% in S9 mixture was also tested in a chromosome aberration test (Chinese hamster lung cells).

S9 mixture and mock mixture: S9 was quickly thawed to prepare a liquid mixture before the assay. The components per 10 mL of mixture are shown in the table below. 0.2 M PBS phosphate buffer (pH 7.4) was used as the mock mixture.

| Component | Volume |
|---|---|
| Sterile water | 3.35 mL |
| 0.2 M phosphate buffer, pH 7.4 | 5.0 mL |
| Saline (0.4 M $MgCl_2$-1.65 M KCl) | 0.2 mL |
| 0.1 M coenzyme II (NADP) | 0.4 mL |
| 1 M sodium glucose 6-phosphate solution | 0.05 mL |
| Liver S9 fraction | 1.0 mL |
| Total volume: | 10 mL |

Preparation of bacterial cultures

The overnight bacterial cultures were prepared from a stock of the strains. 80 μL of the stock was added to 20 mL of broth. For strains TA98 and TA100, 62.5 μL of ampicillin at a concentration of 0.8% were added per 10 mL of broth #2. The inoculated broth was then incubated on a thermostatic shaker overnight at 37 °C with a rotation speed of 160±10 rpm. The overnight cultures over the target absorbance were stored at 2-8 °C before use. The overnight cultures were used for genotyping and mutation tests.

The experimental system was exposed to the test compounds on plates in plate incorporation assay (Ames et al., 1975; N. Flamand et al., 2000). The Mini-Ames assay was developed from a standard Ames assay. It consumes a relatively smaller amount of test substance while evaluating the mutagenic potential.

At the tested doses (1.5, 4, 10, 25, 64, 160, 400 and 1000 μg/well, if the solubility of the compounds allows), the average numbers of revertant colonies induced by the test compounds MY-004-069, MY-004-126-2 and MY-004-127-1 were less than 2 times of the average number of the vehicle control group (for TA98 and TA100) with or without the S9 mixture. No dose-dependent increase in the average number of revertant colonies was observed. The results of the test compounds MY-004-069, MY-004-126-2 and MY-004-127-1 were all negative.

Example 47. Stability of the compounds according to the present disclosure in human, rat and mouse liver microsomes

Procedures

1. Preparation of buffer C:

    Buffer A: 1.0 L of 0.1 M monopotassium phosphate buffer (containing 1.0 mM EDTA);
    Buffer A: 1.0 L of 0.1 M dipotassium phosphate buffer (containing 1.0 mM EDTA);
    Buffer C: Buffer A was added to 700 mL of buffer B until pH reached 7.4.

2. Preparation of 10 mM stock solutions:
The test compound and the reference were separately dissolved in DMSO to give 10 mM stocks.
3. Preparation of dosing solution:

    500 μM solution: 10 μL of 10 mM stock solution was added to 190 μL of acetonitrile;

1.5 μM dosing solution (in liver microsome solution):
18.75 μL of 20 mg/mL liver microsomes were added to 479.75 μL of buffer C, followed by 1.5 μL of 500 μM solution. The mixture was gently shaken to mix well.

4. Preparation of 6 mM NADPH solution:
NADPH was weighed, to which an appropriate amount of buffer C was added to prepare a 6 mM NADPH solution.
5. 30 μL of 1.5 μM dosing solution was added to wells of a 96-well plate set for different time points (0 min, 5 min, 15 min, 30 min, 45 min) in duplicate.
6. Preparation of 0 min samples: 135 μL of acetonitrile (containing internal standard) was added to the 0 min wells, followed by 15 μL of 6 mM NADPH solution.
7. The 96-well plate containing 1.5 μM dosing solution and NADPH solution were preheated for 5 min in a water bath at 37 °C.
8. 15 μL of pre-heated 6 mM NADPH solution was added to wells set for 5 min, 15 min, 30 min and 45 min to start the reaction.
9. At time points of 5 min, 15 min, 30 min and 45 min, the reaction was terminated by adding 135 μL of acetonitrile (containing internal standard). The mixtures were vortexed for 10 min and centrifuged at 5594× g for 15 min (Thermo Multifuge × 3R).
10. 50 μL of the supernatant was transferred to wells of a 96-well plate containing 50 μL of water. The samples were mixed and finally subjected to LC-MS/MS analysis.

The results are shown in Table 7.

Table 7. Stability test in liver microsomes ($t_{1/2}$, min)

| Compound No. | Human | Rat | Mouse | Compound No. | Human | Rat | Mouse |
|---|---|---|---|---|---|---|---|
| MY-004-041 | 157 | 122 | 267 | MY-004-127-1 | 770 | 306 | 46051 |
| MY-004-069 | ∞ | ∞ | 2219 | MY-004-144 | ∞ | 683 | ∞ |
| MY-004-089 | 2138 | 292 | 564 | MY-004-153 | ∞ | 250 | 283 |
| MY-004-126-1 | 469 | 398 | 354 | MY-004-164 | ∞ | ∞ | ∞ |
| MY-004-126-2 | 25695 | ∞ | 802 | MY-004-165 | 3237 | ∞ | ∞ |
| The "∞" in Table 7 denotes that no obvious metabolism or the test compounds was found during assay. | | | | | | | |

The results show good stability of the compounds according to the present disclosure on human, rat and mouse liver microsomes.

Example 48. Inhibition of five major CYP450 enzyme subtypes CYP1A2, 2C9, 2C19, 2D6 and 3A4 in human liver microsomes by the compounds according to the present disclosure

This study was intended to investigate the inhibitory effect of the test compounds on five major CYP450 enzyme subtypes CYP1A2, 2C9, 2C19, 2D6 and 3A4 in human liver microsomes. The mixed human liver microsomes used in this assay were purchased from Corning (USA). The test compounds were co-incubated at 7 concentrations with human liver microsomes and five probe substrates (phenacetin for CYP1a2, diclofenac for CYP2C9, mephenytoin for CYP2C19, dextromethorphan for CYP2D6, midazolam for CYP3A4-M, mixed). See the table below. The reaction was initiated by adding coenzyme NADPH, and terminated by adding acetonitrile containing internal standard. After the proteins were precipitated, the supernatant was centrifuged. The characteristic metabolites in the supernatant (acetaminophen for CYP1A2, 4-hydroxydiclofenac for CYP2C9, 4-hydroxymephenytoin for CYP2C19, dextrorphan for CYP2D6, 1-hydroxymidazolam for CYP3A4-M) were analyzed by LC-MS/MS. The influence of the test compounds on production of the characteristic metabolites was finally analyzed on the basis of the data obtained. A selective inhibitor (ketoconazole for CYP3A4-M) was used as positive control. All samples were tested in duplicate.

Table 8. Inhibition of the compounds for five major CYP450 enzyme subtypes CYP1A2, 2C9, 2C19, 2D6 and 3A4 in human liver microsomes ($IC_{50}$, μM)

| Subtype / Compound No. | 1A2 | 2C9 | 2C19 | 2D6 | 3A4 |
|---|---|---|---|---|---|
| MYA | 2.91 | 19.06 | 23.48 | >30.0 | >30.0 |
| MY-004-126-1 | >30.0 | >30.0 | >30.0 | >30.0 | >30.0 |
| MY-004-127-1 | >30.0 | >30.0 | >30.0 | >30.0 | >30.0 |
| MY-004-164 | >30.0 | 25.6 | >30.0 | >30.0 | >30.0 |
| MY-004-165 | >30.0 | 20.9 | >30.0 | >30.0 | >30.0 |

As can be seen from the table, the compounds according to the present disclosure demonstrated weak or no significant inhibitory effect on the 5 human CYP subtypes. The compound MYA has significant inhibitory effect on the subtype 1A2, suggesting that the compounds MY-004-126-1, MY-004-127-1, MY-004-164 and MY-004-165 have significant superiority.

Example 49. PK assay of the compounds according to the present disclosure in rat

The rats used for pharmacokinetic study in the example were male SPF-grade SD rats (B&K Universal, Shanghai).

Route of administration: a single dose by oral gavage or intravenous injection

Sampling points: 0.083 h, 0.25 h, 0.5 h, 1 h, 2 h, 4 h, 6 h, 8 h and 24 h after administration
Sample processing: 0.2 mL of venous blood was collected, and let stand on ice before centrifugation to separate plasma (centrifugation conditions: 8000 rpm, 6 min, 4 °C). The separated plasma was stored at -80 °C before analysis.

Internal standard working solution: An appropriate amount of tolbutamide internal standard stock solution at 645,000 ng/mL was added into a volumetric flask, and was diluted to the volume with methanol. The solution was well mixed to give an internal standard working solution with a concentration of 50 ng/mL.

Sample pretreatment: 50 μL of plasma was added into a 1.5-mL centrifuge tube, and added with 250 μL of internal standard solution (methanol of the same volume for the blank control). The solution was well mixed by vortex, and centrifuged for 5 min at 14000 rpm. 200 μL of the supernatant was transferred into a 96-well sample feeding plate, and loaded onto a LC-MS/MS system.

LC conditions:

Column: ACQUITY UPLC BEH $C_{18}$ 1.7 μm (50 mm × 2.10 mm)
Mobile phase: A: 0.1% aqueous formic acid; B: 0.1% formic acid in acetonitrile
Flow rate: 0.5 mL/min
The data processing system was Analyst software (Applied Biosystems, USA, ver 1.5.5).

Table 9

| Compound No. | Pharmacokinetic test for intravenous injection | | | | | | | | |
|---|---|---|---|---|---|---|---|---|---|
| | Dose | Formulation | Peak concentration | Area under curve | Area under curve | Half life | Clearance | Volume of distribution at steady state | Average retention time |
| | Dose (mpk) | Formulation | $C_{max}$ (ng/mL) | $AUC_{0-t}$ (ng/mL*h) | $AUC_{0-\infty}$ (ng/mL*h) | $t_{1/2}$ (h) | CL (mL/min/kg) | Vz (L/kg) | MRT (h) |
| MY-004-069 | 1 | 5%DMSO+ 15%solutol+80% Saline | 903.74 | 2914.24 | 3027.43 | 5.48 | 5.51 | 2.69 | 5.99 |
| MY-004-082 | 1 | 5%DMSO+ 15%Solutol+80% Saline | 1318.46 | 789.97 | 793.06 | 0.51 | 21.89 | 0.93 | 0.52 |
| MY-004-089 | 1 | 5%DMSO+ 15%Solutol+80% Saline | 1581.18 | 3389.94 | 3512.73 | 5.5 | 4.89 | 2.26 | 5.2 |
| MY-004-126-1 | 1 | 5%DMSO+ 15%Solutol+80% Saline | 1207.27 | 3153.06 | 3247.61 | 5.25 | 5.14 | 2.34 | 5.46 |
| MY-004-126-2 | 1 | 5%DMSO+ 15%Solutol+80% Saline | 1428.1 | 4076.63 | 4541.04 | 7.59 | 3.74 | 2.40 | 8.81 |
| MY-004-127-1 | 1 | 5%DMSO+ 15%Solutol+80% Saline | 1415.73 | 5530.93 | 5879.53 | 6.29 | 2.84 | 1.55 | 7.54 |

Table 10

| Compound No. | Pharmacokinetic test for oral administration | | | | | | | | |
|---|---|---|---|---|---|---|---|---|---|
| | Dose | Formulation | Time to peak | Peak concentration | Area under curve | Area under curve | Half life | Average retention time | Bioavailability |
| | Dose (mpk) | Formulation | $t_{max}$ (h) | $C_{max}$ (ng/mL) | $AUC_{0-t}$ (ng/mL*h) | $AUC_{0-\infty}$ (ng/mL*h) | $t_{1/2}$ (h) | MRT (h) | F (%) |
| MY-004-069 | 2 | 5%DMSO+ 15%solutol+80% Saline | 4 | 413.49 | 5029.62 | 5770.55 | 7.26 | 11.28 | 86.29 |
| MY-004-082 | 2 | 5%DMSO+ 15%Solutol+80% Saline | 0.5 | 113.69 | 416.21 | 423.9 | 1.09 | 2.69 | 26.34 |
| MY-004-089 | 2 | 5%DMSO+ 15%Solutol+80% Saline | 4 | 226.7 | 2997.82 | 3547.37 | 8.11 | 12.58 | 44.22 |
| MY-004-126-1 | 5 | 15%solutol HS15+85%PBS | 4 | 923.9 | 11524.7 | 17674.8 | 15.25 | 22.44 | 73.1 |
| MY-004-126-2 | 5 | 15%solutol HS15+85%PBS | 4 | 1146.45 | 15449.5 | 35904.1 | 24.81 | 10.31 | 75.79 |
| MY-004-127-1 | 5 | 15%solutol HS15+85%PBS | 4.67 | 1479.5 | 18403.9 | 22461.6 | 8.84 | 9.16 | 66.54 |

All references mentioned in this application are incorporated herein by reference as if each was individually incorporated by reference. In addition, it should be understood that various modifications or changes may be made by those skilled in the art after reading the above teachings of the present disclosure, and these equivalent forms also fall within the scope defined by the claims appended hereto.

## Claims

1. A compound of formula I, or a cis-trans isomer, an optical isomer, a racemate, a pharmaceutically acceptable salt, a prodrug, a deuterated derivative, a hydrate thereof or a solvate thereof:

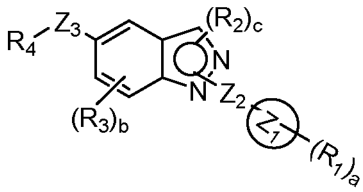

I

wherein in the formula I:

$Z_1$ is selected from substituted or unsubstituted $C_3$-$C_{12}$ cycloalkylenyl, and substituted or unsubstituted 3-12 membered heterocycloalkylenyl;
$Z_2$ is selected from a chemical bond (absent), substituted or unsubstituted $C_1$-$C_6$ alkylenyl, substituted or unsubstituted $C_2$-$C_6$ alkenylenyl, and substituted or unsubstituted $C_2$-$C_6$ alkynylenyl;
each $R_1$ is independently selected from hydroxyl, thiol, carboxyl, substituted or unsubstituted $C_1$-$C_{12}$ alkyl, substituted or unsubstituted $C_3$-$C_{12}$ cycloalkyl, substituted or unsubstituted $C_1$-$C_{12}$ hydroxyalkyl, substituted or unsubstituted $C_1$-$C_{12}$ thiolalkyl, substituted or unsubstituted $C_1$-$C_{12}$ aminoalkyl, substituted or unsubstituted $C_3$-$C_{12}$ hydroxycycloalkyl, substituted or unsubstituted $C_3$-$C_{12}$ thiolcycloalkyl, substituted or unsubstituted $C_3$-$C_{12}$ aminocycloalkyl, substituted or unsubstituted $C_1$-$C_{12}$ alkoxy, substituted or unsubstituted $C_1$-$C_{12}$ alkylthio, $-N(R_5)R_6$, substituted or unsubstituted $C_1$-$C_8$ carboxyl, substituted or unsubstituted $C_2$-$C_8$ acyl, substituted or unsubstituted $C_2$-$C_8$ ester group, and halogen;
$R_2$ and $R_3$ are each independently selected from a chemical bond, substituted or unsubstituted $C_1$-$C_{10}$ alkyl, substituted $C_3$-$C_{10}$ cycloalkyl, halogen, $C_1$-$C_{10}$ haloalkyl, $C_1$-$C_{12}$ hydroxyalkyl, cyano, nitro, $-A-R_7$, $-N(R_5)R_6$, substituted or unsubstituted $C_6$-$C_{12}$ aryl, and substituted or unsubstituted 5-12 membered heteroaryl;
$R_5$ and $R_6$ are each independently selected from hydrogen, substituted or unsubstituted $C_1$-$C_{10}$ alkyl, and substituted or unsubstituted $C_3$-$C_8$ cycloalkyl;
A is selected from a chemical bond, S and O;
$R_7$ is selected from hydrogen, substituted or unsubstituted $C_1$-$C_{10}$ alkyl, substituted or unsubstituted $C_2$-$C_{10}$ alkenylenyl, substituted or unsubstituted $C_3$-$C_8$ cycloalkyl, substituted or unsubstituted 3-12 membered heterocycloalkyl, substituted or unsubstituted 5-20 membered heteroaryl, and $-R_8-R_9$;
$R_8$ is selected from a chemical bond, substituted or unsubstituted $C_1$-$C_6$ alkylenyl, and substituted or unsubstituted $C_2$-$C_6$ alkenylenyl;
$R_9$ is selected from substituted or unsubstituted $C_3$-$C_{12}$ cycloalkyl, and substituted or unsubstituted $C_3$-$C_{12}$ heterocycloalkyl;
$Z_3$ is selected from carbonyl,

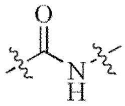

and

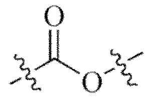

;

$R_4$ is selected from substituted or unsubstituted $C_6$-$C_{20}$ aryl, and substituted or unsubstituted 5-20 membered heteroaryl;
wherein each of the "substituted" means that one, two or more (preferably 1, 2, 3 or 4) hydrogen atoms on the group are substituted by a substituent selected from: $C_1$-$C_{12}$ hydroxyalkyl, $C_2$-$C_8$ acyl, $C_3$-$C_8$ cycloalkyl, $C_1$-$C_6$ alkyl, $C_1$-$C_6$ alkoxy, $C_1$-$C_6$ alkylthio, hydroxyl, thiol, amino, nitro, halogen, 3-12 membered heterocycloalkyl, cyano, $C_1$-$C_{10}$ haloalkyl, $C_3$-$C_8$ halocycloalkyl, $C_2$-$C_4$ ester group, $C_2$-$C_4$ acylamino, $C_1$-$C_4$ carboxyl, $C_2$-$C_6$ alkenyl, $C_2$-$C_6$ alkynyl, $C_6$-$C_{12}$ aryl, and 5-12 membered heteroaryl; and
the heterocycloalkylenyl, heterocycloalkyl and heteroaryl each independently comprises 1-3 (preferably 1, 2 or 3) heteroatoms selected from N, O and S;
a is selected from 1, 2, 3 and 4;
b is selected from 0, 1, 2 and 3; and
c is selected from 0 and 1.

**2.** The compound according to claim 1, wherein the compound has a structure of formula Ia:

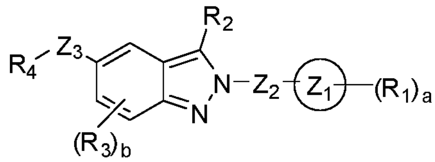

Ia

wherein in the formula Ia, $R_1$, $R_2$, $R_3$, $R_4$, $Z_1$, $Z_2$, $Z_3$, a and b are defined as in claim 1; or the compound has a structure of formula Ib:

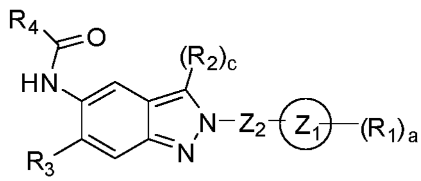

Ib

wherein in the formula Ib, $R_1$, $R_2$, $R_3$, $R_4$, $Z_1$, $Z_2$, a and c are defined as in claim 1; or the compound has a structure of formula Ic:

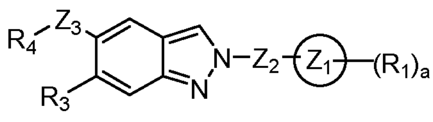

Ic

wherein in the formula Ic, $R_1$, $R_2$, $R_3$, $R_4$, $Z_1$, $Z_2$ and a are defined as in claim 1; or the compound has a structure of formula Id:

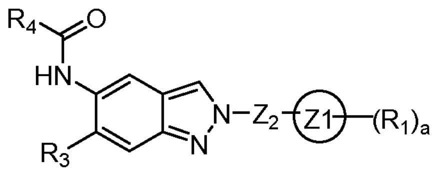

Id

wherein in the formula Id, $R_1$, $R_3$, $R_4$, $Z_1$, $Z_2$ and a are defined as in claim 1; or the compound has a structure of formula Ie:

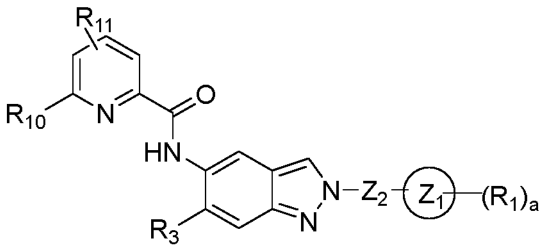

Ie

wherein in the formula Ie, $R_1$, $R_3$, $Z_1$ and $Z_2$ are defined as in claim 1; and $R_{10}$ and $R_{11}$ are each independently selected from a chemical bond, substituted or unsubstituted $C_1$-$C_8$ alkyl, and substituted or unsubstituted $C_3$-$C_6$ cycloalkyl.

**3.** The compound according to claim 1 or 2, wherein the compound comprises one or more features selected from the group consisting of:

(i) $Z_1$ is selected from substituted or unsubstituted $C_3$-$C_8$ cycloalkylenyl, and substituted or unsubstituted 3-8 membered heterocycloalkylenyl;
(ii) $Z_2$ is selected from a chemical bond, and substituted or unsubstituted $C_1$-$C_6$ alkylenyl;
(iii) $R_1$ is selected from hydroxyl, thiol, carboxyl, substituted or unsubstituted $C_1$-$C_6$ alkyl, substituted or unsubstituted $C_3$-$C_8$ cycloalkyl, substituted or unsubstituted $C_1$-$C_6$ hydroxyalkyl, substituted or unsubstituted $C_1$-$C_6$ thiolalkyl, substituted or unsubstituted $C_1$-$C_6$ aminoalkyl, substituted or unsubstituted $C_3$-$C_6$ hydroxycycloalkyl, substituted or unsubstituted $C_3$-$C_6$ thiolcycloalkyl, substituted or unsubstituted $C_3$-$C_6$ aminocycloalkyl, substituted or unsubstituted $C_1$-$C_6$ alkoxy, substituted or unsubstituted $C_1$-$C_6$ alkylthio, -N($R_5$)$R_6$, substituted or unsubstituted $C_1$-$C_4$ carboxyl, substituted or unsubstituted $C_2$-$C_6$ acyl, substituted or unsubstituted $C_2$-$C_6$ ester group, and halogen;
(iv) $R_2$ and $R_3$ are each independently selected from a chemical bond, substituted or unsubstituted $C_1$-$C_6$ alkyl, substituted $C_3$-$C_8$ cycloalkyl, halogen, $C_1$-$C_6$ haloalkyl, $C_1$-$C_{12}$ hydroxyalkyl, cyano, nitro, -A-$R_7$, -N($R_5$)$R_6$, and 5-12 membered heteroaryl; and
(v) $R_4$ is selected from substituted or unsubstituted $C_6$-$C_{12}$ aryl, and substituted or unsubstituted 5-14 membered heteroaryl.

**4.** A compound according to any one of claims 1-3, wherein the compound comprises one or more features selected from the group consisting of:

$Z_1$ is selected from cyclobutylidenyl, azetidinylidenyl and cyclohexylidenyl;
$Z_2$ is selected from a chemical bond, methylenyl and ethylidenyl;
$R_1$ is selected from 2-hydroxyisopropyl, F, 2-hydroxy-2-methylpropyl,

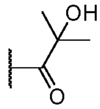

,

hydroxypropyl, hydroxymethyl, hydroxybutyl, hydroxybutyryl, -COOH, hydroxyl, methoxy, $-COOCH_3$, and methyl;
$R_2$ is absent;
$R_3$ is selected from a chemical bond, chloro, fluoro, $-O-R_7$, dimethylamino, methylamino, 2-hydroxyisopropyl and cyano, wherein $R_7$ is selected from tetrahydropyranyl, tetrahydropyridinyl, *N*-methyltetrahydropyridinyl, *N*-acetyltetrahydropyridinyl, tetrahydrofuranyl, cyclopropylmethyl-, *N*-methyltetrahydropyridylmethyl-, methyl, pyrazolyl and 2-pyridinyl;
$Z_3$ is

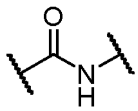

;

and the N is connected with the phenyl ring in the indazole ring;
and $R_4$ is selected from trifluoromethylpyridyl, difluoromethylpyridyl, difluoromethylpyrazinyl, fluoropyridyl,

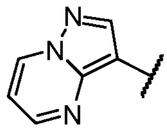

,

trifluoromethylphenyl, methylpyridyl,

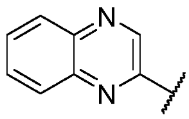

,

cyanopyridyl, cyclopropylpyridyl, and trifluoromethylpyrimidinyl.

**5.** The compound according to any one of claims 1 to 4, wherein the compound is selected from the group consisting of:

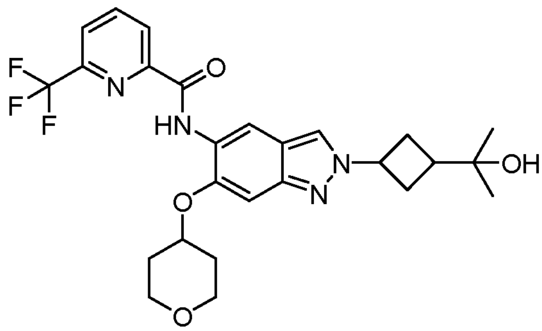

,

I1

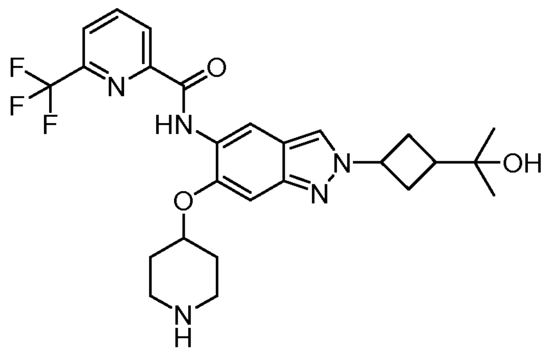

I2

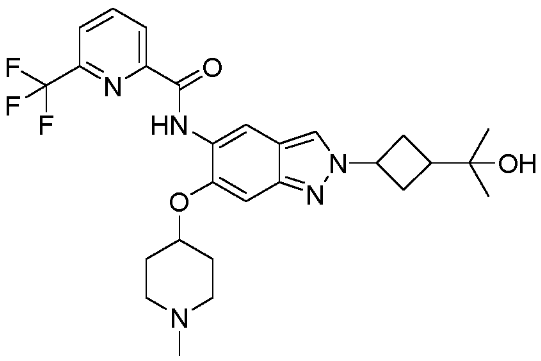

,

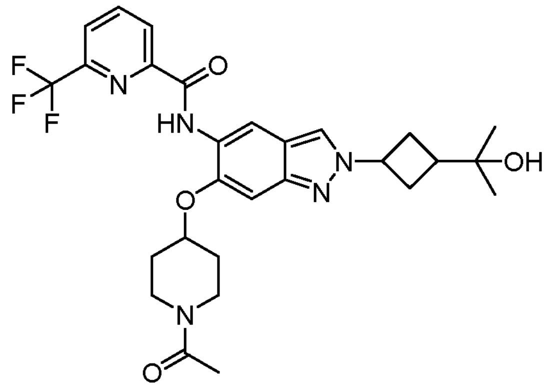

I3

I4

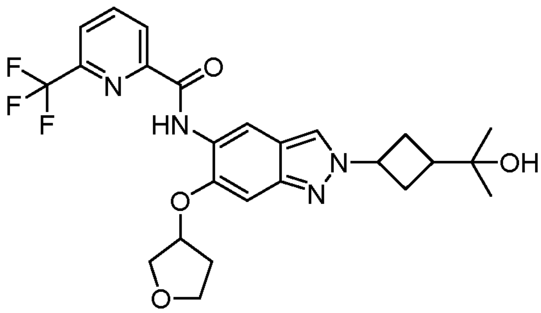

,

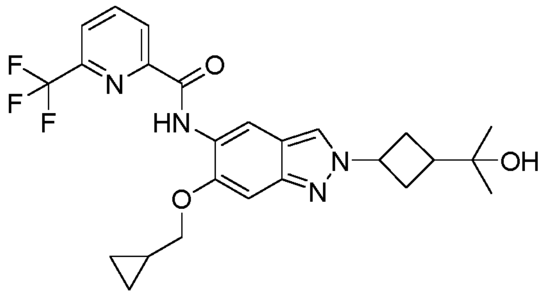

I5

I6

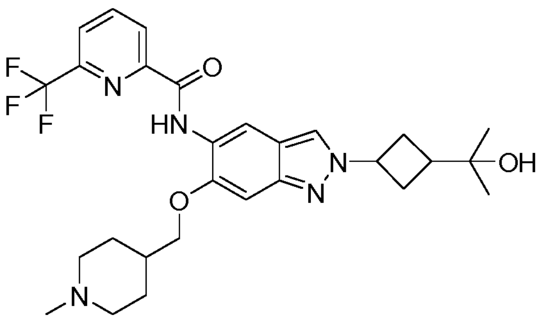

,

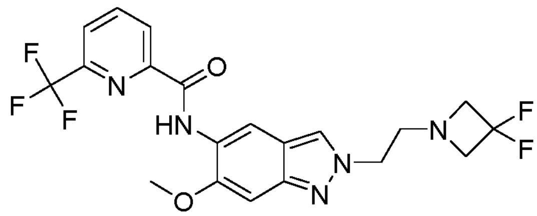

I7

I8

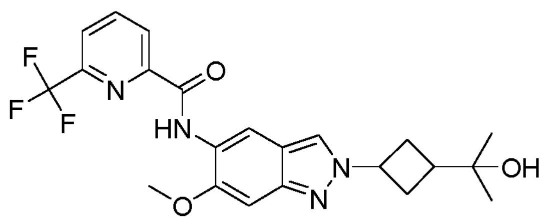

,

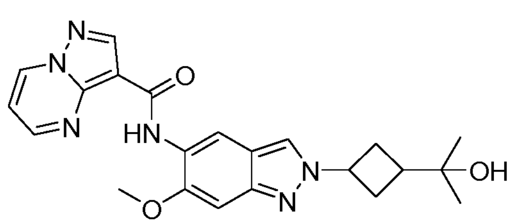

I9

I10

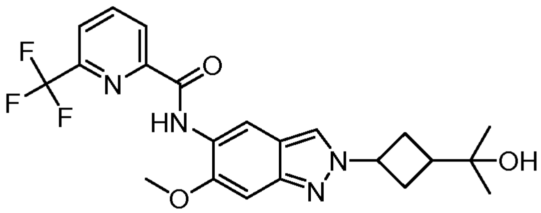
,

I11

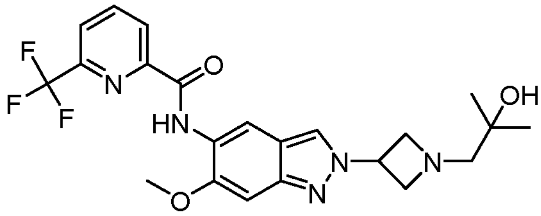

I12

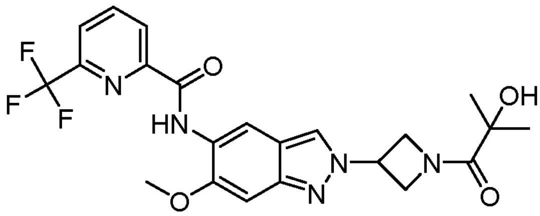
,

I13

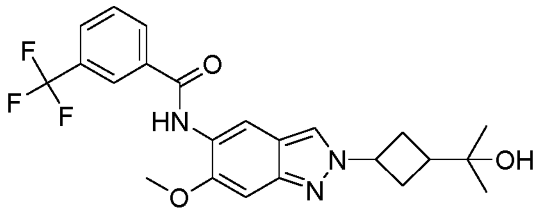

I14

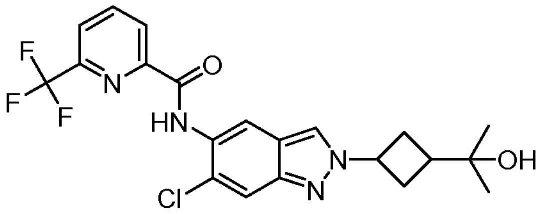
,

I15

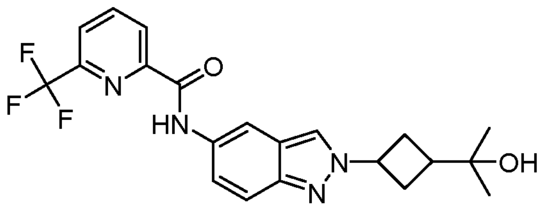

I16

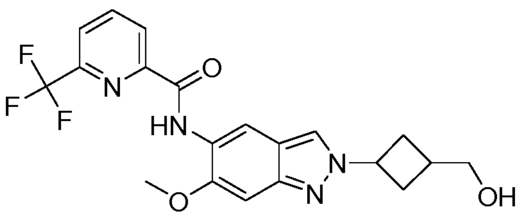
,

I17

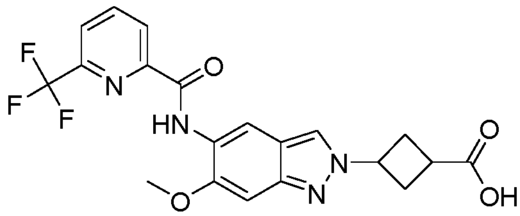

I18

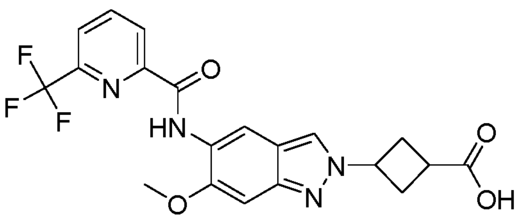
,

I19

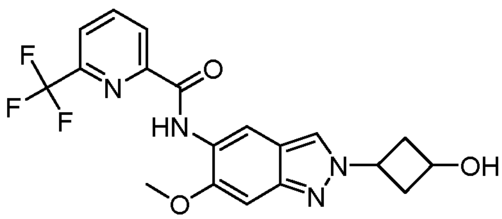

I20

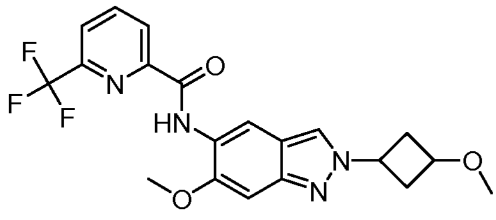,

I21

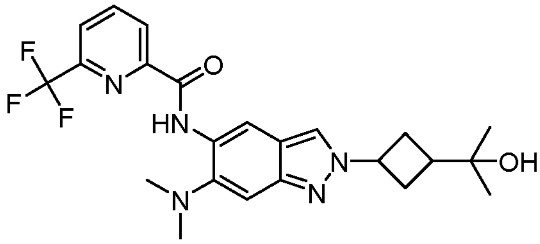

I22

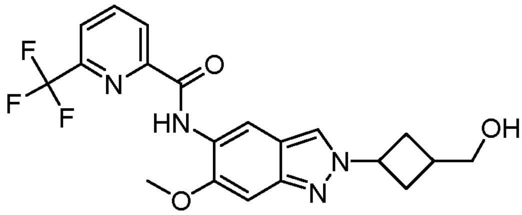,

I23

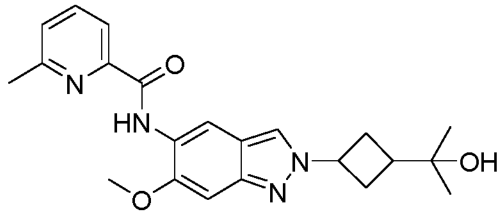

I24

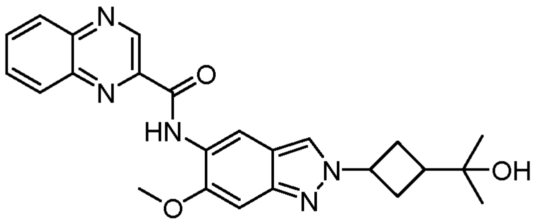,

I25

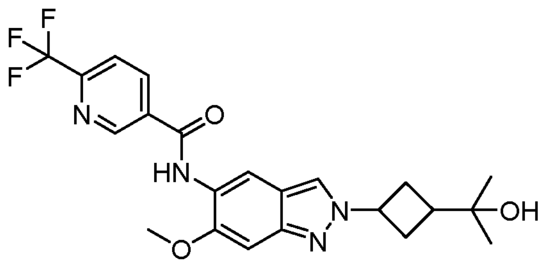

I26

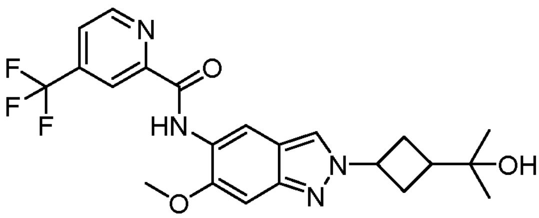,

I27

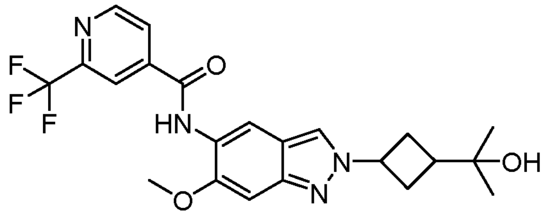

I28

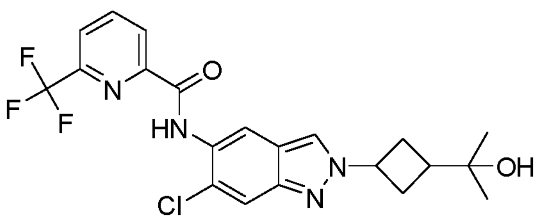,

I29

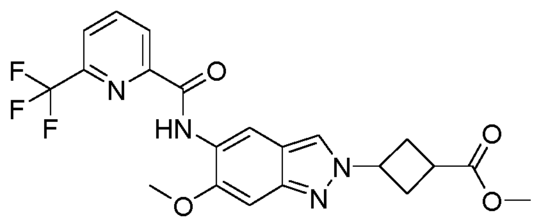

I30

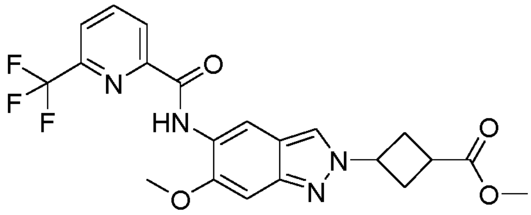,

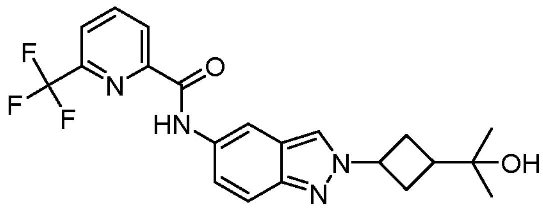

I31 I32

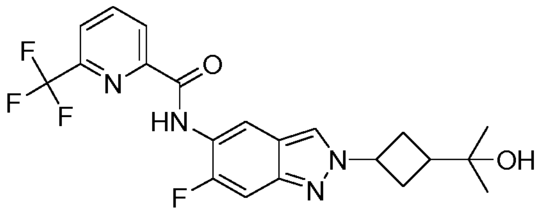,

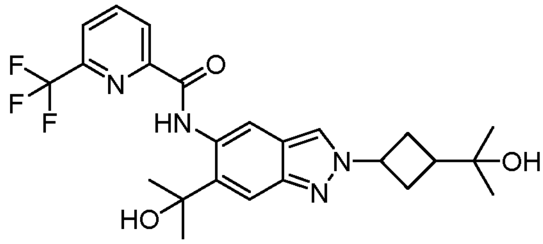

I33 I34

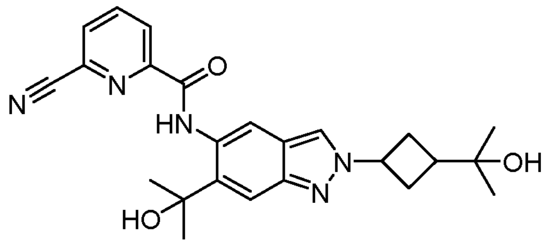,

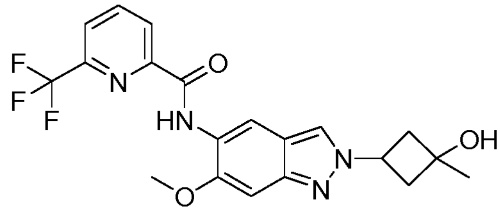

I35 I36

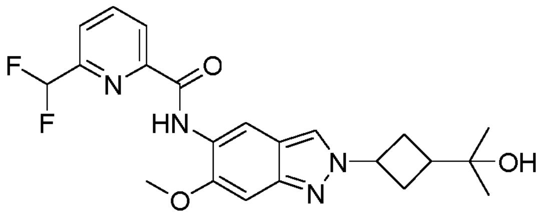,

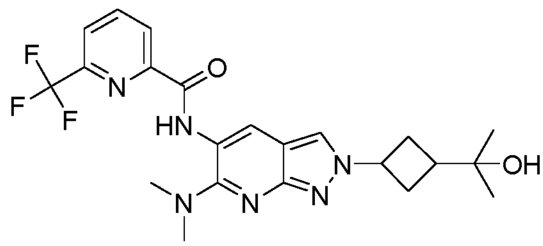

I37 I38

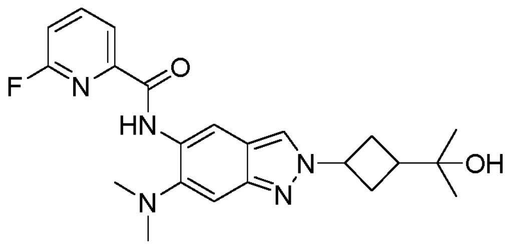,

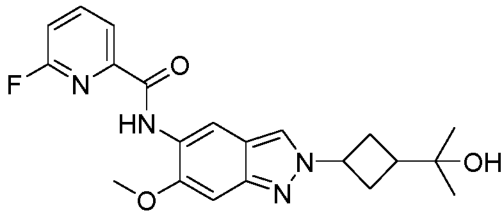

I39 I40

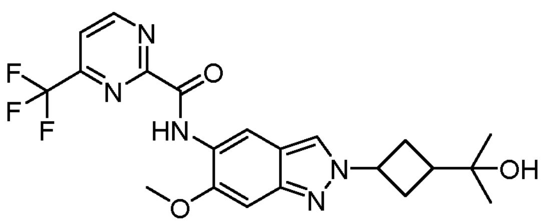,

I41

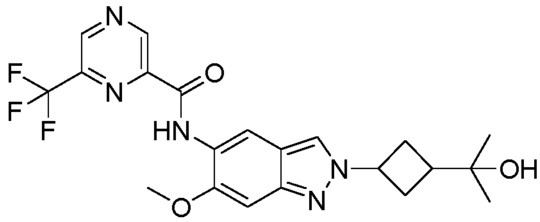

I42

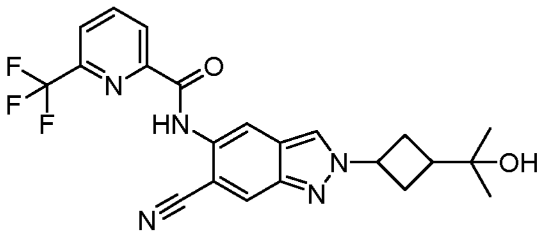,

I43

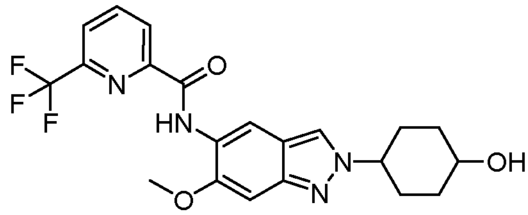

I44

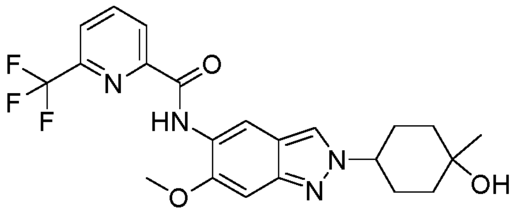,

I45

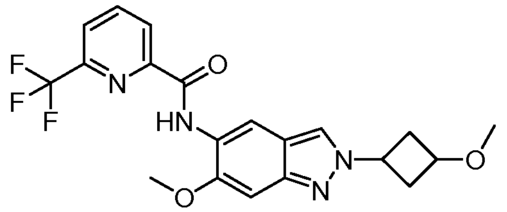

I46

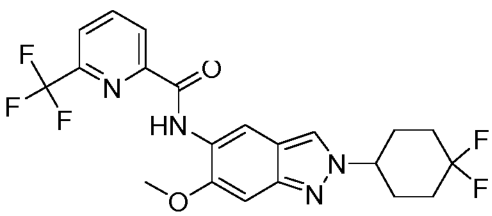,

I47

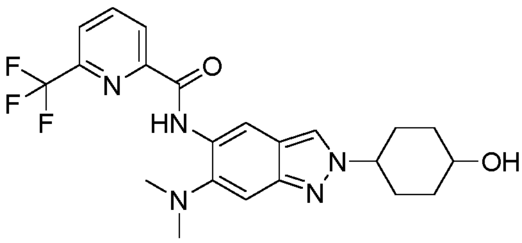

I48

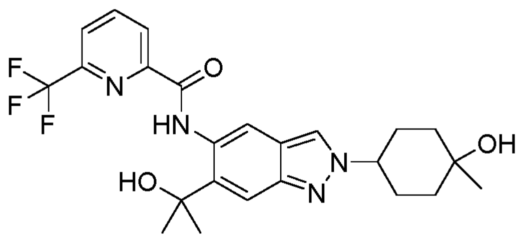,

I49

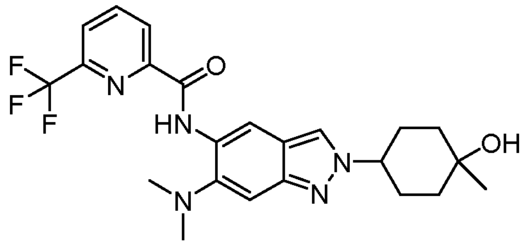.

I50

6. The compound according to any one of claims 1-4, wherein the compound is selected from the group consisting of:

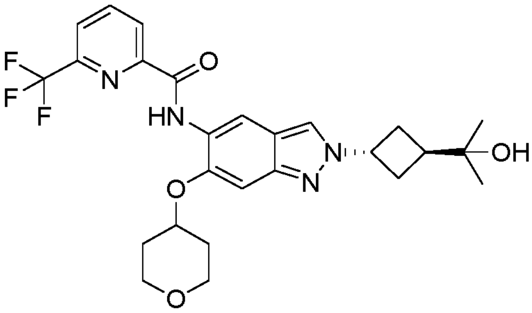
I51,

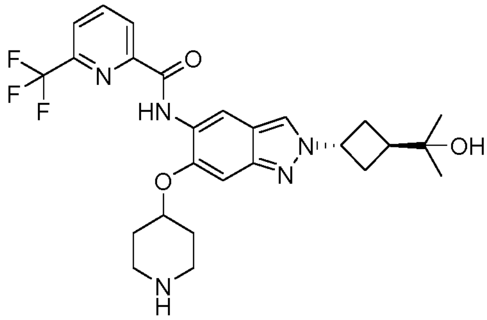
I52

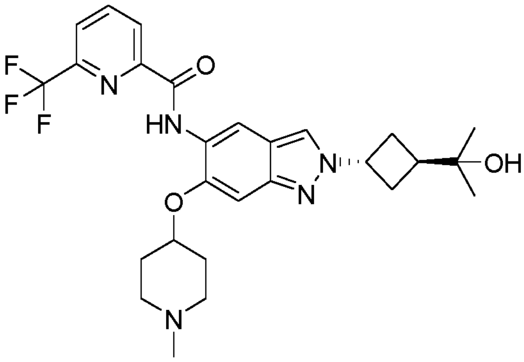
I53,

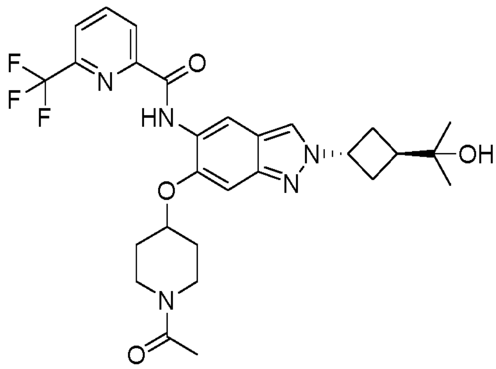
I54

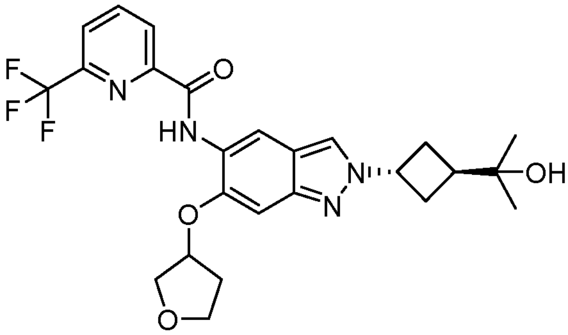
I55,

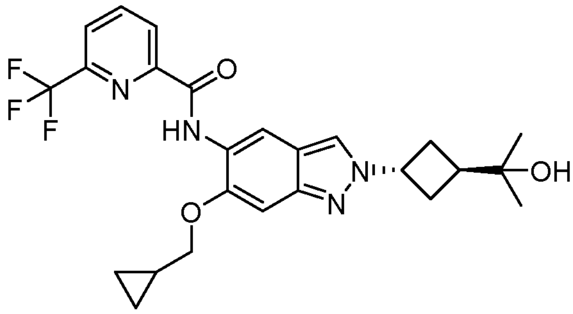
I56

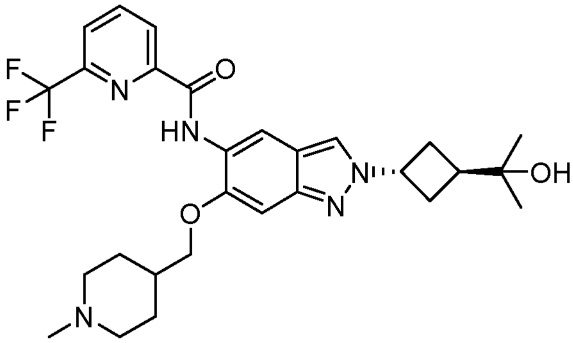
I57,

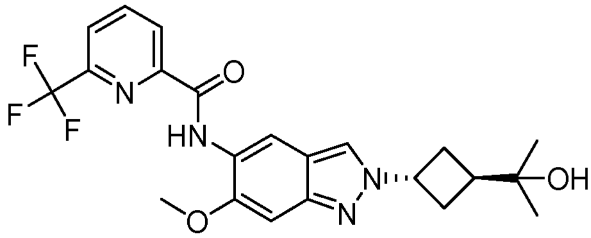
I58

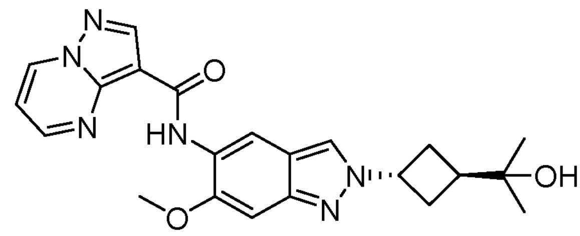
,
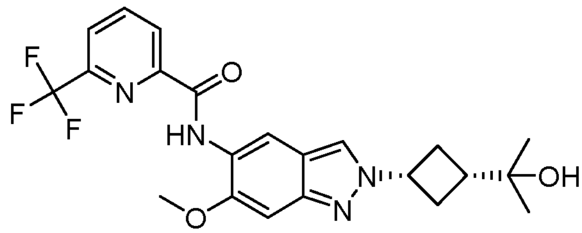

I59 I60

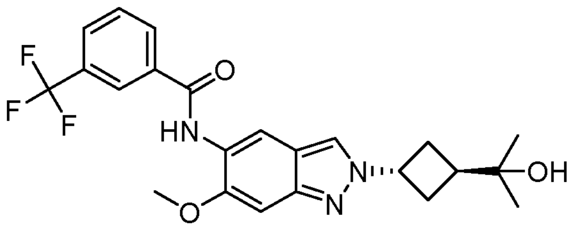
,
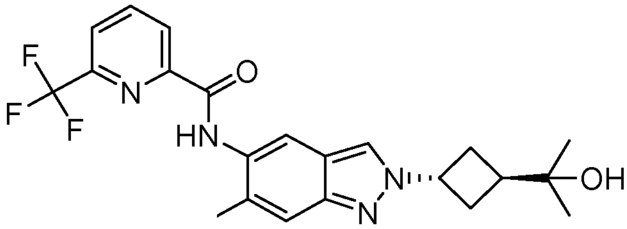

I61 I62

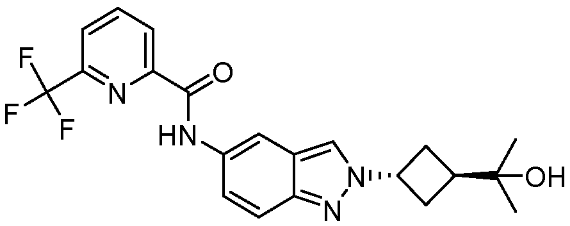
,
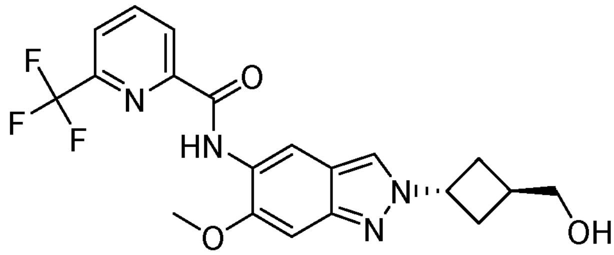

I63 I64

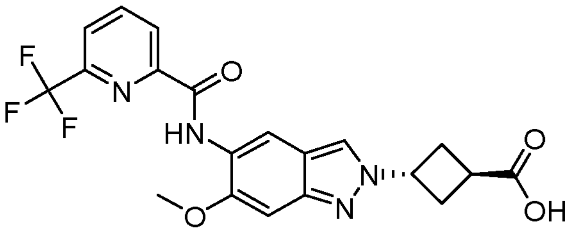
,
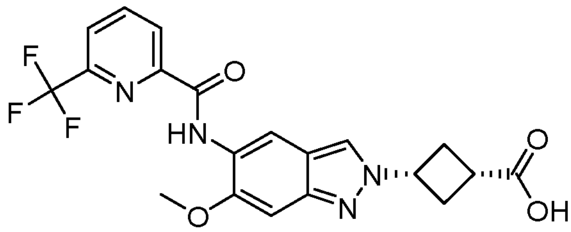

I65 I66

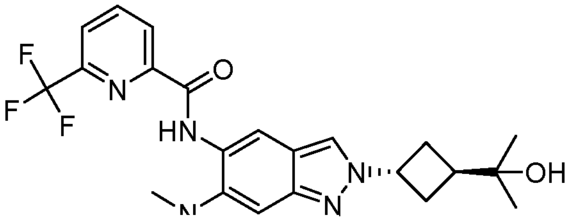
,
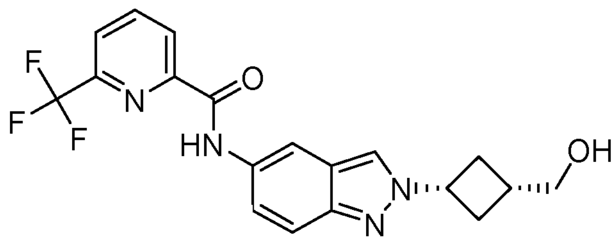

I67

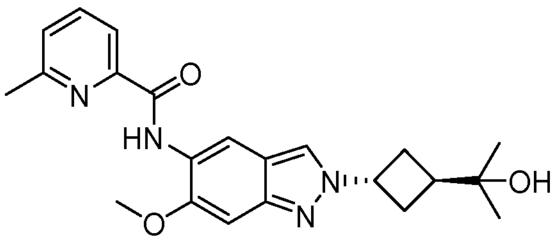
,

I69

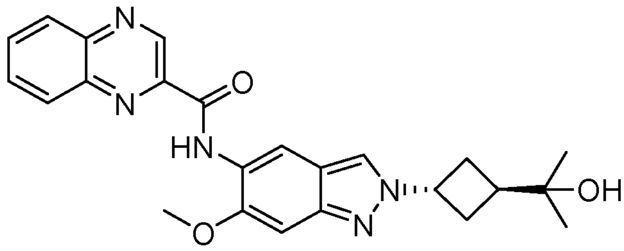

I70

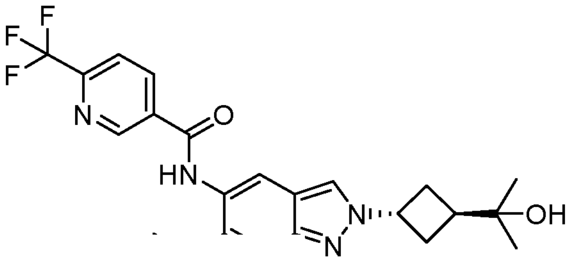
,

I71

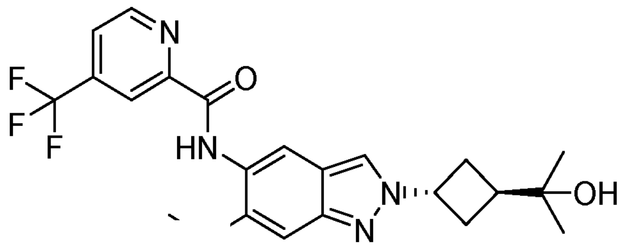

I72

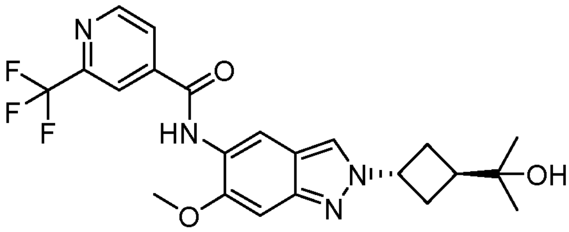
,

I73

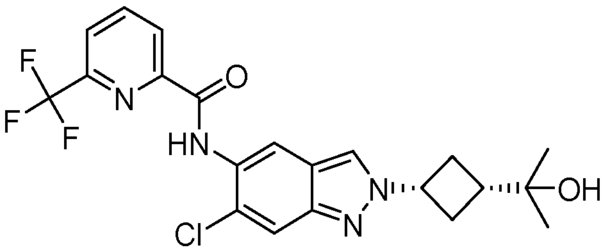

I74

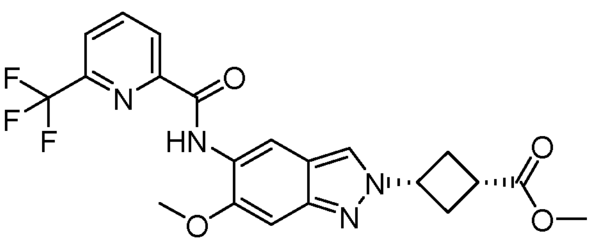
,

I75

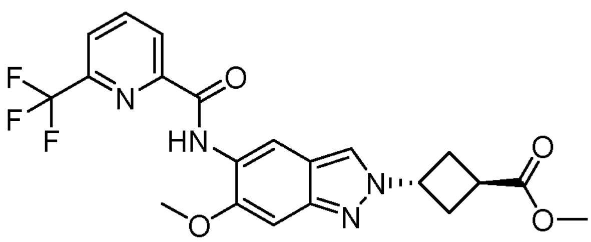

I76

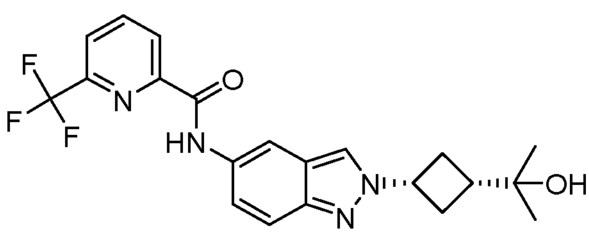
,

I77

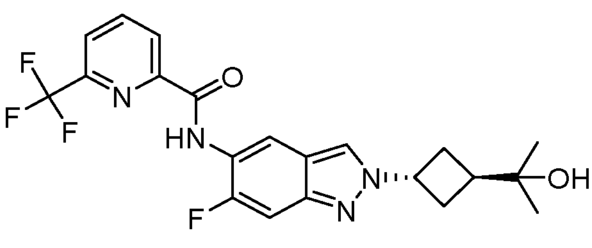

I78

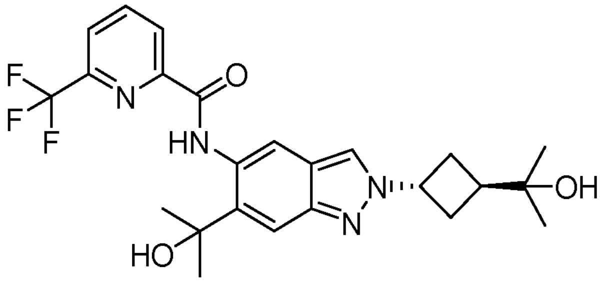

I79

,

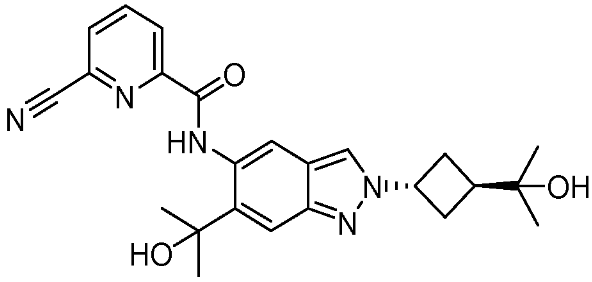

I80

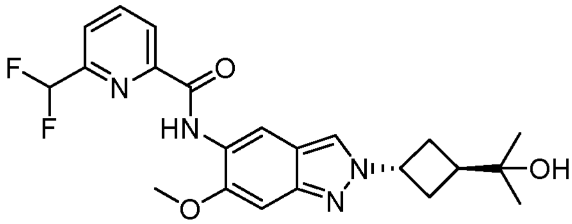

I81

,

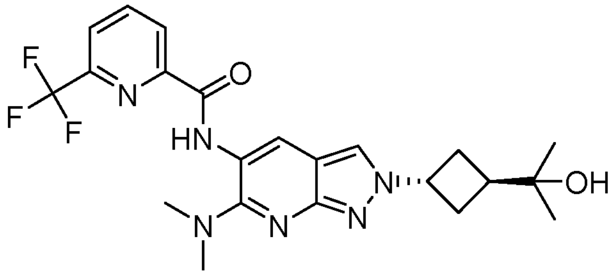

I82

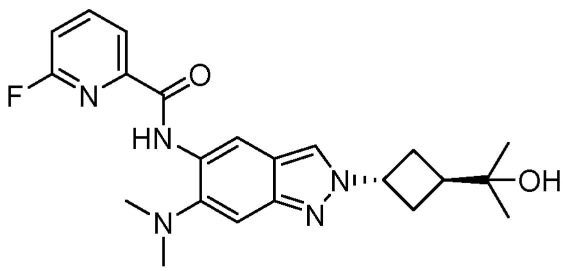

I83

,

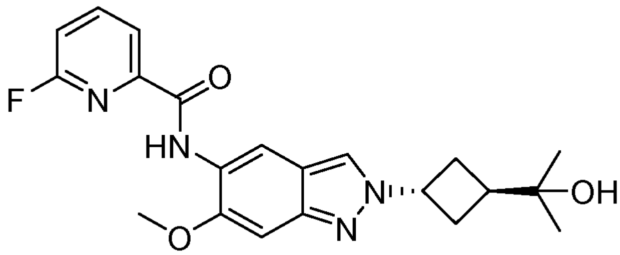

I84

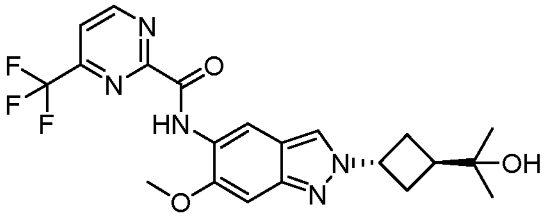

I85

,

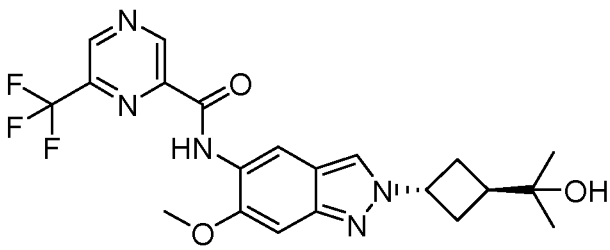

I86

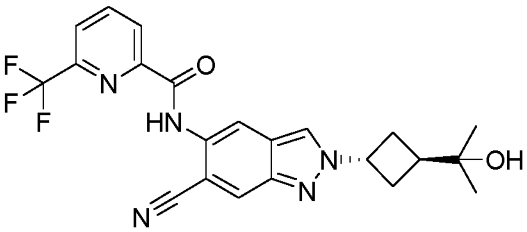

I87

,

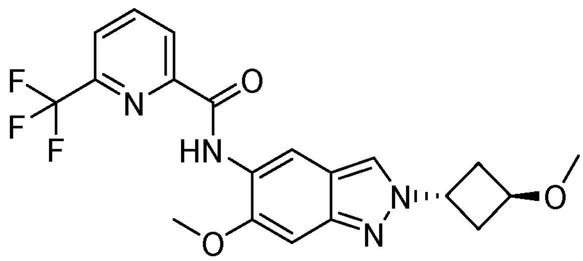

I88

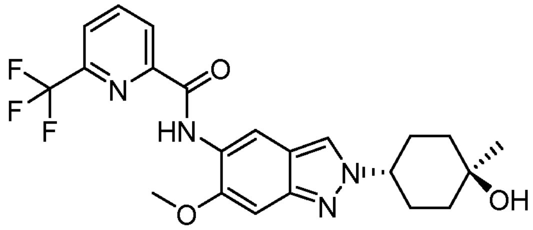

I89,

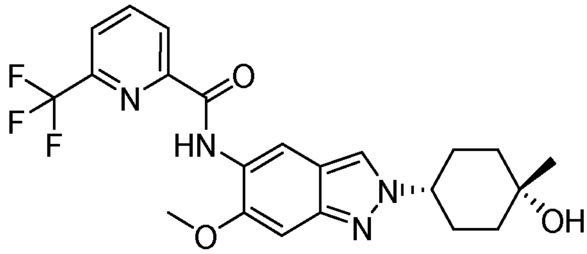

I90

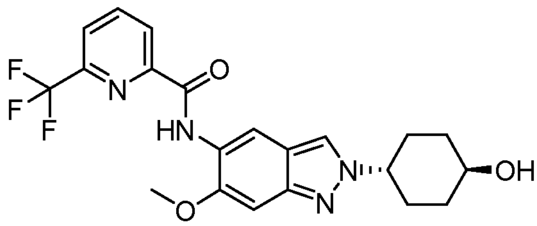

I91,

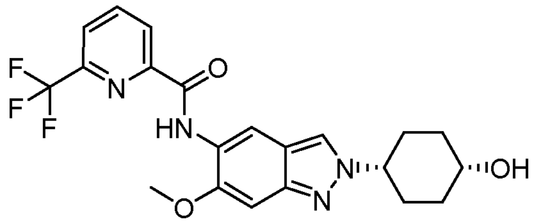

I92

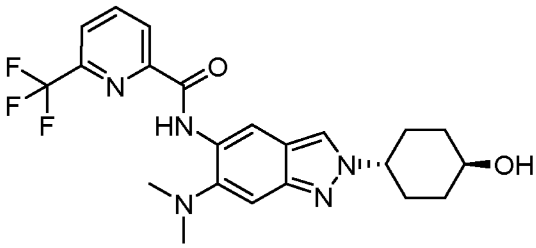

I93,

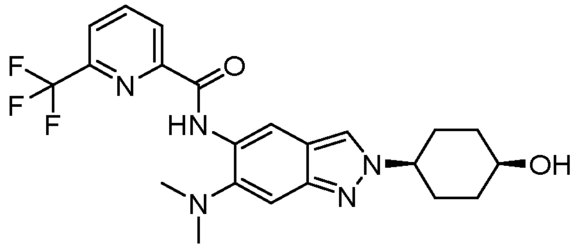

I94

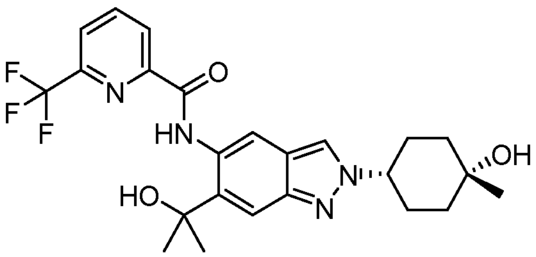

I95,

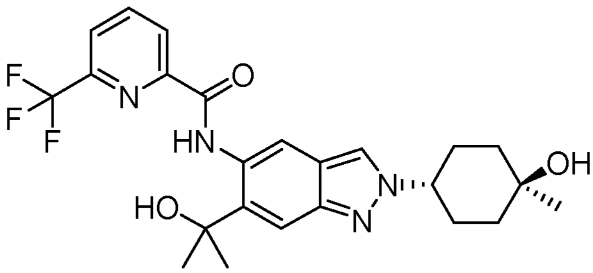

I96

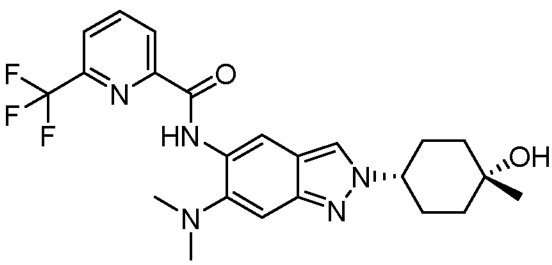

I97,

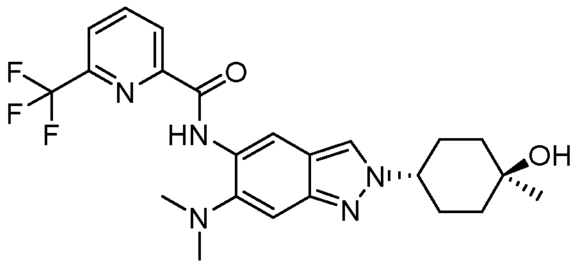

I98

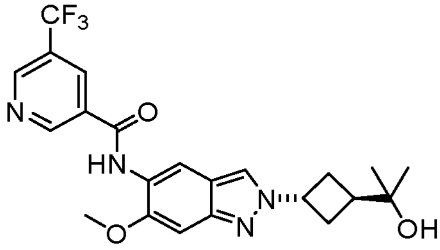,

I99

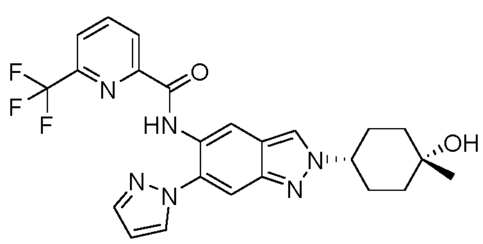

I100

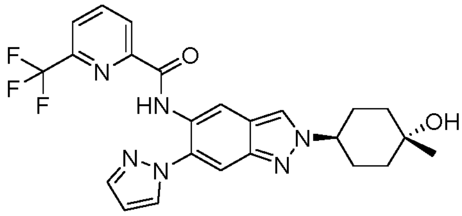,

I101

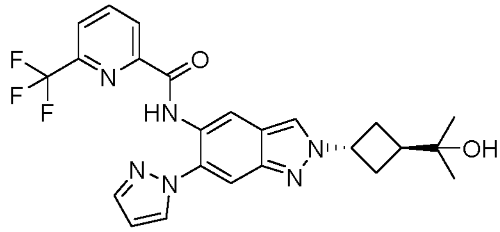

I102

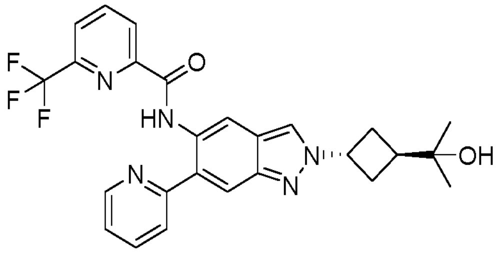,

I103

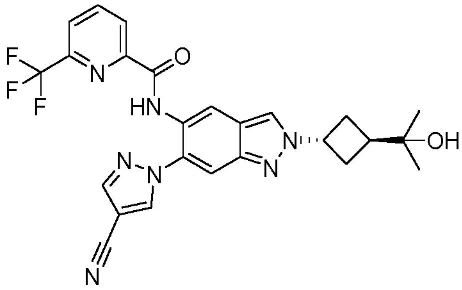

I104

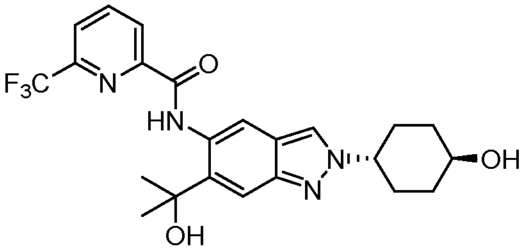

I105

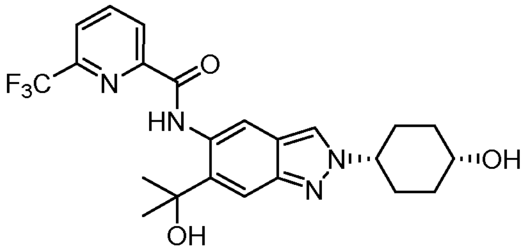

I106

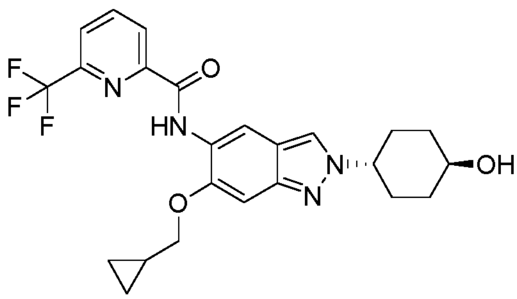

I107

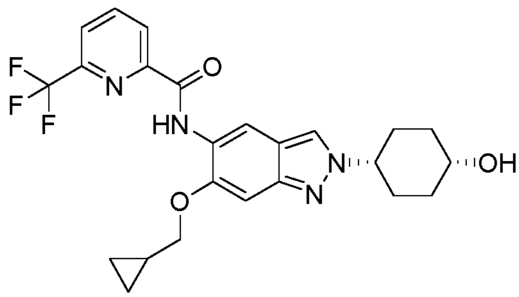.

I108

**7.** A pharmaceutical composition, wherein the pharmaceutical composition comprises: (i) the compound, or the cis-trans isomer, optical isomer, racemate, the pharmaceutically acceptable salt, prodrug, deuterated derivative, the hydrate or the solvate thereof according to any one of claims 1-6; and optionally (ii) a pharmaceutically acceptable carrier.

**8.** The pharmaceutical composition or formulation according to claim 7, wherein the pharmaceutical composition or formulation is used for:

(a) inhibiting interleukin-1 receptor-associated kinase; and/or
(b) preventing and/or treating a disease associated with interleukin-1 receptor-associated kinase;

preferably, the pharmaceutical composition or formulation is further used for: (c) inhibiting cytokine TNF-$\alpha$; and/or (d) preventing and/or treating a disease associated with cytokine TNF.

**9.** Use of the compound, or the cis-trans isomer, optical isomer, racemate, pharmaceutically acceptable salt, prodrug, deuterated derivative, the hydrate thereof or the solvate thereof according to any one of claims 1-6 for preparing a pharmaceutical composition or formulation, wherein the pharmaceutical composition or formulation is used for:

(a) inhibiting interleukin-1 receptor-associated kinase; and/or
(b) preventing and/or treating a disease associated with interleukin-1 receptor-associated kinase.

**10.** The use according to claim 9, wherein the interleukin-1 receptor-associated kinase (IRAK) is selected from the group consisting of: IRAK1, IRAK2, IRAK-M and IRAK4, or a combination thereof.

## INTERNATIONAL SEARCH REPORT

International application No.
**PCT/CN2019/128347**

**A. CLASSIFICATION OF SUBJECT MATTER**

C07D 405/14(2006.01)i; C07D 401/14(2006.01)i; C07D 401/12(2006.01)i; C07D 487/04(2006.01)i; C07D 403/12(2006.01)i; C07D 471/04(2006.01)i; C07D 231/56(2006.01)i; A61K 31/4439(2006.01)i; A61K 31/4545(2006.01)i; A61K 31/519(2006.01)i; A61K 31/416(2006.01)i; A61K 31/498(2006.01)i; A61K 31/497(2006.01)i; A61P 37/00(2006.01)i; A61P 19/02(2006.01)i; A61P 35/00(2006.01)i; A61P 19/06(2006.01)i; A61P 17/06(2006.01)i; A61P 37/02(2006.01)i; A61P 9/10(2006.01)i; A61P 3/00(2006.01)i; A61P 31/04(2006.01)i; A61P 29/00(2006.01)i; A61P 11/06(2006.01)i; A61P 1/00(2006.01)i; A61P 37/08(2006.01)i

According to International Patent Classification (IPC) or to both national classification and IPC

**B. FIELDS SEARCHED**

Minimum documentation searched (classification system followed by classification symbols)

C07D405/-; C07D401/-; C07D487/-; C07D403/-; C07D471/-; C07D231/-; A61K31/-; A61P37/-; A61P19/-; A61P35/-; A61P17/-; A61P9/-; A61P3/-; A61P31/-; A61P29/-; A61P11/-; A61P1/-

Documentation searched other than minimum documentation to the extent that such documents are included in the fields searched

Electronic data base consulted during the international search (name of data base and, where practicable, search terms used)

VEN, DWPI, CNKI, CNABS, STN(REG, CAPLUS): 美悦生物, 冯焱, 野国中, 李世强, 胡治隆, 王朝东, 吲哚, 类风湿, 白介素, 激酶, interleukin, kinase, IRAK, indazo+, IRAK+, RA, rheumatoid w arthritis.

**C. DOCUMENTS CONSIDERED TO BE RELEVANT**

| Category* | Citation of document, with indication, where appropriate, of the relevant passages | Relevant to claim No. |
|---|---|---|
| X | CN 107406416 A (BAYER PHARMA AKTIENGESELLSCHAFT) 28 November 2017 (2017-11-28)<br>claims 1-19, embodiment 22 | 1-10 |
| X | WO 2017108744 A1 (BAYER PHARMA AKTIENGESELLSCHAFT) 29 June 2017 (2017-06-29)<br>embodiments 39, 42 and 45 | 1-3 |
| X | CN 108026065 A (AURIGENE DISCOVERY TECHNOLOGIES LIMITED) 11 May 2018 (2018-05-11)<br>claims 1-36, embodiments 6, 31-32, 54-56 and 64 | 1-10 |
| X | CN 107872977 A (BAYER PHARMA AG) 03 April 2018 (2018-04-03)<br>claims 1-25 | 1-10 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 March 2020** | **31 March 2020** |

Name and mailing address of the ISA/CN

**China National Intellectual Property Administration (ISA/CN)**
**No. 6, Xitucheng Road, Jimenqiao Haidian District, Beijing 100088**
**China**

Facsimile No. **(86-10)62019451**

Authorized officer

Telephone No.

**INTERNATIONAL SEARCH REPORT**
**Information on patent family members**

International application No.
**PCT/CN2019/128347**

| Patent document cited in search report | Publication date (day/month/year) | Patent family member(s) | Publication date (day/month/year) |
|---|---|---|---|
| CN 107406416 A | 28 November 2017 | UY 36411 A | 30 June 2016 |
| | | KR 20170085590 A | 24 July 2017 |
| | | AR 102827 A1 | 29 March 2017 |
| | | US 10308634 B2 | 04 June 2019 |
| | | BR 112017011005 A2 | 14 May 2019 |
| | | NZ 732126 A | 28 September 2018 |
| | | DO P2017000127 A | 31 July 2017 |
| | | SG 10201903474P A | 30 May 2019 |
| | | CA 2968614 A1 | 02 June 2016 |
| | | PE 13762017 A1 | 15 September 2017 |
| | | CL 2017001364 A1 | 15 December 2017 |
| | | US 2019233395 A1 | 01 August 2019 |
| | | IL 267537 D0 | 29 August 2019 |
| | | SG 11201704092Y A | 29 June 2017 |
| | | JP 2017535585 A | 30 November 2017 |
| | | WO 2016083433 A1 | 02 June 2016 |
| | | IL 252185 D0 | 31 July 2017 |
| | | TW 201629037 A | 16 August 2016 |
| | | AU 2015352603 A1 | 01 June 2017 |
| | | EA 201791137 A1 | 30 November 2017 |
| | | EP 3224254 A1 | 04 October 2017 |
| | | TN 2017000226 A1 | 19 October 2018 |
| | | JP 6496823 B2 | 10 April 2019 |
| | | MX 2017006910 A | 15 August 2017 |
| | | US 2017349570 A1 | 07 December 2017 |
| | | CU 20170073 A7 | 05 October 2017 |
| | | PH 12017500972 A1 | 18 December 2017 |
| | | CA 2968614 C | 29 October 2019 |
| | | IL 252185 A | 31 October 2019 |
| | | SG 10201903475T A | 30 May 2019 |
| | | CN 110305109 A | 08 October 2019 |
| | | EA 032509 B1 | 28 June 2019 |
| | | CR 20 February 2017 A | 31 October 2017 |
| | | PE 20171376 A1 | 15 September 2017 |
| | | IL 269444 D0 | 28 November 2019 |
| WO 2017108744 A1 | 29 June 2017 | UY 37048 A | 31 July 2017 |
| | | TW 201725202 A | 16 July 2017 |
| | | AR 107127 A1 | 21 March 2018 |
| CN 108026065 A | 11 May 2018 | CA 2992406 A1 | 19 January 2017 |
| | | EP 3322698 A4 | 09 January 2019 |
| | | JP 2018524372 A | 30 August 2018 |
| | | IL 256584 D0 | 28 February 2018 |
| | | US 2018201609 A1 | 19 July 2018 |
| | | PH 12018500040 A1 | 09 July 2018 |
| | | KR 20180025896 A | 09 March 2018 |
| | | CU 20180006 A7 | 05 June 2018 |
| | | AU 2016293441 A1 | 01 February 2018 |
| | | WO 2017009798 A1 | 19 January 2017 |
| | | MX 2018000512 A | 13 April 2018 |
| | | EP 3322698 A1 | 23 May 2018 |

Form PCT/ISA/210 (patent family annex) (January 2015)

**INTERNATIONAL SEARCH REPORT**
**Information on patent family members**

International application No.
**PCT/CN2019/128347**

| Patent document cited in search report | Publication date (day/month/year) | Patent family member(s) | Publication date (day/month/year) |
|---|---|---|---|
| | | BR 112018000624 A2 | 18 September 2018 |
| | | EA 201890307 A1 | 31 October 2018 |
| CN 107872977 A | 03 April 2018 | UY 36660 A | 30 November 2016 |
| | | CA 2984259 A1 | 03 November 2016 |
| | | WO 2016174183 A1 | 03 November 2016 |
| | | US 2018289685 A1 | 11 October 2018 |
| | | US 2019388410 A1 | 26 December 2019 |
| | | EP 3288558 A1 | 07 March 2018 |
| | | JP 2018514539 A | 07 June 2018 |
| | | TW 201701879 A | 16 January 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201811593893X


### Non-patent literature cited in the description


- **FLANNERY.** *Biochem. Pharmacol.,* 2010, vol. 80 (12), 1981-1991
- **PRISCILLA N.** *J. Exp. Med.,* 2015, vol. 13 (212), 2189-2201
- **CHAUDHARY D.** *J. Med. Chem.,* 2015, vol. 58 (1), 96-110